# EUROPEAN PATENT SPECIFICATION

(11) **EP 0 661 987 B1**
(45) Date of publication and mention of the grant of the patent: **14.01.1998**
(21) Application number: 93922700.5
(22) Date of filing: 16.09.1993
(51) Int. Cl.: A61K 38/18

(54) **MORPHOGEN-INDUCED LIVER REGENERATION**
MORPHOGENINDUZIERTE REGENERIERUNG DER LEBER
REGENERATION DU FOIE INDUITE PAR DES MORPHOGENES

(30) Priority: 16.09.1992 US 946238; 04.03.1993 US 29335; 31.03.1993 US 40510
(43) Date of publication of application: 12.07.1995
(73) Proprietor: CREATIVE BIOMOLECULES, INC., Hopkinton, MA 01748 (US)
(72) Inventor: KUBERASAMPATH, Thangavel, Medway, MA 02053 (US); RUEGER, David, C., Hopkinton, MA 01748 (US); OPPERMANN, Hermann, Medway, MA 02053 (US); PANG, Roy, H., L., Etna, NH 03750 (US); COHEN, Charles, M., Weston, Massachusetts 02193, (US); OZKAYNAK, Engin, Milford, MA 01757 (US); SMART, John, E., Weston, MA 02193 (US)
(74) Representative: Price, Vincent Andrew
(86) International application number: US9308808
(87) International publication number: WO9406449

(56) References cited:
- WO-A-89/10409
- WO-A-92/09301
- WO-A-92/15323
- WO-A-93/04692

SEQUENCE LISTING

(1) GENERAL INFORMATION:

(i) APPLICANT:
(A) NAME: CREATIVE BIOMOLECULES, INC.
(B) STREET: 45 SOUTH STREET
(C) CITY: HOPKINTON
(D) STATE: MA
(E) COUNTRY: USA
(F) POSTAL CODE (ZIP): 01748
(G) TELEPHONE: 1-508-435-9001
(H) TELEFAX: 1-508-435-0454
(I) TELEX:

(ii) TITLE OF INVENTION: MORPHOGEN-INDUCED LIVER REGENERATION

(iii) NUMBER OF SEQUENCES: 33

(iv) CORRESPONDENCE ADDRESS:
(A) ADDRESSEE: CREATIVE BIOMOLECULES, INC.
(B) STREET: 45 SOUTH STREET
(C) CITY: HOPKINTON
(D) STATE: MA
(E) COUNTRY: USA
(F) ZIP: 01748

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER:
(B) FILING DATE:

(viii) ATTORNEY/AGENT INFORMATION:
(A) NAME: KELLEY ESQ, ROBIN D.
(B) REGISTRATION NUMBER: 34,637
(C) REFERENCE/DOCKET NUMBER: CRP-072

(ix) TELECOMMUNICATION INFORMATION:
(A) TELEPHONE: 617/248-7477
(B) TELEFAX: 617/248-7100

(2) INFORMATION FOR SEQ ID NO:1:

(i) SEQUENCE CHARACTERISTICS:
(A) LENGTH: 97 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..97
(D) OTHER INFORMATION: /label= GENERIC-SEQ1
/note= "WHEREIN EACH XAA INDEPENDENTLY INDICATES
ONE OF THE 20 NATURALLY-OCCURING L-ISOMER, A-AMINO
ACIDS, OR A DERIVATIVE THEREOF."

(xi) SEQUENCE DESCRIPTION: SEQ ID NO:1:

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Xaa Cys Xaa Xaa Xaa Cys Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Cys Cys Xaa Xaa
    50                  55                  60

Xaa Xaa Xaa Xaa Xaa Xaa Xaa Xaa Xaa Xaa Xaa Xaa Xaa Xaa Xaa Xaa
65                  70                  75                  80

Xaa Xaa Xaa Xaa Xaa Xaa Xaa Xaa Xaa Xaa Xaa Xaa Xaa Cys Xaa Cys
                85                  90                  95

Xaa

(2) INFORMATION FOR SEQ ID NO:2:

(i) SEQUENCE CHARACTERISTICS:
(A) LENGTH: 97 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..97
(D) OTHER INFORMATION: /label= GENERIC-SEQ2
/note= "WHEREIN EACH XAA INDEPENDENTLY INDICATES
ONE OF THE 20 NATURALLY OCCURING L-ISOMER A-AMINO
ACIDS, OR A DERIVATIVE THEREOF."

(xi) SEQUENCE DESCRIPTION: SEQ ID NO:2:

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Xaa Cys Xaa Xaa Xaa Cys Xaa Xaa Xaa
            20                  25                  30

Xaa Xaa Xaa Cys Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Cys Cys Xaa Xaa
    50                  55                  60

Xaa Xaa Xaa Xaa Xaa Xaa Xaa Xaa Xaa Xaa Xaa Xaa Xaa Xaa Xaa Xaa
65                  70                  75                  80

Xaa Xaa Xaa Xaa Xaa Xaa Xaa Xaa Xaa Xaa Xaa Xaa Xaa Cys Xaa Cys
                85                  90                  95

Xaa

(2) INFORMATION FOR SEQ ID NO:3:

(i) SEQUENCE CHARACTERISTICS:
(A) LENGTH: 97 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..97
(D) OTHER INFORMATION: /label= GENERIC-SEQ3
/note= "WHEREIN EACH XAA IS INDEPENDENTLY SELECTED
FROM A GROUP OF ONE OR MORE SPECIFIED AMINO ACIDS
AS DEFINED IN THE SPECIFICATION."

(xi) SEQUENCE DESCRIPTION: SEQ ID NO:3:

Leu Tyr Val Xaa Phe Xaa Xaa Xaa Gly Trp Xaa Xaa Trp Xaa Xaa Ala
1               5                   10                  15

Pro Xaa Gly Xaa Xaa Ala Xaa Tyr Cys Xaa Gly Xaa Cys Xaa Xaa Pro
            20                  25                  30

Xaa Xaa Xaa Xaa Xaa Xaa Xaa Xaa Asn His Ala Xaa Xaa Xaa Xaa Leu
        35                  40                  45

Xaa Xaa Xaa Xaa Xaa Xaa Xaa Xaa Xaa Xaa Xaa Xaa Cys Cys Xaa Pro
    50                  55                  60

Xaa Xaa Xaa Xaa Xaa Xaa Xaa Xaa Leu Xaa Xaa Xaa Xaa Xaa Xaa Xaa
65                  70                  75                  80

Val Xaa Leu Xaa Xaa Xaa Xaa Xaa Met Xaa Val Xaa Xaa Cys Gly Cys
                85                  90                  95

Xaa

(2) INFORMATION FOR SEQ ID NO:4:

    (i) SEQUENCE CHARACTERISTICS:
        (A) LENGTH: 102 amino acids
        (B) TYPE: amino acid
        (C) STRANDEDNESS: single
        (D) TOPOLOGY: linear

   (ii) MOLECULE TYPE: protein

   (ix) FEATURE:
        (A) NAME/KEY: Protein
        (B) LOCATION: 1..102
        (D) OTHER INFORMATION: /label= GENERIC-SEQ4
              /note= "WHEREIN EACH XAA IS INDEPENDENTLY SELECTED
              FROM A GROUP OF ONE OR MORE SPECIFIED AMINO ACIDS
              AS DEFINED IN THE SPECIFICATION."

   (xi) SEQUENCE DESCRIPTION: SEQ ID NO:4:

Cys Xaa Xaa Xaa Xaa Leu Tyr Val Xaa Phe Xaa Xaa Xaa Gly Trp Xaa
1               5                   10                  15

Xaa Trp Xaa Xaa Ala Pro Xaa Gly Xaa Xaa Ala Xaa Tyr Cys Xaa Gly
            20                  25                  30

Xaa Cys Xaa Xaa Pro Xaa Xaa Xaa Xaa Xaa Xaa Xaa Xaa Asn His Ala
        35                  40                  45

Xaa Xaa Xaa Xaa Leu Xaa Xaa Xaa Xaa Xaa Xaa Xaa Xaa Xaa Xaa Xaa
    50                  55                  60

Xaa Cys Cys Xaa Pro Xaa Xaa Xaa Xaa Xaa Xaa Xaa Xaa Leu Xaa Xaa
65                  70                  75                  80

Xaa Xaa Xaa Xaa Xaa Val Xaa Leu Xaa Xaa Xaa Xaa Xaa Met Xaa Val
                85                  90                  95

Xaa Xaa Cys Gly Cys Xaa
            100

(2) INFORMATION FOR SEQ ID NO:5:

    (i) SEQUENCE CHARACTERISTICS:
        (A) LENGTH: 139 amino acids
        (B) TYPE: amino acid
        (C) STRANDEDNESS: single
        (D) TOPOLOGY: linear

    (ii) MOLECULE TYPE: protein

    (vi) ORIGINAL SOURCE:
        (A) ORGANISM: Homo sapiens
        (F) TISSUE TYPE: HIPPOCAMPUS

    (ix) FEATURE:
        (A) NAME/KEY: Protein
        (B) LOCATION: 1..139
        (D) OTHER INFORMATION: /label= hOP1-MATURE

    (xi) SEQUENCE DESCRIPTION: SEQ ID NO:5:

Ser Thr Gly Ser Lys Gln Arg Ser Gln Asn Arg Ser Lys Thr Pro Lys
1               5                   10                  15

Asn Gln Glu Ala Leu Arg Met Ala Asn Val Ala Glu Asn Ser Ser Ser
            20                  25                  30

Asp Gln Arg Gln Ala Cys Lys Lys His Glu Leu Tyr Val Ser Phe Arg
        35                  40                  45

Asp Leu Gly Trp Gln Asp Trp Ile Ile Ala Pro Glu Gly Tyr Ala Ala
    50                  55                  60

Tyr Tyr Cys Glu Gly Glu Cys Ala Phe Pro Leu Asn Ser Tyr Met Asn
65                  70                  75                  80

Ala Thr Asn His Ala Ile Val Gln Thr Leu Val His Phe Ile Asn Pro
                85                  90                  95

Glu Thr Val Pro Lys Pro Cys Cys Ala Pro Thr Gln Leu Asn Ala Ile
            100                 105                 110

Ser Val Leu Tyr Phe Asp Asp Ser Ser Asn Val Ile Leu Lys Lys Tyr
        115                 120                 125

Arg Asn Met Val Val Arg Ala Cys Gly Cys His
    130                 135

(2) INFORMATION FOR SEQ ID NO:6:

(i) SEQUENCE CHARACTERISTICS:
(A) LENGTH: 139 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: MURIDAE
(F) TISSUE TYPE: EMBRYO

(ix) FEATURE:
(A) NAME/KEY: Protein
(B) LOCATION: 1..139
(D) OTHER INFORMATION: /label= MOP1-MATURE

(xi) SEQUENCE DESCRIPTION: SEQ ID NO:6:

Ser Thr Gly Gly Lys Gln Arg Ser Gln Asn Arg Ser Lys Thr Pro Lys
1               5                   10                  15

Asn Gln Glu Ala Leu Arg Met Ala Ser Val Ala Glu Asn Ser Ser Ser
            20                  25                  30

Asp Gln Arg Gln Ala Cys Lys Lys His Glu Leu Tyr Val Ser Phe Arg
        35                  40                  45

Asp Leu Gly Trp Gln Asp Trp Ile Ile Ala Pro Glu Gly Tyr Ala Ala
    50                  55                  60

Tyr Tyr Cys Glu Gly Glu Cys Ala Phe Pro Leu Asn Ser Tyr Met Asn
65                  70                  75                  80

Ala Thr Asn His Ala Ile Val Gln Thr Leu Val His Phe Ile Asn Pro
                85                  90                  95

Asp Thr Val Pro Lys Pro Cys Cys Ala Pro Thr Gln Leu Asn Ala Ile
            100                 105                 110

Ser Val Leu Tyr Phe Asp Asp Ser Ser Asn Val Ile Leu Lys Lys Tyr
        115                 120                 125

Arg Asn Met Val Val Arg Ala Cys Gly Cys His
    130                 135

(2) INFORMATION FOR SEQ ID NO:7:

(i) SEQUENCE CHARACTERISTICS:
(A) LENGTH: 139 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: HOMO SAPIENS
(F) TISSUE TYPE: HIPPOCAMPUS

(ix) FEATURE:
(A) NAME/KEY: Protein
(B) LOCATION: 1..139
(D) OTHER INFORMATION: /label= HOP2-MATURE

(xi) SEQUENCE DESCRIPTION: SEQ ID NO:7:

Ala Val Arg Pro Leu Arg Arg Arg Gln Pro Lys Lys Ser Asn Glu Leu
1               5                   10                  15

Pro Gln Ala Asn Arg Leu Pro Gly Ile Phe Asp Asp Val His Gly Ser
            20                  25                  30

His Gly Arg Gln Val Cys Arg Arg His Glu Leu Tyr Val Ser Phe Gln
        35                  40                  45

Asp Leu Gly Trp Leu Asp Trp Val Ile Ala Pro Gln Gly Tyr Ser Ala
    50                  55                  60

Tyr Tyr Cys Glu Gly Glu Cys Ser Phe Pro Leu Asp Ser Cys Met Asn
65                  70                  75                  80

Ala Thr Asn His Ala Ile Leu Gln Ser Leu Val His Leu Met Lys Pro
                85                  90                  95

Asn Ala Val Pro Lys Ala Cys Cys Ala Pro Thr Lys Leu Ser Ala Thr
            100                 105                 110

Ser Val Leu Tyr Tyr Asp Ser Ser Asn Asn Val Ile Leu Arg Lys His
        115                 120                 125

Arg Asn Met Val Val Lys Ala Cys Gly Cys His
    130                 135

(2) INFORMATION FOR SEQ ID NO:8:

(i) SEQUENCE CHARACTERISTICS:
(A) LENGTH: 139 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: MURIDAE
(F) TISSUE TYPE: EMBRYO

(ix) FEATURE:
(A) NAME/KEY: Protein
(B) LOCATION: 1..139
(D) OTHER INFORMATION: /label= MOP2-MATURE

(xi) SEQUENCE DESCRIPTION: SEQ ID NO:8:

Ala Ala Arg Pro Leu Lys Arg Arg Gln Pro Lys Lys Thr Asn Glu Leu
1               5                   10                  15

Pro His Pro Asn Lys Leu Pro Gly Ile Phe Asp Asp Gly His Gly Ser
            20                  25                  30

Arg Gly Arg Glu Val Cys Arg Arg His Glu Leu Tyr Val Ser Phe Arg
        35                  40                  45

Asp Leu Gly Trp Leu Asp Trp Val Ile Ala Pro Gln Gly Tyr Ser Ala
    50                  55                  60

Tyr Tyr Cys Glu Gly Glu Cys Ala Phe Pro Leu Asp Ser Cys Met Asn
65                  70                  75                  80

Ala Thr Asn His Ala Ile Leu Gln Ser Leu Val His Leu Met Lys Pro
                85                  90                  95

Asp Val Val Pro Lys Ala Cys Cys Ala Pro Thr Lys Leu Ser Ala Thr
            100                 105                 110

Ser Val Leu Tyr Tyr Asp Ser Ser Asn Asn Val Ile Leu Arg Lys His
        115                 120                 125

Arg Asn Met Val Val Lys Ala Cys Gly Cys His
    130                 135

(2) INFORMATION FOR SEQ ID NO:9:

(i) SEQUENCE CHARACTERISTICS:
(A) LENGTH: 101 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: bovinae

(ix) FEATURE:
(A) NAME/KEY: Protein
(B) LOCATION: 1..101
(D) OTHER INFORMATION: /label= CBMP-2A-FX

(xi) SEQUENCE DESCRIPTION: SEQ ID NO:9:

Cys Lys Arg His Pro Leu Tyr Val Asp Phe Ser Asp Val Gly Trp Asn
1               5                   10                  15

Asp Trp Ile Val Ala Pro Pro Gly Tyr His Ala Phe Tyr Cys His Gly
            20                  25                  30

Glu Cys Pro Phe Pro Leu Ala Asp His Leu Asn Ser Thr Asn His Ala
        35                  40                  45

Ile Val Gln Thr Leu Val Asn Ser Val Asn Ser Lys Ile Pro Lys Ala
    50                  55                  60

Cys Cys Val Pro Thr Glu Leu Ser Ala Ile Ser Met Leu Tyr Leu Asp
65                  70                  75                  80

Glu Asn Glu Lys Val Val Leu Lys Asn Tyr Gln Asp Met Val Val Glu
                85                  90                  95

Gly Cys Gly Cys Arg
            100

(2) INFORMATION FOR SEQ ID NO:10:

(i) SEQUENCE CHARACTERISTICS:
(A) LENGTH: 101 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: HOMO SAPIENS
(F) TISSUE TYPE: hippocampus

(ix) FEATURE:
(A) NAME/KEY: Protein
(B) LOCATION: 1..101
(D) OTHER INFORMATION: /label= CBMP-2B-FX

(xi) SEQUENCE DESCRIPTION: SEQ ID NO:10:

Cys Arg Arg His Ser Leu Tyr Val Asp Phe Ser Asp Val Gly Trp Asn
1               5                   10                  15

Asp Trp Ile Val Ala Pro Pro Gly Tyr Gln Ala Phe Tyr Cys His Gly
            20                  25                  30

Asp Cys Pro Phe Pro Leu Ala Asp His Leu Asn Ser Thr Asn His Ala
        35                  40                  45

Ile Val Gln Thr Leu Val Asn Ser Val Asn Ser Ser Ile Pro Lys Ala
    50                  55                  60

Cys Cys Val Pro Thr Glu Leu Ser Ala Ile Ser Met Leu Tyr Leu Asp
65                  70                  75                  80

Glu Tyr Asp Lys Val Val Leu Lys Asn Tyr Gln Glu Met Val Val Glu
                85                  90                  95

Gly Cys Gly Cys Arg
            100

(2) INFORMATION FOR SEQ ID NO:11:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: DROSOPHILA MELANOGASTER

(ix) FEATURE:
(A) NAME/KEY: Protein
(B) LOCATION: 1..101
(D) OTHER INFORMATION: /label= DPP-FX

(xi) SEQUENCE DESCRIPTION: SEQ ID NO:11:

Cys Arg Arg His Ser Leu Tyr Val Asp Phe Ser Asp Val Gly Trp Asp
1               5                   10                  15

Asp Trp Ile Val Ala Pro Leu Gly Tyr Asp Ala Tyr Tyr Cys His Gly
            20                  25                  30

Lys Cys Pro Phe Pro Leu Ala Asp His Phe Asn Ser Thr Asn His Ala
        35                  40                  45

Val Val Gln Thr Leu Val Asn Asn Asn Asn Pro Gly Lys Val Pro Lys
    50                  55                  60

Ala Cys Cys Val Pro Thr Gln Leu Asp Ser Val Ala Met Leu Tyr Leu
65                  70                  75                  80

Asn Asp Gln Ser Thr Val Val Leu Lys Asn Tyr Gln Glu Met Thr Val
                85                  90                  95

Val Gly Cys Gly Cys Arg
            100

(2) INFORMATION FOR SEQ ID NO:12:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: XENOPUS

(ix) FEATURE:
(A) NAME/KEY: Protein
(B) LOCATION: 1..102
(D) OTHER INFORMATION: /label= VGL-FX

(xi) SEQUENCE DESCRIPTION: SEQ ID NO:12:

Cys Lys Lys Arg His Leu Tyr Val Glu Phe Lys Asp Val Gly Trp Gln
1               5                   10                  15

Asn Trp Val Ile Ala Pro Gln Gly Tyr Met Ala Asn Tyr Cys Tyr Gly
            20                  25                  30

Glu Cys Pro Tyr Pro Leu Thr Glu Ile Leu Asn Gly Ser Asn His Ala
        35                  40                  45

Ile Leu Gln Thr Leu Val His Ser Ile Glu Pro Glu Asp Ile Pro Leu
    50                  55                  60

Pro Cys Cys Val Pro Thr Lys Met Ser Pro Ile Ser Met Leu Phe Tyr
65                  70                  75                  80

Asp Asn Asn Asp Asn Val Val Leu Arg His Tyr Glu Asn Met Ala Val
                85                  90                  95

Asp Glu Cys Gly Cys Arg
            100

(2) INFORMATION FOR SEQ ID NO:13:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: MURIDAE

(ix) FEATURE:
(A) NAME/KEY: Protein
(B) LOCATION: 1..102
(D) OTHER INFORMATION: /label= VGR-1-FX

(xi) SEQUENCE DESCRIPTION: SEQ ID NO:13:

Cys Lys Lys His Glu Leu Tyr Val Ser Phe Gln Asp Val Gly Trp Gln
1               5                   10                  15

Asp Trp Ile Ile Ala Pro Lys Gly Tyr Ala Ala Asn Tyr Cys Asp Gly
            20                  25                  30

Glu Cys Ser Phe Pro Leu Asn Ala His Met Asn Ala Thr Asn His Ala
        35                  40                  45

Ile Val Gln Thr Leu Val His Val Met Asn Pro Glu Tyr Val Pro Lys
    50                  55                  60

Pro Cys Cys Ala Pro Thr Lys Val Asn Ala Ile Ser Val Leu Tyr Phe
65                  70                  75                  80

Asp Asp Asn Ser Asn Val Ile Leu Lys Lys Tyr Arg Asn Met Val Val
                85                  90                  95

Arg Ala Cys Gly Cys His
            100

(2) INFORMATION FOR SEQ ID NO:14:

(i) SEQUENCE CHARACTERISTICS:
(A) LENGTH: 106 amino acids
(B) TYPE: amino acid
(C) STRANDEDNESS: single
(D) TOPOLOGY: linear

(ii) MOLECULE TYPE: protein

(iii) HYPOTHETICAL: NO

(iv) ANTI-SENSE: NO

(vi) ORIGINAL SOURCE:
(A) ORGANISM: Homo sapiens
(F) TISSUE TYPE: brain

(ix) FEATURE:
(A) NAME/KEY: Protein
(B) LOCATION: 1..106
(D) OTHER INFORMATION: /note= "GDF-1 (fx)"

(xi) SEQUENCE DESCRIPTION: SEQ ID NO:14:

Cys Arg Ala Arg Arg Leu Tyr Val Ser Phe Arg Glu Val Gly Trp His
1               5                   10                  15

Arg Trp Val Ile Ala Pro Arg Gly Phe Leu Ala Asn Tyr Cys Gln Gly
            20                  25                  30

Gln Cys Ala Leu Pro Val Ala Leu Ser Gly Ser Gly Gly Pro Pro Ala
        35                  40                  45

Leu Asn His Ala Val Leu Arg Ala Leu Met His Ala Ala Ala Pro Gly
    50                  55                  60

Ala Ala Asp Leu Pro Cys Cys Val Pro Ala Arg Leu Ser Pro Ile Ser
65                  70                  75                  80

Val Leu Phe Phe Asp Asn Ser Asp Asn Val Val Leu Arg Gln Tyr Glu
                85                  90                  95

Asp Met Val Val Asp Glu Cys Gly Cys Arg
            100                 105

(2) INFORMATION FOR SEQ ID NO:15:

(i) SEQUENCE CHARACTERISTICS:
(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:15:

Cys Xaa Xaa Xaa Xaa
1               5

(2) INFORMATION FOR SEQ ID NO:16:

(i) SEQUENCE CHARACTERISTICS:
(A) LENGTH: 1822 base pairs
(B) TYPE: nucleic acid
(C) STRANDEDNESS: single
(D) TOPOLOGY: linear

(ii) MOLECULE TYPE: cDNA

(iii) HYPOTHETICAL: NO

(iv) ANTI-SENSE: NO

(vi) ORIGINAL SOURCE:
(A) ORGANISM: HOMO SAPIENS
(F) TISSUE TYPE: HIPPOCAMPUS

(ix) FEATURE:
(A) NAME/KEY: CDS
(B) LOCATION: 49..1341
(C) IDENTIFICATION METHOD: experimental
(D) OTHER INFORMATION: /function= "OSTEOGENIC PROTEIN"
/product= "OP1"
/evidence= EXPERIMENTAL
/standard_name= "OP1"

(xi) SEQUENCE DESCRIPTION: SEQ ID NO:16:

GGTGCGGGCC CGGAGCCCGG AGCCCGGGTA GCGCGTAGAG CCGGCGCG ATG CAC GTG 57
Met His Val
1

CGC TCA CTG CGA GCT GCG GCG CCG CAC AGC TTC GTG GCG CTC TGG GCA 105
Arg Ser Leu Arg Ala Ala Ala Pro His Ser Phe Val Ala Leu Trp Ala
    5                   10                  15

CCC CTG TTC CTG CTG CGC TCC GCC CTG GCC GAC TTC AGC CTG GAC AAC 153
Pro Leu Phe Leu Leu Arg Ser Ala Leu Ala Asp Phe Ser Leu Asp Asn
20                  25                  30                  35

GAG GTG CAC TCG AGC TTC ATC CAC CGG CGC CTC CGC AGC CAG GAG CGG 201
Glu Val His Ser Ser Phe Ile His Arg Arg Leu Arg Ser Gln Glu Arg
                40                  45                  50

CGG GAG ATG CAG CGC GAG ATC CTC TCC ATT TTG GGC TTG CCC CAC CGC 249
Arg Glu Met Gln Arg Glu Ile Leu Ser Ile Leu Gly Leu Pro His Arg
            55                  60                  65

CCG CGC CCG CAC CTC CAG GGC AAG CAC AAC TCG GCA CCC ATG TTC ATG 297
Pro Arg Pro His Leu Gln Gly Lys His Asn Ser Ala Pro Met Phe Met
        70                  75                  80

CTG GAC CTG TAC AAC GCC ATG GCG GTG GAG GAG GGC GGC GGG CCC GGC 345
Leu Asp Leu Tyr Asn Ala Met Ala Val Glu Glu Gly Gly Gly Pro Gly
    85                  90                  95

GGC CAG GGC TTC TCC TAC CCC TAC AAG GCC GTC TTC AGT ACC CAG GGC 393
Gly Gln Gly Phe Ser Tyr Pro Tyr Lys Ala Val Phe Ser Thr Gln Gly
100                 105                 110                 115

CCC CCT CTG GCC AGC CTG CAA GAT AGC CAT TTC CTC ACC GAC GCC GAC 441
Pro Pro Leu Ala Ser Leu Gln Asp Ser His Phe Leu Thr Asp Ala Asp
                120                 125                 130

ATG GTC ATG AGC TTC GTC AAC CTC GTG GAA CAT GAC AAG GAA TTC TTC 489
Met Val Met Ser Phe Val Asn Leu Val Glu His Asp Lys Glu Phe Phe
            135                 140                 145

CAC CCA CGC TAC CAC CAT CGA GAG TTC CGG TTT GAT CTT TCC AAG ATC 537
His Pro Arg Tyr His His Arg Glu Phe Arg Phe Asp Leu Ser Lys Ile
        150                 155                 160

CCA GAA GGG GAA GCT GTC ACG GCA GCC GAA TTC CGG ATC TAC AAG GAC 585
Pro Glu Gly Glu Ala Val Thr Ala Ala Glu Phe Arg Ile Tyr Lys Asp
    165                 170                 175

TAC ATC CGG GAA CGC TTC GAC AAT GAG ACG TTC CGG ATC AGC GTT TAT 633
Tyr Ile Arg Glu Arg Phe Asp Asn Glu Thr Phe Arg Ile Ser Val Tyr
180                 185                 190                 195

CAG GTG CTC CAG GAG CAC TTG GGC AGG GAA TCG GAT CTC TTC CTG CTC 681
Gln Val Leu Gln Glu His Leu Gly Arg Glu Ser Asp Leu Phe Leu Leu
                200                 205                 210

GAC AGC CGT ACC CTC TGG GCC TCG GAG GAG GGC TGG CTG GTG TTT GAC 729
Asp Ser Arg Thr Leu Trp Ala Ser Glu Glu Gly Trp Leu Val Phe Asp
            215                 220                 225

ATC ACA GCC ACC AGC AAC CAC TGG GTG GTC AAT CCG CGG CAC AAC CTG 777
Ile Thr Ala Thr Ser Asn His Trp Val Val Asn Pro Arg His Asn Leu
        230                 235                 240

GGC CTG CAG CTC TCG GTG GAG ACG CTG GAT GGG CAG AGC ATC AAC CCC 825
Gly Leu Gln Leu Ser Val Glu Thr Leu Asp Gly Gln Ser Ile Asn Pro
    245                 250                 255

AAG TTG GCG GGC CTG ATT GGG CGG CAC GGG CCC CAG AAC AAG CAG CCC 873
Lys Leu Ala Gly Leu Ile Gly Arg His Gly Pro Gln Asn Lys Gln Pro
260                 265                 270                 275

TTC ATG GTG GCT TTC TTC AAG GCC ACG GAG GTC CAC TTC CGC AGC ATC 921
Phe Met Val Ala Phe Phe Lys Ala Thr Glu Val His Phe Arg Ser Ile
                280                 285                 290

CGG TCC ACG GGG AGC AAA CAG CGC AGC CAG AAC CGC TCC AAG ACG CCC 969
Arg Ser Thr Gly Ser Lys Gln Arg Ser Gln Asn Arg Ser Lys Thr Pro
            295                 300                 305

AAG AAC CAG GAA GCC CTG CGG ATG GCC AAC GTG GCA GAG AAC AGC AGC 1017
Lys Asn Gln Glu Ala Leu Arg Met Ala Asn Val Ala Glu Asn Ser Ser
        310                 315                 320

AGC GAC CAG AGG CAG GCC TGT AAG AAG CAC GAG CTG TAT GTC AGC TTC 1065
Ser Asp Gln Arg Gln Ala Cys Lys Lys His Glu Leu Tyr Val Ser Phe
    325                 330                 335

CGA GAC CTG GGC TGG CAG GAC TGG ATC ATC GCG CCT GAA GGC TAC GCC 1113
Arg Asp Leu Gly Trp Gln Asp Trp Ile Ile Ala Pro Glu Gly Tyr Ala
340                 345                 350                 355

GCC TAC TAC TGT GAG GGG GAG TGT GCC TTC CCT CTG AAC TCC TAC ATG 1161
Ala Tyr Tyr Cys Glu Gly Glu Cys Ala Phe Pro Leu Asn Ser Tyr Met
                360                 365                 370

AAC GCC ACC AAC CAC GCC ATC GTG CAG ACG CTG GTC CAC TTC ATC AAC 1209
Asn Ala Thr Asn His Ala Ile Val Gln Thr Leu Val His Phe Ile Asn
            375                 380                 385

CCG GAA ACG GTG CCC AAG CCC TGC TGT GCG CCC ACG CAG CTC AAT GCC 1257
Pro Glu Thr Val Pro Lys Pro Cys Cys Ala Pro Thr Gln Leu Asn Ala
        390                 395                 400

ATC TCC GTC CTC TAC TTC GAT GAC AGC TCC AAC GTC ATC CTG AAG AAA 1305
Ile Ser Val Leu Tyr Phe Asp Asp Ser Ser Asn Val Ile Leu Lys Lys
    405                 410                 415

TAC AGA AAC ATG GTG GTC CGG GCC TGT GGC TGC CAC TAGCTCCTCC 1351
Tyr Arg Asn Met Val Val Arg Ala Cys Gly Cys His
420                 425                 430

GAGAATTCAG ACCCTTTGGG GCCAAGTTTT TCTGGATCCT CCATTGCTCG CCTTGGCCAG 1411

GAACCAGCAG ACCAACTGCC TTTTGTGAGA CCTTCCCCTC CCTATCCCCA ACTTTAAAGG 1471

TGTGAGAGTA TTAGGAAACA TGAGCAGCAT ATGGCTTTTG ATCAGTTTTT CAGTGGCAGC 1531

ATCCAATGAA CAAGATCCTA CAAGCTGTGC AGGCAAAACC TAGCAGGAAA AAAAAACAAC 1591

GCATAAAGAA AAATGGCCGG GCCAGGTCAT TGGCTGGGAA GTCTCAGCCA TGCACGGACT 1651

CGTTTCCAGA GGTAATTATG AGCGCCTACC AGCCAGGCCA CCCAGCCGTG GGAGGAAGGG 1711

GGCGTGGCAA GGGGTGGGCA CATTGGTGTC TGTGCGAAAG GAAAATTGAC CCGGAAGTTC 1771

CTGTAATAAA TGTCACAATA AAACGAATGA ATGAAAAAAA AAAAAAAAAA A 1822

(2) INFORMATION FOR SEQ ID NO:17:

(i) SEQUENCE CHARACTERISTICS:
(A) LENGTH: 431 amino acids
(B) TYPE: amino acid
(D) TOPOLOGY: linear

(ii) MOLECULE TYPE: protein

(xi) SEQUENCE DESCRIPTION: SEQ ID NO:17:

Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Gly Gly
                85                  90                  95

Gly Pro Gly Gly Gln Gly Phe Ser Tyr Pro Tyr Lys Ala Val Phe Ser
            100                 105                 110

Thr Gln Gly Pro Pro Leu Ala Ser Leu Gln Asp Ser His Phe Leu Thr
        115                 120                 125

Asp Ala Asp Met Val Met Ser Phe Val Asn Leu Val Glu His Asp Lys
    130                 135                 140

Glu Phe Phe His Pro Arg Tyr His His Arg Glu Phe Arg Phe Asp Leu
145                 150                 155                 160

Ser Lys Ile Pro Glu Gly Glu Ala Val Thr Ala Ala Glu Phe Arg Ile
                165                 170                 175

Tyr Lys Asp Tyr Ile Arg Glu Arg Phe Asp Asn Glu Thr Phe Arg Ile
            180                 185                 190

Ser Val Tyr Gln Val Leu Gln Glu His Leu Gly Arg Glu Ser Asp Leu
        195                 200                 205

Phe Leu Leu Asp Ser Arg Thr Leu Trp Ala Ser Glu Glu Gly Trp Leu
    210                 215                 220

Val Phe Asp Ile Thr Ala Thr Ser Asn His Trp Val Val Asn Pro Arg
225                 230                 235                 240

His Asn Leu Gly Leu Gln Leu Ser Val Glu Thr Leu Asp Gly Gln Ser
                245                 250                 255

Ile Asn Pro Lys Leu Ala Gly Leu Ile Gly Arg His Gly Pro Gln Asn
            260                 265                 270

Lys Gln Pro Phe Met Val Ala Phe Phe Lys Ala Thr Glu Val His Phe
        275                 280                 285

Arg Ser Ile Arg Ser Thr Gly Ser Lys Gln Arg Ser Gln Asn Arg Ser
    290                 295                 300

Lys Thr Pro Lys Asn Gln Glu Ala Leu Arg Met Ala Asn Val Ala Glu
305                 310                 315                 320

Asn Ser Ser Ser Asp Gln Arg Gln Ala Cys Lys Lys His Glu Leu Tyr
                325                 330                 335

Val Ser Phe Arg Asp Leu Gly Trp Gln Asp Trp Ile Ile Ala Pro Glu
            340                 345                 350

Gly Tyr Ala Ala Tyr Tyr Cys Glu Gly Glu Cys Ala Phe Pro Leu Asn
        355                 360                 365

Ser Tyr Met Asn Ala Thr Asn His Ala Ile Val Gln Thr Leu Val His
    370                 375                 380

Phe Ile Asn Pro Glu Thr Val Pro Lys Pro Cys Cys Ala Pro Thr Gln
385                 390                 395                 400

Leu Asn Ala Ile Ser Val Leu Tyr Phe Asp Asp Ser Ser Asn Val Ile
                405                 410                 415

Leu Lys Lys Tyr Arg Asn Met Val Val Arg Ala Cys Gly Cys His
            420                 425                 430

(2) INFORMATION FOR SEQ ID NO:18:

     (i) SEQUENCE CHARACTERISTICS:
          (A) LENGTH: 1873 base pairs
          (B) TYPE: nucleic acid
          (C) STRANDEDNESS: single
          (D) TOPOLOGY: linear

    (ii) MOLECULE TYPE: cDNA

   (iii) HYPOTHETICAL: NO

    (iv) ANTI-SENSE: NO

    (vi) ORIGINAL SOURCE:
          (A) ORGANISM: MURIDAE
          (F) TISSUE TYPE: EMBRYO

    (ix) FEATURE:
          (A) NAME/KEY: CDS
          (B) LOCATION: 104..1393
          (D) OTHER INFORMATION: /function= "OSTEOGENIC PROTEIN"
                /product= "MOP1"
                /note= "MOP1 (CDNA)"

    (xi) SEQUENCE DESCRIPTION: SEQ ID NO:18:

CTGCAGCAAG TGACCTCGGG TCGTGGACCG CTGCCCTGCC CCCTCCGCTG CCACCTGGGG      60

CGGCGCGGGC CCGGTGCCCC GGATCGCGCG TAGAGCCGGC GCG ATG CAC GTG CGC      115
                                                Met His Val Arg
                                                  1

TCG CTG CGC GCT GCG GCG CCA CAC AGC TTC GTG GCG CTC TGG GCG CCT      163
Ser Leu Arg Ala Ala Ala Pro His Ser Phe Val Ala Leu Trp Ala Pro
  5                  10                  15                  20

CTG TTC TTG CTG CGC TCC GCC CTG GCC GAT TTC AGC CTG GAC AAC GAG      211
Leu Phe Leu Leu Arg Ser Ala Leu Ala Asp Phe Ser Leu Asp Asn Glu
                 25                  30                  35

GTG CAC TCC AGC TTC ATC CAC CGG CGC CTC CGC AGC CAG GAG CGG CGG 259
Val His Ser Ser Phe Ile His Arg Arg Leu Arg Ser Gln Glu Arg Arg
            40                  45                  50

GAG ATG CAG CGG GAG ATC CTG TCC ATC TTA GGG TTG CCC CAT CGC CCG 307
Glu Met Gln Arg Glu Ile Leu Ser Ile Leu Gly Leu Pro His Arg Pro
        55                  60                  65

CGC CCG CAC CTC CAG GGA AAG CAT AAT TCG GCG CCC ATG TTC ATG TTG 355
Arg Pro His Leu Gln Gly Lys His Asn Ser Ala Pro Met Phe Met Leu
    70                  75                  80

GAC CTG TAC AAC GCC ATG GCG GTG GAG GAG AGC GGG CCG GAC GGA CAG 403
Asp Leu Tyr Asn Ala Met Ala Val Glu Glu Ser Gly Pro Asp Gly Gln
85                  90                  95                  100

GGC TTC TCC TAC CCC TAC AAG GCC GTC TTC AGT ACC CAG GGC CCC CCT 451
Gly Phe Ser Tyr Pro Tyr Lys Ala Val Phe Ser Thr Gln Gly Pro Pro
                105                 110                 115

TTA GCC AGC CTG CAG GAC AGC CAT TTC CTC ACT GAC GCC GAC ATG GTC 499
Leu Ala Ser Leu Gln Asp Ser His Phe Leu Thr Asp Ala Asp Met Val
            120                 125                 130

ATG AGC TTC GTC AAC CTA GTG GAA CAT GAC AAA GAA TTC TTC CAC CCT 547
Met Ser Phe Val Asn Leu Val Glu His Asp Lys Glu Phe Phe His Pro
        135                 140                 145

CGA TAC CAC CAT CGG GAG TTC CGG TTT GAT CTT TCC AAG ATC CCC GAG 595
Arg Tyr His His Arg Glu Phe Arg Phe Asp Leu Ser Lys Ile Pro Glu
    150                 155                 160

GGC GAA CGG GTG ACC GCA GCC GAA TTC AGG ATC TAT AAG GAC TAC ATC 643
Gly Glu Arg Val Thr Ala Ala Glu Phe Arg Ile Tyr Lys Asp Tyr Ile
165                 170                 175                 180

CGG GAG CGA TTT GAC AAC GAG ACC TTC CAG ATC ACA GTC TAT CAG GTG 691
Arg Glu Arg Phe Asp Asn Glu Thr Phe Gln Ile Thr Val Tyr Gln Val
                185                 190                 195

CTC CAG GAG CAC TCA GGC AGG GAG TCG GAC CTC TTC TTG CTG GAC AGC 739
Leu Gln Glu His Ser Gly Arg Glu Ser Asp Leu Phe Leu Leu Asp Ser
            200                 205                 210

CGC ACC ATC TGG GCT TCT GAG GAG GGC TGG TTG GTG TTT GAT ATC ACA 787
Arg Thr Ile Trp Ala Ser Glu Glu Gly Trp Leu Val Phe Asp Ile Thr
        215                 220                 225

GCC ACC AGC AAC CAC TGG GTG GTC AAC CCT CGG CAC AAC CTG GGC TTA 835
Ala Thr Ser Asn His Trp Val Val Asn Pro Arg His Asn Leu Gly Leu
    230                 235                 240

CAG CTC TCT GTG GAG ACC CTG GAT GGG CAG AGC ATC AAC CCC AAG TTG 883
Gln Leu Ser Val Glu Thr Leu Asp Gly Gln Ser Ile Asn Pro Lys Leu
245                 250                 255                 260

GCA GGC CTG ATT GGA CGG CAT GGA CCC CAG AAC AAG CAA CCC TTC ATG 931
Ala Gly Leu Ile Gly Arg His Gly Pro Gln Asn Lys Gln Pro Phe Met
                265                 270                 275

GTG GCC TTC TTC AAG GCC ACG GAA GTC CAT CTC CGT AGT ATC CGG TCC 979
Val Ala Phe Phe Lys Ala Thr Glu Val His Leu Arg Ser Ile Arg Ser
            280                 285                 290

ACG GGG GGC AAG CAG CGC AGC CAG AAT CGC TCC AAG ACG CCA AAG AAC 1027
Thr Gly Gly Lys Gln Arg Ser Gln Asn Arg Ser Lys Thr Pro Lys Asn
        295                 300                 305

CAA GAG GCC CTG AGG ATG GCC AGT GTG GCA GAA AAC AGC AGC AGT GAC 1075
Gln Glu Ala Leu Arg Met Ala Ser Val Ala Glu Asn Ser Ser Ser Asp
    310                 315                 320

CAG AGG CAG GCC TGC AAG AAA CAT GAG CTG TAC GTC AGC TTC CGA GAC 1123
Gln Arg Gln Ala Cys Lys Lys His Glu Leu Tyr Val Ser Phe Arg Asp
325                 330                 335                 340

CTT GGC TGG CAG GAC TGG ATC ATT GCA CCT GAA GGC TAT GCT GCC TAC 1171
Leu Gly Trp Gln Asp Trp Ile Ile Ala Pro Glu Gly Tyr Ala Ala Tyr
                345                 350                 355

TAC TGT GAG GGA GAG TGC GCC TTC CCT CTG AAC TCC TAC ATG AAC GCC 1219
Tyr Cys Glu Gly Glu Cys Ala Phe Pro Leu Asn Ser Tyr Met Asn Ala
            360                 365                 370

ACC AAC CAC GCC ATC GTC CAG ACA CTG GTT CAC TTC ATC AAC CCA GAC 1267
Thr Asn His Ala Ile Val Gln Thr Leu Val His Phe Ile Asn Pro Asp
        375                 380                 385

ACA GTA CCC AAG CCC TGC TGT GCG CCC ACC CAG CTC AAC GCC ATC TCT 1315
Thr Val Pro Lys Pro Cys Cys Ala Pro Thr Gln Leu Asn Ala Ile Ser
    390                 395                 400

GTC CTC TAC TTC GAC GAC AGC TCT AAT GTC GAC CTG AAG AAG TAC AGA 1363
Val Leu Tyr Phe Asp Asp Ser Ser Asn Val Asp Leu Lys Lys Tyr Arg
405                 410                 415                 420

AAC ATG GTG GTC CGG GCC TGT GGC TGC CAC TAGCTCTTCC TGAGACCCTG 1413
Asn Met Val Val Arg Ala Cys Gly Cys His
                425                 430

ACCTTTGCGG GGCCACACCT TTCCAAATCT TCGATGTCTC ACCATCTAAG TCTCTCACTG 1473

CCCACCTTGG CGAGGAGAAC AGACCAACCT CTCCTGAGCC TTCCCTCACC TCCCAACCGG 1533

AAGCATGTAA GGGTTCCAGA AACCTGAGCG TGCAGCAGCT GATGAGCGCC CTTTCCTTCT 1593

GGCACGTGAC GGACAAGATC CTACCAGCTA CCACAGCAAA CGCCTAAGAG CAGGAAAAAT 1653

GTCTGCCAGG AAAGTGTCCA GTGTCCACAT GGCCCCTGGC GCTCTGAGTC TTTGAGGAGT 1713

AATCGCAAGC CTCGTTCAGC TGCAGCAGAA GGAAGGGCTT AGCCAGGGTG GGCGCTGGCG 1773

TCTGTGTTGA AGGGAAACCA AGCAGAAGCC ACTGTAATGA TATGTCACAA TAAAACCCAT 1833

GAATGAAAAA AAAAAAAAAA AAAAAAAAAA AAAAGAATTC 1873

(2) INFORMATION FOR SEQ ID NO:19:

(i) SEQUENCE CHARACTERISTICS:
(A) LENGTH: 430 amino acids
(B) TYPE: amino acid
(D) TOPOLOGY: linear

(ii) MOLECULE TYPE: protein

(xi) SEQUENCE DESCRIPTION: SEQ ID NO:19:

Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Ser Gly
                85                  90                  95

Pro Asp Gly Gln Gly Phe Ser Tyr Pro Tyr Lys Ala Val Phe Ser Thr
            100                 105                 110

Gln Gly Pro Pro Leu Ala Ser Leu Gln Asp Ser His Phe Leu Thr Asp
        115                 120                 125

Ala Asp Met Val Met Ser Phe Val Asn Leu Val Glu His Asp Lys Glu
    130                 135                 140

Phe Phe His Pro Arg Tyr His His Arg Glu Phe Arg Phe Asp Leu Ser
145                 150                 155                 160

Lys Ile Pro Glu Gly Glu Arg Val Thr Ala Ala Glu Phe Arg Ile Tyr
                165                 170                 175

Lys Asp Tyr Ile Arg Glu Arg Phe Asp Asn Glu Thr Phe Gln Ile Thr
            180                 185                 190

Val Tyr Gln Val Leu Gln Glu His Ser Gly Arg Glu Ser Asp Leu Phe
        195                 200                 205

Leu Leu Asp Ser Arg Thr Ile Trp Ala Ser Glu Glu Gly Trp Leu Val
    210                 215                 220

Phe Asp Ile Thr Ala Thr Ser Asn His Trp Val Val Asn Pro Arg His
225                 230                 235                 240

Asn Leu Gly Leu Gln Leu Ser Val Glu Thr Leu Asp Gly Gln Ser Ile
                245                 250                 255

Asn Pro Lys Leu Ala Gly Leu Ile Gly Arg His Gly Pro Gln Asn Lys
            260                 265                 270

Gln Pro Phe Met Val Ala Phe Phe Lys Ala Thr Glu Val His Leu Arg
        275                 280                 285

Ser Ile Arg Ser Thr Gly Gly Lys Gln Arg Ser Gln Asn Arg Ser Lys
    290                 295                 300

Thr Pro Lys Asn Gln Glu Ala Leu Arg Met Ala Ser Val Ala Glu Asn
305                 310                 315                 320

Ser Ser Ser Asp Gln Arg Gln Ala Cys Lys Lys His Glu Leu Tyr Val
                325                 330                 335

Ser Phe Arg Asp Leu Gly Trp Gln Asp Trp Ile Ile Ala Pro Glu Gly
            340                 345                 350

Tyr Ala Ala Tyr Tyr Cys Glu Gly Glu Cys Ala Phe Pro Leu Asn Ser
        355                 360                 365

Tyr Met Asn Ala Thr Asn His Ala Ile Val Gln Thr Leu Val His Phe
    370                 375                 380

Ile Asn Pro Asp Thr Val Pro Lys Pro Cys Cys Ala Pro Thr Gln Leu
385                 390                 395                 400

Asn Ala Ile Ser Val Leu Tyr Phe Asp Asp Ser Ser Asn Val Asp Leu
                405                 410                 415

Lys Lys Tyr Arg Asn Met Val Val Arg Ala Cys Gly Cys His
            420                 425                 430

(2) INFORMATION FOR SEQ ID NO:20:

     (i) SEQUENCE CHARACTERISTICS:
          (A) LENGTH: 1723 base pairs
          (B) TYPE: nucleic acid
          (C) STRANDEDNESS: single
          (D) TOPOLOGY: linear

    (ii) MOLECULE TYPE: cDNA

    (vi) ORIGINAL SOURCE:
          (A) ORGANISM: Homo sapiens
          (F) TISSUE TYPE: HIPPOCAMPUS

(ix) FEATURE:
(A) NAME/KEY: CDS
(B) LOCATION: 490..1696
(D) OTHER INFORMATION: /function= "OSTEOGENIC PROTEIN"
/product= "hOP2-PP"
/note= "hOP2 (cDNA)"

(xi) SEQUENCE DESCRIPTION: SEQ ID NO:20:

GGCGCCGGCA GAGCAGGAGT GGCTGGAGGA GCTGTGGTTG GAGCAGGAGG TGGCACGGCA 60

GGGCTGGAGG GCTCCCTATG AGTGGCGGAG ACGGCCCAGG AGGCGCTGGA GCAACAGCTC 120

CCACACCGCA CCAAGCGGTG GCTGCAGGAG CTCGCCCATC GCCCCTGCGC TGCTCGGACC 180

GCGGCCACAG CCGGACTGGC GGGTACGGCG GCGACAGAGG CATTGGCCGA GAGTCCCAGT 240

CCGCAGAGTA GCCCCGGCCT CGAGGCGGTG GCGTCCCGGT CCTCTCCGTC CAGGAGCCAG 300

GACAGGTGTC GCGCGGCGGG GCTCCAGGGA CCGCGCCTGA GGCCGGCTGC CCGCCCGTCC 360

CGCCCCGCCC CGCCGCCCGC CGCCCGCCGA GCCCAGCCTC CTTGCCGTCG GGGCGTCCCC 420

AGGCCCTGGG TCGGCCGCGG AGCCGATGCG CGCCCGCTGA GCGCCCCAGC TGAGCGCCCC 480

CGGCCTGCC ATG ACC GCG CTC CCC GGC CCG CTC TGG CTC CTG GGC CTG 528
Met Thr Ala Leu Pro Gly Pro Leu Trp Leu Leu Gly Leu
1               5                   10

GCG CTA TGC GCG CTG GGC GGG GGC GGC CCC GGC CTG CGA CCC CCG CCC 576
Ala Leu Cys Ala Leu Gly Gly Gly Gly Pro Gly Leu Arg Pro Pro Pro
    15                  20                  25

GGC TGT CCC CAG CGA CGT CTG GGC GCG CGC GAG CGC CGG GAC GTG CAG 624
Gly Cys Pro Gln Arg Arg Leu Gly Ala Arg Glu Arg Arg Asp Val Gln
30                  35                  40                  45

CGC GAG ATC CTG GCG GTG CTC GGG CTG CCT GGG CGG CCC CGG CCC CGC 672
Arg Glu Ile Leu Ala Val Leu Gly Leu Pro Gly Arg Pro Arg Pro Arg
                50                  55                  60

GCG CCA CCC GCC GCC TCC CGG CTG CCC GCG TCC GCG CCG CTC TTC ATG 720
Ala Pro Pro Ala Ala Ser Arg Leu Pro Ala Ser Ala Pro Leu Phe Met
            65                  70                  75

CTG GAC CTG TAC CAC GCC ATG GCC GGC GAC GAC GAC GAG GAC GGC GCG 768
Leu Asp Leu Tyr His Ala Met Ala Gly Asp Asp Asp Glu Asp Gly Ala
        80                  85                  90

CCC GCG GAG CGG CGC CTG GGC CGC GCC GAC CTG GTC ATG AGC TTC GTT 816
Pro Ala Glu Arg Arg Leu Gly Arg Ala Asp Leu Val Met Ser Phe Val
    95                  100                 105

AAC ATG GTG GAG CGA GAC CGT GCC CTG GGC CAC CAG GAG CCC CAT TGG 864
Asn Met Val Glu Arg Asp Arg Ala Leu Gly His Gln Glu Pro His Trp
110                 115                 120                 125

AAG GAG TTC CGC TTT GAC CTG ACC CAG ATC CCG GCT GGG GAG GCG GTC 912
Lys Glu Phe Arg Phe Asp Leu Thr Gln Ile Pro Ala Gly Glu Ala Val
                130                 135                 140

ACA GCT GCG GAG TTC CGG ATT TAC AAG GTG CCC AGC ATC CAC CTG CTC 960
Thr Ala Ala Glu Phe Arg Ile Tyr Lys Val Pro Ser Ile His Leu Leu
            145                 150                 155

AAC AGG ACC CTC CAC GTC AGC ATG TTC CAG GTG GTC CAG GAG CAG TCC 1008
Asn Arg Thr Leu His Val Ser Met Phe Gln Val Val Gln Glu Gln Ser
        160                 165                 170

AAC AGG GAG TCT GAC TTG TTC TTT TTG GAT CTT CAG ACG CTC CGA GCT 1056
Asn Arg Glu Ser Asp Leu Phe Phe Leu Asp Leu Gln Thr Leu Arg Ala
    175                 180                 185

GGA GAC GAG GGC TGG CTG GTG CTG GAT GTC ACA GCA GCC AGT GAC TGC 1104
Gly Asp Glu Gly Trp Leu Val Leu Asp Val Thr Ala Ala Ser Asp Cys
190                 195                 200                 205

TGG TTG CTG AAG CGT CAC AAG GAC CTG GGA CTC CGC CTC TAT GTG GAG 1152
Trp Leu Leu Lys Arg His Lys Asp Leu Gly Leu Arg Leu Tyr Val Glu
                210                 215                 220

ACT GAG GAC GGG CAC AGC GTG GAT CCT GGC CTG GCC GGC CTG CTG GGT 1200
Thr Glu Asp Gly His Ser Val Asp Pro Gly Leu Ala Gly Leu Leu Gly
            225                 230                 235

CAA CGG GCC CCA CGC TCC CAA CAG CCT TTC GTG GTC ACT TTC TTC AGG 1248
Gln Arg Ala Pro Arg Ser Gln Gln Pro Phe Val Val Thr Phe Phe Arg
        240                 245                 250

GCC AGT CCG AGT CCC ATC CGC ACC CCT CGG GCA GTG AGG CCA CTG AGG 1296
Ala Ser Pro Ser Pro Ile Arg Thr Pro Arg Ala Val Arg Pro Leu Arg
    255                 260                 265

AGG AGG CAG CCG AAG AAA AGC AAC GAG CTG CCG CAG GCC AAC CGA CTC 1344
Arg Arg Gln Pro Lys Lys Ser Asn Glu Leu Pro Gln Ala Asn Arg Leu
270                 275                 280                 285

CCA GGG ATC TTT GAT GAC GTC CAC GGC TCC CAC GGC CGG CAG GTC TGC 1392
Pro Gly Ile Phe Asp Asp Val His Gly Ser His Gly Arg Gln Val Cys
                290                 295                 300

CGT CGG CAC GAG CTC TAC GTC AGC TTC CAG GAC CTC GGC TGG CTG GAC 1440
Arg Arg His Glu Leu Tyr Val Ser Phe Gln Asp Leu Gly Trp Leu Asp
            305                 310                 315

TGG GTC ATC GCT CCC CAA GGC TAC TCG GCC TAT TAC TGT GAG GGG GAG 1488
Trp Val Ile Ala Pro Gln Gly Tyr Ser Ala Tyr Tyr Cys Glu Gly Glu
        320                 325                 330

TGC TCC TTC CCA CTG GAC TCC TGC ATG AAT GCC ACC AAC CAC GCC ATC 1536
Cys Ser Phe Pro Leu Asp Ser Cys Met Asn Ala Thr Asn His Ala Ile
    335                 340                 345

CTG CAG TCC CTG GTG CAC CTG ATG AAG CCA AAC GCA GTC CCC AAG GCG 1584
Leu Gln Ser Leu Val His Leu Met Lys Pro Asn Ala Val Pro Lys Ala
350                 355                 360                 365

TGC TGT GCA CCC ACC AAG CTG AGC GCC ACC TCT GTG CTC TAC TAT GAC 1632
Cys Cys Ala Pro Thr Lys Leu Ser Ala Thr Ser Val Leu Tyr Tyr Asp
                370                 375                 380

AGC AGC AAC AAC GTC ATC CTG CGC AAA GCC CGC AAC ATG GTG GTC AAG 1680
Ser Ser Asn Asn Val Ile Leu Arg Lys Ala Arg Asn Met Val Val Lys
            385                 390                 395

GCC TGC GGC TGC CAC T GAGTCAGCCC GCCCAGCCCT ACTGCAG 1723
Ala Cys Gly Cys His
        400

(2) INFORMATION FOR SEQ ID NO:21:

(i) SEQUENCE CHARACTERISTICS:
(A) LENGTH: 402 amino acids
(B) TYPE: amino acid
(D) TOPOLOGY: linear

(ii) MOLECULE TYPE: protein

(xi) SEQUENCE DESCRIPTION: SEQ ID NO:21:

Met Thr Ala Leu Pro Gly Pro Leu Trp Leu Leu Gly Leu Ala Leu Cys
1               5                   10                  15

Ala Leu Gly Gly Gly Gly Pro Gly Leu Arg Pro Pro Pro Gly Cys Pro
            20                  25                  30

Gln Arg Arg Leu Gly Ala Arg Glu Arg Arg Asp Val Gln Arg Glu Ile
        35                  40                  45

Leu Ala Val Leu Gly Leu Pro Gly Arg Pro Arg Pro Arg Ala Pro Pro
    50                  55                  60

Ala Ala Ser Arg Leu Pro Ala Ser Ala Pro Leu Phe Met Leu Asp Leu
65                  70                  75                  80

Tyr His Ala Met Ala Gly Asp Asp Asp Glu Asp Gly Ala Pro Ala Glu
                85                  90                  95

Arg Arg Leu Gly Arg Ala Asp Leu Val Met Ser Phe Val Asn Met Val
            100                 105                 110

Glu Arg Asp Arg Ala Leu Gly His Gln Glu Pro His Trp Lys Glu Phe
        115                 120                 125

Arg Phe Asp Leu Thr Gln Ile Pro Ala Gly Glu Ala Val Thr Ala Ala
    130                 135                 140

Glu Phe Arg Ile Tyr Lys Val Pro Ser Ile His Leu Leu Asn Arg Thr
145                 150                 155                 160

Leu His Val Ser Met Phe Gln Val Val Gln Glu Gln Ser Asn Arg Glu
                165                 170                 175

Ser Asp Leu Phe Phe Leu Asp Leu Gln Thr Leu Arg Ala Gly Asp Glu
            180                 185                 190

Gly Trp Leu Val Leu Asp Val Thr Ala Ala Ser Asp Cys Trp Leu Leu
        195                 200                 205

Lys Arg His Lys Asp Leu Gly Leu Arg Leu Tyr Val Glu Thr Glu Asp
    210                 215                 220

Gly His Ser Val Asp Pro Gly Leu Ala Gly Leu Leu Gly Gln Arg Ala
225                 230                 235                 240

Pro Arg Ser Gln Gln Pro Phe Val Val Thr Phe Phe Arg Ala Ser Pro
                245                 250                 255

Ser Pro Ile Arg Thr Pro Arg Ala Val Arg Pro Leu Arg Arg Arg Gln
            260                 265                 270

Pro Lys Lys Ser Asn Glu Leu Pro Gln Ala Asn Arg Leu Pro Gly Ile
        275                 280                 285

Phe Asp Asp Val His Gly Ser His Gly Arg Gln Val Cys Arg Arg His
    290                 295                 300

Glu Leu Tyr Val Ser Phe Gln Asp Leu Gly Trp Leu Asp Trp Val Ile
305                 310                 315                 320

Ala Pro Gln Gly Tyr Ser Ala Tyr Tyr Cys Glu Gly Glu Cys Ser Phe
                325                 330                 335

Pro Leu Asp Ser Cys Met Asn Ala Thr Asn His Ala Ile Leu Gln Ser
            340                 345                 350

Leu Val His Leu Met Lys Pro Asn Ala Val Pro Lys Ala Cys Cys Ala
        355                 360                 365

Pro Thr Lys Leu Ser Ala Thr Ser Val Leu Tyr Tyr Asp Ser Ser Asn
    370                 375                 380

Asn Val Ile Leu Arg Lys Ala Arg Asn Met Val Val Lys Ala Cys Gly
385                 390                 395                 400

Cys His

(2) INFORMATION FOR SEQ ID NO:22:

(i) SEQUENCE CHARACTERISTICS:
(A) LENGTH: 1926 base pairs
(B) TYPE: nucleic acid
(C) STRANDEDNESS: single
(D) TOPOLOGY: linear

(vi) ORIGINAL SOURCE:
(A) ORGANISM: MURIDAE
(F) TISSUE TYPE: EMBRYO

(ix) FEATURE:
(A) NAME/KEY: CDS
(B) LOCATION: 93..1289
(D) OTHER INFORMATION: /function= "OSTEOGENIC PROTEIN"
/product= "mOP2-PP"
/note= "mOP2 cDNA"

(xi) SEQUENCE DESCRIPTION: SEQ ID NO:22:
   GCCAGGCACA GGTGCGCCGT CTGGTCCTCC CCGTCTGGCG TCAGCCGAGC CCGACCAGCT 60
   ACCAGTGGAT GCGCGCCGGC TGAAAGTCCG AG ATG GCT ATG CGT CCC GGG CCA 113
   Met Ala Met Arg Pro Gly Pro
   1 5
   CTC TGG CTA TTG GGC CTT GCT CTG TGC GCG CTG GGA GGC GGC CAC GGT 161
   Leu Trp Leu Leu Gly Leu Ala Leu Cys Ala Leu Gly Gly Gly His Gly
   10 15 20
   CCG CGT CCC CCG CAC ACC TGT CCC CAG CGT CGC CTG GGA GCG CGC GAG 209
   Pro Arg Pro Pro His Thr Cys Pro Gln Arg Arg Leu Gly Ala Arg Glu
   25 30 35
   CGC CGC GAC ATG CAG CGT GAA ATC CTG GCG GTG CTC GGG CTA CCG GGA 257
   Arg Arg Asp Met Gln Arg Glu Ile Leu Ala Val Leu Gly Leu Pro Gly
   40 45 50 55
   CGG CCC CGA CCC CGT GCA CAA CCC GCC GCT GCC CGG CAG CCA GCG TCC 305
   Arg Pro Arg Pro Arg Ala Gln Pro Ala Ala Ala Arg Gln Pro Ala Ser
   60 65 70
   GCG CCC CTC TTC ATG TTG GAC CTA TAC CAC GCC ATG ACC GAT GAC GAC 353
   Ala Pro Leu Phe Met Leu Asp Leu Tyr His Ala Met Thr Asp Asp Asp
   75 80 85
   GAC GGC GGG CCA CCA CAG GCT CAC TTA GGC CGT GCC GAC CTG GTC ATG 401
   Asp Gly Gly Pro Pro Gln Ala His Leu Gly Arg Ala Asp Leu Val Met
   90 95 100
   AGC TTC GTC AAC ATG GTG GAA CGC GAC CGT ACC CTG GGC TAC CAG GAG 449
   Ser Phe Val Asn Met Val Glu Arg Asp Arg Thr Leu Gly Tyr Gln Glu
   105 110 115
   CCA CAC TGG AAG GAA TTC CAC TTT GAC CTA ACC CAG ATC CCT GCT GGG 497
   Pro His Trp Lys Glu Phe His Phe Asp Leu Thr Gln Ile Pro Ala Gly
   120 125 130 135
   GAG GCT GTC ACA GCT GCT GAG TTC CGG ATC TAC AAA GAA CCC AGC ACC 545
   Glu Ala Val Thr Ala Ala Glu Phe Arg Ile Tyr Lys Glu Pro Ser Thr
   140 145 150
   CAC CCG CTC AAC ACA ACC CTC CAC ATC AGC ATG TTC GAA GTG GTC CAA 593
   His Pro Leu Asn Thr Thr Leu His Ile Ser Met Phe Glu Val Val Gln
   155 160 165
   GAG CAC TCC AAC AGG GAG TCT GAC TTG TTC TTT TTG GAT CTT CAG ACG 641
   Glu His Ser Asn Arg Glu Ser Asp Leu Phe Phe Leu Asp Leu Gln Thr
   170 175 180
   CTC CGA TCT GGG GAC GAG GGC TGG CTG GTG CTG GAC ATC ACA GCA GCC 689
   Leu Arg Ser Gly Asp Glu Gly Trp Leu Val Leu Asp Ile Thr Ala Ala
   185 190 195
   AGT GAC CGA TGG CTG CTG AAC CAT CAC AAG GAC CTG GGA CTC CGC CTC 737
   Ser Asp Arg Trp Leu Leu Asn His His Lys Asp Leu Gly Leu Arg Leu
   200 205 210 215
   TAT GTG GAA ACC GCG GAT GGG CAC AGC ATG GAT CCT GGC CTG GCT GGT 785
   Tyr Val Glu Thr Ala Asp Gly His Ser Met Asp Pro Gly Leu Ala Gly
   220 225 230
   CTG CTT GGA CGA CAA GCA CCA CGC TCC AGA CAG CCT TTC ATG GTA ACC 833
   Leu Leu Gly Arg Gln Ala Pro Arg Ser Arg Gln Pro Phe Met Val Thr
   235 240 245
   TTC TTC AGG GCC AGC CAG AGT CCT GTG CGG GCC CCT CGG GCA GCG AGA 881
   Phe Phe Arg Ala Ser Gln Ser Pro Val Arg Ala Pro Arg Ala Ala Arg
   250 255 260
   CCA CTG AAG AGG AGG CAG CCA AAG AAA ACG AAC GAG CTT CCG CAC CCC 929
   Pro Leu Lys Arg Arg Gln Pro Lys Lys Thr Asn Glu Leu Pro His Pro
   265 270 275
   AAC AAA CTC CCA GGG ATC TTT GAT GAT GGC CAC GGT TCC CGC GGC AGA 977
   Asn Lys Leu Pro Gly Ile Phe Asp Asp Gly His Gly Ser Arg Gly Arg
   280 285 290 295
   GAG GTT TGC CGC AGG CAT GAG CTC TAC GTC AGC TTC CGT GAC CTT GGC 1025
   Glu Val Cys Arg Arg His Glu Leu Tyr Val Ser Phe Arg Asp Leu Gly
   300 305 310
   TGG CTG GAC TGG GTC ATC GCC CCC CAG GGC TAC TCT GCC TAT TAC TGT 1073
   Trp Leu Asp Trp Val Ile Ala Pro Gln Gly Tyr Ser Ala Tyr Tyr Cys
   315 320 325
   GAG GGG GAG TGT GCT TTC CCA CTG GAC TCC TGT ATG AAC GCC ACC AAC 1121
   Glu Gly Glu Cys Ala Phe Pro Leu Asp Ser Cys Met Asn Ala Thr Asn
   330 335 340
   CAT GCC ATC TTG CAG TCT CTG GTG CAC CTG ATG AAG CCA GAT GTT GTC     1169
   His Ala Ile Leu Gln Ser Leu Val His Leu Met Lys Pro Asp Val Val
       345                 350                 355
   CCC AAG GCA TGC TGT GCA CCC ACC AAA CTG AGT GCC ACC TCT GTG CTG     1217
   Pro Lys Ala Cys Cys Ala Pro Thr Lys Leu Ser Ala Thr Ser Val Leu
   360                 365                 370                 375
   TAC TAT GAC AGC AGC AAC AAT GTC ATC CTG CGT AAA CAC CGT AAC ATG     1265
   Tyr Tyr Asp Ser Ser Asn Asn Val Ile Leu Arg Lys His Arg Asn Met
                   380                 385                 390
   GTG GTC AAG GCC TGT GGC TGC CAC TGAGGCCCCG CCCAGCATCC TGCTTCTACT     1319
   Val Val Lys Ala Cys Gly Cys His
               395
   ACCTTACCAT CTGGCCGGGC CCCTCTCCAG AGGCAGAAAC CCTTCTATGT TATCATAGCT     1379
   CAGACAGGGG CAATGGGAGG CCCTTCACTT CCCCTGGCCA CTTCCTGCTA AAATTCTGGT     1439
   CTTTCCCAGT TCCTCTGTCC TTCATGGGGT TTCGGGGCTA TCACCCCGCC CTCTCCATCC     1499
   TCCTACCCCA AGCATAGACT GAATGCACAC AGCATCCCAG AGCTATGCTA ACTGAGAGGT     1559
   CTGGGGTCAG CACTGAAGGC CCACATGAGG AAGACTGATC CTTGGCCATC CTCAGCCCAC     1619
   AATGGCAAAT TCTGGATGGT CTAAGAAGGC CCTGGAATTC TAAACTAGAT GATCTGGGCT     1679
   CTCTGCACCA TTCATTGTGG CAGTTGGGAC ATTTTTAGGT ATAACAGACA CATACACTTA     1739
   GATCAATGCA TCGCTGTACT CCTTGAAATC AGAGCTAGCT TGTTAGAAAA AGAATCAGAG     1799
   CCAGGTATAG CGGTGCATGT CATTAATCCC AGCGCTAAAG AGACAGAGAC AGGAGAATCT     1859
   CTGTGAGTTC AAGGCCACAT AGAAAGAGCC TGTCTCGGGA GCAGGAAAAA AAAAAAAAAC     1919
   GGAATTC                                                             1926

(2) INFORMATION FOR SEQ ID NO:23:

    (i) SEQUENCE CHARACTERISTICS:
        (A) LENGTH: 399 amino acids
        (B) TYPE: amino acid
        (D) TOPOLOGY: linear

    (ii) MOLECULE TYPE: protein

    (xi) SEQUENCE DESCRIPTION: SEQ ID NO:23:

Met Ala Met Arg Pro Gly Pro Leu Trp Leu Leu Gly Leu Ala Leu Cys
  1               5                  10                  15

Ala Leu Gly Gly Gly His Gly Pro Arg Pro Pro His Thr Cys Pro Gln
             20                  25                  30

Arg Arg Leu Gly Ala Arg Glu Arg Arg Asp Met Gln Arg Glu Ile Leu
        35                  40                  45

Ala Val Leu Gly Leu Pro Gly Arg Pro Arg Pro Arg Ala Gln Pro Ala
    50                  55                  60

Ala Ala Arg Gln Pro Ala Ser Ala Pro Leu Phe Met Leu Asp Leu Tyr
65                  70                  75                  80

His Ala Met Thr Asp Asp Asp Asp Gly Gly Pro Pro Gln Ala His Leu
                85                  90                  95

Gly Arg Ala Asp Leu Val Met Ser Phe Val Asn Met Val Glu Arg Asp
            100                 105                 110

Arg Thr Leu Gly Tyr Gln Glu Pro His Trp Lys Glu Phe His Phe Asp
        115                 120                 125

Leu Thr Gln Ile Pro Ala Gly Glu Ala Val Thr Ala Ala Glu Phe Arg
    130                 135                 140

Ile Tyr Lys Glu Pro Ser Thr His Pro Leu Asn Thr Thr Leu His Ile
145                 150                 155                 160

Ser Met Phe Glu Val Val Gln Glu His Ser Asn Arg Glu Ser Asp Leu
                165                 170                 175

Phe Phe Leu Asp Leu Gln Thr Leu Arg Ser Gly Asp Glu Gly Trp Leu
            180                 185                 190

Val Leu Asp Ile Thr Ala Ala Ser Asp Arg Trp Leu Leu Asn His His
        195                 200                 205

Lys Asp Leu Gly Leu Arg Leu Tyr Val Glu Thr Ala Asp Gly His Ser
    210                 215                 220

Met Asp Pro Gly Leu Ala Gly Leu Leu Gly Arg Gln Ala Pro Arg Ser
225                 230                 235                 240

Arg Gln Pro Phe Met Val Thr Phe Phe Arg Ala Ser Gln Ser Pro Val
                245                 250                 255

Arg Ala Pro Arg Ala Ala Arg Pro Leu Lys Arg Arg Gln Pro Lys Lys
            260                 265                 270

Thr Asn Glu Leu Pro His Pro Asn Lys Leu Pro Gly Ile Phe Asp Asp
        275                 280                 285

Gly His Gly Ser Arg Gly Arg Glu Val Cys Arg Arg His Glu Leu Tyr
    290                 295                 300

Val Ser Phe Arg Asp Leu Gly Trp Leu Asp Trp Val Ile Ala Pro Gln
305                 310                 315                 320

Gly Tyr Ser Ala Tyr Tyr Cys Glu Gly Glu Cys Ala Phe Pro Leu Asp
                325                 330                 335

Ser Cys Met Asn Ala Thr Asn His Ala Ile Leu Gln Ser Leu Val His
            340                 345                 350

Leu Met Lys Pro Asp Val Val Pro Lys Ala Cys Cys Ala Pro Thr Lys
        355                 360                 365

Leu Ser Ala Thr Ser Val Leu Tyr Tyr Asp Ser Ser Asn Asn Val Ile
    370                 375                 380

Leu Arg Lys His Arg Asn Met Val Val Lys Ala Cys Gly Cys His
385                 390                 395

(2) INFORMATION FOR SEQ ID NO:24:

     (i) SEQUENCE CHARACTERISTICS:
          (A) LENGTH: 1368 base pairs
          (B) TYPE: nucleic acid
          (C) STRANDEDNESS: single
          (D) TOPOLOGY: linear

    (ii) MOLECULE TYPE: cDNA


    (ix) FEATURE:
          (A) NAME/KEY: CDS
          (B) LOCATION: 1..1368


    (xi) SEQUENCE DESCRIPTION: SEQ ID NO:24:

ATG TCG GGA CTG CGA AAC ACC TCG GAG GCC GTT GCA GTG CTC GCC TCC      48
Met Ser Gly Leu Arg Asn Thr Ser Glu Ala Val Ala Val Leu Ala Ser
  1               5                  10                  15

CTG GGA CTC GGA ATG GTT CTG CTC ATG TTC GTG GCG ACC ACG CCG CCG      96
Leu Gly Leu Gly Met Val Leu Leu Met Phe Val Ala Thr Thr Pro Pro
             20                  25                  30

GCC GTT GAG GCC ACC CAG TCG GGG ATT TAC ATA GAC AAC GGC AAG GAC     144
Ala Val Glu Ala Thr Gln Ser Gly Ile Tyr Ile Asp Asn Gly Lys Asp
         35                  40                  45

CAG ACG ATC ATG CAC AGA GTG CTG AGC GAG GAC GAC AAG CTG GAC GTC     192
Gln Thr Ile Met His Arg Val Leu Ser Glu Asp Asp Lys Leu Asp Val
     50                  55                  60

TCG TAC GAG ATC CTC GAG TTC CTG GGC ATC GCC GAA CGG CCG ACG CAC     240
Ser Tyr Glu Ile Leu Glu Phe Leu Gly Ile Ala Glu Arg Pro Thr His
 65                  70                  75                  80

CTG AGC AGC CAC CAG TTG TCG CTG AGG AAG TCG GCT CCC AAG TTC CTG     288
Leu Ser Ser His Gln Leu Ser Leu Arg Lys Ser Ala Pro Lys Phe Leu
                 85                  90                  95

CTG GAC GTC TAC CAC CGC ATC ACG GCG GAG GAG GGT CTC AGC GAT CAG 336
Leu Asp Val Tyr His Arg Ile Thr Ala Glu Glu Gly Leu Ser Asp Gln
            100                 105                 110

GAT GAG GAC GAC GAC TAC GAA CGC GGC CAT CGG TCC AGG AGG AGC GCC 384
Asp Glu Asp Asp Asp Tyr Glu Arg Gly His Arg Ser Arg Arg Ser Ala
        115                 120                 125

GAC CTC GAG GAG GAT GAG GGC GAG CAG CAG AAG AAC TTC ATC ACC GAC 432
Asp Leu Glu Glu Asp Glu Gly Glu Gln Gln Lys Asn Phe Ile Thr Asp
    130                 135                 140

CTG GAC AAG CGG GCC ATC GAC GAG AGC GAC ATC ATC ATG ACC TTC CTG 480
Leu Asp Lys Arg Ala Ile Asp Glu Ser Asp Ile Ile Met Thr Phe Leu
145                 150                 155                 160

AAC AAG CGC CAC CAC AAT GTG GAC GAA CTG CGT CAC GAG CAC GGC CGT 528
Asn Lys Arg His His Asn Val Asp Glu Leu Arg His Glu His Gly Arg
                165                 170                 175

CGC CTG TGG TTC GAC GTC TCC AAC GTG CCC AAC GAC AAC TAC CTG GTG 576
Arg Leu Trp Phe Asp Val Ser Asn Val Pro Asn Asp Asn Tyr Leu Val
            180                 185                 190

ATG GCC GAG CTG CGC ATC TAT CAG AAC GCC AAC GAG GGC AAG TGG CTG 624
Met Ala Glu Leu Arg Ile Tyr Gln Asn Ala Asn Glu Gly Lys Trp Leu
        195                 200                 205

ACC GCC AAC AGG GAG TTC ACC ATC ACG GTA TAC GCC ATT GGC ACC GGC 672
Thr Ala Asn Arg Glu Phe Thr Ile Thr Val Tyr Ala Ile Gly Thr Gly
    210                 215                 220

ACG CTG GGC CAG CAC ACC ATG GAG CCG CTG TCC TCG GTG AAC ACC ACC 720
Thr Leu Gly Gln His Thr Met Glu Pro Leu Ser Ser Val Asn Thr Thr
225                 230                 235                 240

GGG GAC TAC GTG GGC TGG TTG GAG CTC AAC GTG ACC GAG GGC CTG CAC 768
Gly Asp Tyr Val Gly Trp Leu Glu Leu Asn Val Thr Glu Gly Leu His
                245                 250                 255

GAG TGG CTG GTC AAG TCG AAG GAC AAT CAT GGC ATC TAC ATT GGA GCA 816
Glu Trp Leu Val Lys Ser Lys Asp Asn His Gly Ile Tyr Ile Gly Ala
            260                 265                 270

CAC GCT GTC AAC CGA CCC GAC CGC GAG GTG AAG CTG GAC GAC ATT GGA 864
His Ala Val Asn Arg Pro Asp Arg Glu Val Lys Leu Asp Asp Ile Gly
        275                 280                 285

CTG ATC CAC CGC AAG GTG GAC GAC GAG TTC CAG CCC TTC ATG ATC GGC 912
Leu Ile His Arg Lys Val Asp Asp Glu Phe Gln Pro Phe Met Ile Gly
    290                 295                 300

TTC TTC CGC GGA CCG GAG CTG ATC AAG GCG ACG GCC CAC AGC AGC CAC 960
Phe Phe Arg Gly Pro Glu Leu Ile Lys Ala Thr Ala His Ser Ser His
305                 310                 315                 320

CAC AGG AGC AAG CGA AGC GCC AGC CAT CCA CGC AAG CGC AAG AAG TCG 1008
His Arg Ser Lys Arg Ser Ala Ser His Pro Arg Lys Arg Lys Lys Ser
                325                 330                 335

GTG TCG CCC AAC AAC GTG CCG CTG CTG GAA CCG ATG GAG AGC ACG CGC 1056
Val Ser Pro Asn Asn Val Pro Leu Leu Glu Pro Met Glu Ser Thr Arg
            340                 345                 350

AGC TGC CAG ATG CAG ACC CTG TAC ATA GAC TTC AAG GAT CTG GGC TGG 1104
Ser Cys Gln Met Gln Thr Leu Tyr Ile Asp Phe Lys Asp Leu Gly Trp
        355                 360                 365

CAT GAC TGG ATC ATC GCA CCA GAG GGC TAT GGC GCC TTC TAC TGC AGC 1152
His Asp Trp Ile Ile Ala Pro Glu Gly Tyr Gly Ala Phe Tyr Cys Ser
    370                 375                 380

GGC GAG TGC AAT TTC CCG CTC AAT GCG CAC ATG AAC GCC ACG AAC CAT 1200
Gly Glu Cys Asn Phe Pro Leu Asn Ala His Met Asn Ala Thr Asn His
385                 390                 395                 400

GCG ATC GTC CAG ACC CTG GTC CAC CTG CTG GAG CCC AAG AAG GTG CCC 1248
Ala Ile Val Gln Thr Leu Val His Leu Leu Glu Pro Lys Lys Val Pro
                405                 410                 415

AAG CCC TGC TGC GCT CCG ACC AGG CTG GGA GCA CTA CCC GTT CTG TAC 1296
Lys Pro Cys Cys Ala Pro Thr Arg Leu Gly Ala Leu Pro Val Leu Tyr
            420                 425                 430

CAC CTG AAC GAC GAG AAT GTG AAC CTG AAA AAG TAT AGA AAC ATG ATT 1344
His Leu Asn Asp Glu Asn Val Asn Leu Lys Lys Tyr Arg Asn Met Ile
        435                 440                 445

GTG AAA TCC TGC GGG TGC CAT TGA 1368
Val Lys Ser Cys Gly Cys His
    450                 455

(2) INFORMATION FOR SEQ ID NO:25:

(i) SEQUENCE CHARACTERISTICS:
(A) LENGTH: 455 amino acids
(B) TYPE: amino acid
(D) TOPOLOGY: linear

(ii) MOLECULE TYPE: protein

(xi) SEQUENCE DESCRIPTION: SEQ ID NO:25:

Met Ser Gly Leu Arg Asn Thr Ser Glu Ala Val Ala Val Leu Ala Ser
1               5                   10                  15

Leu Gly Leu Gly Met Val Leu Leu Met Phe Val Ala Thr Thr Pro Pro
            20                  25                  30

Ala Val Glu Ala Thr Gln Ser Gly Ile Tyr Ile Asp Asn Gly Lys Asp
        35                  40                  45

Gln Thr Ile Met His Arg Val Leu Ser Glu Asp Asp Lys Leu Asp Val
    50                  55                  60

Ser Tyr Glu Ile Leu Glu Phe Leu Gly Ile Ala Glu Arg Pro Thr His
65                  70                  75                  80

Leu Ser Ser His Gln Leu Ser Leu Arg Lys Ser Ala Pro Lys Phe Leu
                85                  90                  95

Leu Asp Val Tyr His Arg Ile Thr Ala Glu Glu Gly Leu Ser Asp Gln
            100                 105                 110

Asp Glu Asp Asp Asp Tyr Glu Arg Gly His Arg Ser Arg Arg Ser Ala
        115                 120                 125

Asp Leu Glu Glu Asp Glu Gly Glu Gln Gln Lys Asn Phe Ile Thr Asp
    130                 135                 140

Leu Asp Lys Arg Ala Ile Asp Glu Ser Asp Ile Ile Met Thr Phe Leu
145                 150                 155                 160

Asn Lys Arg His His Asn Val Asp Glu Leu Arg His Glu His Gly Arg
                165                 170                 175

Arg Leu Trp Phe Asp Val Ser Asn Val Pro Asn Asp Asn Tyr Leu Val
            180                 185                 190

Met Ala Glu Leu Arg Ile Tyr Gln Asn Ala Asn Glu Gly Lys Trp Leu
        195                 200                 205

Thr Ala Asn Arg Glu Phe Thr Ile Thr Val Tyr Ala Ile Gly Thr Gly
    210                 215                 220

Thr Leu Gly Gln His Thr Met Glu Pro Leu Ser Ser Val Asn Thr Thr
225                 230                 235                 240

Gly Asp Tyr Val Gly Trp Leu Glu Leu Asn Val Thr Glu Gly Leu His
                245                 250                 255

Glu Trp Leu Val Lys Ser Lys Asp Asn His Gly Ile Tyr Ile Gly Ala
            260                 265                 270

His Ala Val Asn Arg Pro Asp Arg Glu Val Lys Leu Asp Asp Ile Gly
        275                 280                 285

Leu Ile His Arg Lys Val Asp Asp Glu Phe Gln Pro Phe Met Ile Gly
    290                 295                 300

Phe Phe Arg Gly Pro Glu Leu Ile Lys Ala Thr Ala His Ser Ser His
305                 310                 315                 320

His Arg Ser Lys Arg Ser Ala Ser His Pro Arg Lys Arg Lys Lys Ser
                325                 330                 335

Val Ser Pro Asn Asn Val Pro Leu Leu Glu Pro Met Glu Ser Thr Arg
            340                     345                     350

Ser Cys Gln Met Gln Thr Leu Tyr Ile Asp Phe Lys Asp Leu Gly Trp
        355                     360                     365

His Asp Trp Ile Ile Ala Pro Glu Gly Tyr Gly Ala Phe Tyr Cys Ser
    370                     375                     380

Gly Glu Cys Asn Phe Pro Leu Asn Ala His Met Asn Ala Thr Asn His
385                     390                     395                     400

Ala Ile Val Gln Thr Leu Val His Leu Leu Glu Pro Lys Lys Val Pro
                405                     410                     415

Lys Pro Cys Cys Ala Pro Thr Arg Leu Gly Ala Leu Pro Val Leu Tyr
            420                     425                     430

His Leu Asn Asp Glu Asn Val Asn Leu Lys Lys Tyr Arg Asn Met Ile
        435                     440                     445

Val Lys Ser Cys Gly Cys His
    450                     455

(2) INFORMATION FOR SEQ ID NO:26:

(i) SEQUENCE CHARACTERISTICS:
(A) LENGTH: 104 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..104
(D) OTHER INFORMATION: /note= "BMP3"

(xi) SEQUENCE DESCRIPTION: SEQ ID NO:26:

Cys Ala Arg Arg Tyr Leu Lys Val Asp Phe Ala Asp Ile Gly Trp Ser
1               5                   10                  15

Glu Trp Ile Ile Ser Pro Lys Ser Phe Asp Ala Tyr Tyr Cys Ser Gly
            20                  25                  30

Ala Cys Gln Phe Pro Met Pro Lys Ser Leu Lys Pro Ser Asn His Ala
        35                  40                  45

Thr Ile Gln Ser Ile Val Ala Arg Ala Val Gly Val Val Pro Gly Ile
    50                  55                  60

Pro Glu Pro Cys Cys Val Pro Glu Lys Met Ser Ser Leu Ser Ile Leu
65                  70                  75                  80

Phe Phe Asp Glu Asn Lys Asn Val Val Leu Lys Val Tyr Pro Asn Met
                85                  90                  95

Thr Val Glu Ser Cys Ala Cys Arg
            100

(2) INFORMATION FOR SEQ ID NO:27:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: HOMO SAPIENS

(ix) FEATURE:
(A) NAME/KEY: Protein
(B) LOCATION: 1..102
(D) OTHER INFORMATION: /note= "BMP5"

(xi) SEQUENCE DESCRIPTION: SEQ ID NO:27:

Cys Lys Lys His Glu Leu Tyr Val Ser Phe Arg Asp Leu Gly Trp Gln
1               5                   10                  15

Asp Trp Ile Ile Ala Pro Glu Gly Tyr Ala Ala Phe Tyr Cys Asp Gly
            20                  25                  30

Glu Cys Ser Phe Pro Leu Asn Ala His Met Asn Ala Thr Asn His Ala
        35                  40                  45

Ile Val Gln Thr Leu Val His Leu Met Phe Pro Asp His Val Pro Lys
    50                  55                  60

Pro Cys Cys Ala Pro Thr Lys Leu Asn Ala Ile Ser Val Leu Tyr Phe
65                  70                  75                  80

Asp Asp Ser Ser Asn Val Ile Leu Lys Lys Tyr Arg Asn Met Val Val
                85                  90                  95

Arg Ser Cys Gly Cys His
            100

(2) INFORMATION FOR SEQ ID NO:28:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: protein

(vi) ORIGINAL SOURCE:
(A) ORGANISM: HOMO SAPIENS

(ix) FEATURE:
(A) NAME/KEY: Protein
(B) LOCATION: 1..102
(D) OTHER INFORMATION: /note= "BMP6"

(xi) SEQUENCE DESCRIPTION: SEQ ID NO:28:

Cys Arg Lys His Glu Leu Tyr Val Ser Phe Gln Asp Leu Gly Trp Gln
1               5                   10                  15

Asp Trp Ile Ile Ala Pro Lys Gly Tyr Ala Ala Asn Tyr Cys Asp Gly
            20                  25                  30

Glu Cys Ser Phe Pro Leu Asn Ala His Met Asn Ala Thr Asn His Ala
        35                  40                  45

Ile Val Gln Thr Leu Val His Leu Met Asn Pro Glu Tyr Val Pro Lys
    50                  55                  60

Pro Cys Cys Ala Pro Thr Lys Leu Asn Ala Ile Ser Val Leu Tyr Phe
65                  70                  75                  80

Asp Asp Asn Ser Asn Val Ile Leu Lys Lys Tyr Arg Trp Met Val Val
                85                  90                  95

Arg Ala Cys Gly Cys His
            100

(2) INFORMATION FOR SEQ ID NO:29:

(i) SEQUENCE CHARACTERISTICS:
(A) LENGTH: 102 amino acids
(B) TYPE: amino acid
(D) TOPOLOGY: linear

(ii) MOLECULE TYPE: protein

(ix) FEATURE:
(A) NAME/KEY: Protein
(B) LOCATION: 1..102
(D) OTHER INFORMATION: /label= OPX
/note= "WHEREIN EACH XAA IS INDEPENDENTLY SELECTED
FROM A GROUP OF ONE OR MORE SPECIFIED AMINO ACIDS
AS DEFINED IN THE SPECIFICATION (SECTION II.B.2.)"

(xi) SEQUENCE DESCRIPTION: SEQ ID NO:29:

Cys Xaa Xaa His Glu Leu Tyr Val Xaa Phe Xaa Asp Leu Gly Trp Xaa
1               5                   10                  15

Asp Trp Xaa Ile Ala Pro Xaa Gly Tyr Xaa Ala Tyr Tyr Cys Glu Gly
            20                  25                  30

Glu Cys Xaa Phe Pro Leu Xaa Ser Xaa Met Asn Ala Thr Asn His Ala
        35                  40                  45

Ile Xaa Gln Xaa Leu Val His Xaa Xaa Xaa Pro Xaa Xaa Val Pro Lys
    50                  55                  60

Xaa Cys Cys Ala Pro Thr Xaa Leu Xaa Ala Xaa Ser Val Leu Tyr Xaa
65                  70                  75                  80

Asp Xaa Ser Xaa Asn Val Xaa Leu Xaa Lys Xaa Arg Asn Met Val Val
                85                  90                  95

Xaa Ala Cys Gly Cys His
            100

(2) INFORMATION FOR SEQ ID NO:30:

(i) SEQUENCE CHARACTERISTICS:
(A) LENGTH: 97 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..97
(D) OTHER INFORMATION: /label= GENERIC-SEQ5
/note= "WHEREIN EACH XAA IS INDEPENDENTLY SELECTED
FROM A GROUP OF ONE OR MORE SPECIFIED AMINO ACIDS
AS DEFINED IN THE SPECIFICATION."

(xi) SEQUENCE DESCRIPTION: SEQ ID NO:30:

Leu Xaa Xaa Xaa Phe Xaa Xaa Xaa Gly Trp Xaa Xaa Trp Xaa Xaa Xaa
1               5                   10                  15

Pro Xaa Xaa Xaa Xaa Ala Xaa Tyr Cys Xaa Gly Xaa Cys Xaa Xaa Pro
            20                      25                      30

Xaa Xaa Xaa Xaa Xaa Xaa Xaa Xaa Asn His Ala Xaa Xaa Xaa Xaa Xaa
        35                      40                      45

Xaa Xaa Xaa Xaa Xaa Xaa Xaa Xaa Xaa Xaa Xaa Xaa Cys Cys Xaa Pro
    50                      55                      60

Xaa Xaa Xaa Xaa Xaa Xaa Xaa Xaa Leu Xaa Xaa Xaa Xaa Xaa Xaa Xaa
65                      70                      75                      80

Val Xaa Leu Xaa Xaa Xaa Xaa Xaa Met Xaa Val Xaa Xaa Cys Xaa Cys
                85                      90                      95

Xaa

(2) INFORMATION FOR SEQ ID NO:31:

    (i) SEQUENCE CHARACTERISTICS:
        (A) LENGTH: 102 amino acids
        (B) TYPE: amino acid
        (C) STRANDEDNESS: single
        (D) TOPOLOGY: linear

    (ii) MOLECULE TYPE: protein

    (ix) FEATURE:
        (A) NAME/KEY: Protein
        (B) LOCATION: 1..102
        (D) OTHER INFORMATION: /label= GENERIC-SEQ6
            /note= "WHEREIN EACH XAA IS INDEPENDENTLY SELECTED
            FROM A GROUP OF ONE OR MORE SPECIFIED AMINO ACIDS
            AS DEFINED IN THE SPECIFICATION. "

    (xi) SEQUENCE DESCRIPTION: SEQ ID NO:31:

Cys Xaa Xaa Xaa Xaa Leu Xaa Xaa Xaa Phe Xaa Xaa Xaa Gly Trp Xaa
1               5                       10                      15

Xaa Trp Xaa Xaa Xaa Pro Xaa Xaa Xaa Xaa Ala Xaa Tyr Cys Xaa Gly
            20                      25                      30

Xaa Cys Xaa Xaa Pro Xaa Xaa Xaa Xaa Xaa Xaa Xaa Xaa Asn His Ala
        35                      40                      45

Xaa Xaa Xaa Xaa Xaa Xaa Xaa Xaa Xaa Xaa Xaa Xaa Xaa Xaa Xaa Xaa
    50                      55                      60

Xaa Cys Cys Xaa Pro Xaa Xaa Xaa Xaa Xaa Xaa Xaa Xaa Leu Xaa Xaa
65                      70                      75                      80

Xaa Xaa Xaa Xaa Xaa Val Xaa Leu Xaa Xaa Xaa Xaa Xaa Met Xaa Val
                85                  90                  95

Xaa Xaa Cys Xaa Cys Xaa
            100

(2) INFORMATION FOR SEQ ID NO:32:

     (i) SEQUENCE CHARACTERISTICS:
          (A) LENGTH: 1247 base pairs
          (B) TYPE: nucleic acid
          (C) STRANDEDNESS: single
          (D) TOPOLOGY: linear

    (ii) MOLECULE TYPE: cDNA

    (vi) ORIGINAL SOURCE:
          (A) ORGANISM: HOMO SAPIENS
          (F) TISSUE TYPE: BRAIN

    (ix) FEATURE:
          (A) NAME/KEY: CDS
          (B) LOCATION: 84..1199
          (D) OTHER INFORMATION: /product= "GDF-1"
                /note= "GDF-1 CDNA"

    (xi) SEQUENCE DESCRIPTION: SEQ ID NO:32:

GGGGACACCG GCCCCGCCCT CAGCCCACTG GTCCCGGGCC GCCGCGGACC CTGCGCACTC      60

TCTGGTCATC GCCTGGGAGG AAG ATG CCA CCG CCG CAG CAA GGT CCC TGC      110
                          Met Pro Pro Pro Gln Gln Gly Pro Cys
                           1               5

GGC CAC CAC CTC CTC CTC CTC CTG GCC CTG CTG CTG CCC TCG CTG CCC      158
Gly His His Leu Leu Leu Leu Leu Ala Leu Leu Leu Pro Ser Leu Pro
 10                  15                  20                  25

CTG ACC CGC GCC CCC GTG CCC CCA GGC CCA GCC GCC GCC CTG CTC CAG      206
Leu Thr Arg Ala Pro Val Pro Pro Gly Pro Ala Ala Ala Leu Leu Gln
                 30                  35                  40

GCT CTA GGA CTG CGC GAT GAG CCC CAG GGT GCC CCC AGG CTC CGG CCG      254
Ala Leu Gly Leu Arg Asp Glu Pro Gln Gly Ala Pro Arg Leu Arg Pro
             45                  50                  55

GTT CCC CCG GTC ATG TGG CGC CTG TTT CGA CGC CGG GAC CCC CAG GAG      302
Val Pro Pro Val Met Trp Arg Leu Phe Arg Arg Arg Asp Pro Gln Glu
         60                  65                  70

ACC AGG TCT GGC TCG CGG CGG ACG TCC CCA GGG GTC ACC CTG CAA CCG      350
Thr Arg Ser Gly Ser Arg Arg Thr Ser Pro Gly Val Thr Leu Gln Pro
     75                  80                  85

TGC CAC GTG GAG GAG CTG GGG GTC GCC GGA AAC ATC GTG CGC CAC ATC 398
Cys His Val Glu Glu Leu Gly Val Ala Gly Asn Ile Val Arg His Ile
90                  95                  100                 105

CCG GAC CGC GGT GCG CCC ACC CGG GCC TCG GAG CCT GTC TCG GCC GCG 446
Pro Asp Arg Gly Ala Pro Thr Arg Ala Ser Glu Pro Val Ser Ala Ala
                110                 115                 120

GGG CAT TGC CCT GAG TGG ACA GTC GTC TTC GAC CTG TCG GCT GTG GAA 494
Gly His Cys Pro Glu Trp Thr Val Val Phe Asp Leu Ser Ala Val Glu
            125                 130                 135

CCC GCT GAG CGC CCG AGC CGG GCC CGC CTG GAG CTG CGT TTC GCG GCG 542
Pro Ala Glu Arg Pro Ser Arg Ala Arg Leu Glu Leu Arg Phe Ala Ala
        140                 145                 150

GCG GCG GCG GCA GCC CCG GAG GGC GGC TGG GAG CTG AGC GTG GCG CAA 590
Ala Ala Ala Ala Ala Pro Glu Gly Gly Trp Glu Leu Ser Val Ala Gln
    155                 160                 165

GCG GGC CAG GGC GCG GGC GCG GAC CCC GGG CCG GTG CTG CTC CGC CAG 638
Ala Gly Gln Gly Ala Gly Ala Asp Pro Gly Pro Val Leu Leu Arg Gln
170                 175                 180                 185

TTG GTG CCC GCC CTG GGG CCG CCA GTG CGC GCG GAG CTG CTG GGC GCC 686
Leu Val Pro Ala Leu Gly Pro Pro Val Arg Ala Glu Leu Leu Gly Ala
                190                 195                 200

GCT TGG GCT CGC AAC GCC TCA TGG CCG CGC AGC CTC CGC CTG GCG CTG 734
Ala Trp Ala Arg Asn Ala Ser Trp Pro Arg Ser Leu Arg Leu Ala Leu
            205                 210                 215

GCG CTA CGC CCC CGG GCC CCT GCC GCC TGC GCG CGC CTG GCC GAG GCC 782
Ala Leu Arg Pro Arg Ala Pro Ala Ala Cys Ala Arg Leu Ala Glu Ala
        220                 225                 230

TCG CTG CTG CTG GTG ACC CTC GAC CCG CGC CTG TGC CAC CCC CTG GCC 830
Ser Leu Leu Leu Val Thr Leu Asp Pro Arg Leu Cys His Pro Leu Ala
    235                 240                 245

CGG CCG CGG CGC GAC GCC GAA CCC GTG TTG GGC GGC GGC CCC GGG GGC 878
Arg Pro Arg Arg Asp Ala Glu Pro Val Leu Gly Gly Gly Pro Gly Gly
250                 255                 260                 265

GCT TGT CGC GCG CGG CGG CTG TAC GTG AGC TTC CGC GAG GTG GGC TGG 926
Ala Cys Arg Ala Arg Arg Leu Tyr Val Ser Phe Arg Glu Val Gly Trp
                270                 275                 280

CAC CGC TGG GTC ATC GCG CCG CGC GGC TTC CTG GCC AAC TAC TGC CAG 974
His Arg Trp Val Ile Ala Pro Arg Gly Phe Leu Ala Asn Tyr Cys Gln
            285                 290                 295

GGT CAG TGC GCG CTG CCC GTC GCG CTG TCG GGG TCC GGG GGG CCG CCG 1022
Gly Gln Cys Ala Leu Pro Val Ala Leu Ser Gly Ser Gly Gly Pro Pro
        300                 305                 310

GCG CTC AAC CAC GCT GTG CTG CGC GCG CTC ATG CAC GCG GCC GCC CCG 1070
Ala Leu Asn His Ala Val Leu Arg Ala Leu Met His Ala Ala Ala Pro
    315                 320                 325

GGA GCC GCC GAC CTG CCC TGC TGC GTG CCC GCG CGC CTG TCG CCC ATC 1118
Gly Ala Ala Asp Leu Pro Cys Cys Val Pro Ala Arg Leu Ser Pro Ile
330                 335                 340                 345

TCC GTG CTC TTC TTT GAC AAC AGC GAC AAC GTG GTG CTG CGG CAG TAT 1166
Ser Val Leu Phe Phe Asp Asn Ser Asp Asn Val Val Leu Arg Gln Tyr
                350                 355                 360

GAG GAC ATG GTG GTG GAC GAG TGC GGC TGC CGC TAACCCGGGG CGGGCAGGGA 1219
Glu Asp Met Val Val Asp Glu Cys Gly Cys Arg
            365                 370

CCCGGGCCCA ACAATAAATG CCGCGTGG 1247

(2) INFORMATION FOR SEQ ID NO:33:

(i) SEQUENCE CHARACTERISTICS:
(A) LENGTH: 372 amino acids
(B) TYPE: amino acid
(D) TOPOLOGY: linear

(ii) MOLECULE TYPE: protein

(xi) SEQUENCE DESCRIPTION: SEQ ID NO:33:

Met Pro Pro Pro Gln Gln Gly Pro Cys Gly His His Leu Leu Leu Leu
1               5                   10                  15

Leu Ala Leu Leu Leu Pro Ser Leu Pro Leu Thr Arg Ala Pro Val Pro
            20                  25                  30

Pro Gly Pro Ala Ala Ala Leu Leu Gln Ala Leu Gly Leu Arg Asp Glu
        35                  40                  45

Pro Gln Gly Ala Pro Arg Leu Arg Pro Val Pro Pro Val Met Trp Arg
    50                  55                  60

Leu Phe Arg Arg Arg Asp Pro Gln Glu Thr Arg Ser Gly Ser Arg Arg
65                  70                  75                  80

Thr Ser Pro Gly Val Thr Leu Gln Pro Cys His Val Glu Glu Leu Gly
                85                  90                  95

Val Ala Gly Asn Ile Val Arg His Ile Pro Asp Arg Gly Ala Pro Thr
            100                 105                 110

Arg Ala Ser Glu Pro Val Ser Ala Ala Gly His Cys Pro Glu Trp Thr
        115                 120                 125

Val Val Phe Asp Leu Ser Ala Val Glu Pro Ala Glu Arg Pro Ser Arg
    130                 135                 140
Ala Arg Leu Glu Leu Arg Phe Ala Ala Ala Ala Ala Ala Ala Pro Glu
145                 150                 155                 160
Gly Gly Trp Glu Leu Ser Val Ala Gln Ala Gly Gln Gly Ala Gly Ala
                165                 170                 175
Asp Pro Gly Pro Val Leu Leu Arg Gln Leu Val Pro Ala Leu Gly Pro
            180                 185                 190
Pro Val Arg Ala Glu Leu Leu Gly Ala Ala Trp Ala Arg Asn Ala Ser
        195                 200                 205
Trp Pro Arg Ser Leu Arg Leu Ala Leu Ala Leu Arg Pro Arg Ala Pro
    210                 215                 220
Ala Ala Cys Ala Arg Leu Ala Glu Ala Ser Leu Leu Leu Val Thr Leu
225                 230                 235                 240
Asp Pro Arg Leu Cys His Pro Leu Ala Arg Pro Arg Arg Asp Ala Glu
                245                 250                 255
Pro Val Leu Gly Gly Gly Pro Gly Gly Ala Cys Arg Ala Arg Arg Leu
            260                 265                 270
Tyr Val Ser Phe Arg Glu Val Gly Trp His Arg Trp Val Ile Ala Pro
        275                 280                 285
Arg Gly Phe Leu Ala Asn Tyr Cys Gln Gly Gln Cys Ala Leu Pro Val
    290                 295                 300
Ala Leu Ser Gly Ser Gly Gly Pro Pro Ala Leu Asn His Ala Val Leu
305                 310                 315                 320
Arg Ala Leu Met His Ala Ala Ala Pro Gly Ala Ala Asp Leu Pro Cys
                325                 330                 335
Cys Val Pro Ala Arg Leu Ser Pro Ile Ser Val Leu Phe Phe Asp Asn
            340                 345                 350
Ser Asp Asn Val Val Leu Arg Gln Tyr Glu Asp Met Val Val Asp Glu
        355                 360                 365
Cys Gly Cys Arg
    370

## Description

### BACKGROUND OF THE INVENTION

The present invention relates to compositions for correcting a liver function deficiency or a protein deficiency in a mammal, and to various kits, methods and uses concerned with liver function.

The liver is the largest viceral organ in the body and consists of two main lobes, a larger right lobe and a smaller left lobe. The right lobe also contains two smaller segments referred to as the cuadata and quadrata lobes. The liver has a dual blood supply, consisting of the hepatic artery and the portal vein. The hepatic lymphatics drain principally into lymph nodes of the porta hepatis and celiac axis.

The liver is responsible for a wide variety of functions, broadly characterized as metabolic, storage, synthetic, catabolic and excretory. Specifically, the liver is the central organ of glucose homeostasis, responsible for both storing excess blood glucose as glycogen and restoring blood glucose by glycogenolysis and gluconeogenesis and by converting free fatty acids to triglycerides and lipoproteins. The liver also stores triglycerides, iron, copper and lipid-soluble vitamins and synthesizes many of the binding proteins for iron, copper and Vitamin A.

In addition, most serum proteins, with the exception of immunoglobulins, are synthesized in the liver, including albumin, the principal source of plasma oncotic pressure, blood clotting factors such as prothrombin, fibrinogen and Factor VIII, as well as complement and other acute phase reactants involved in an immune response. The liver also functions as a catabolic site for hormones, serum proteins, and other endogenous proteins, as well as acting as the detoxification site for foreign compounds, including drugs (pharmaceuticals), industrial chemicals, environmental contaminants, and various bacterial metabolism byproducts. Finally, the liver excretes bile, which provides a repository for the products of hemecatabolism and also is vital for fat absorption in the small intestine.

Not surprisingly, liver function disorders, whether resulting from a particular protein deficiency or from hepatic tissue damage and/or loss, has serious and far-reaching consequences. For example, reduced albumin levels in chronic liver disease contribute to the development of edema and ascites; liver failure also is characterized by severe and often life-threatening bleeding, due to the reduced production of essential blood clotting factors. Hepatic failure also can induce neurological dysfunction, characterized broadly as hepatic encephalopathy, as well as associated renal failure, jaundice, pulmonary complications, and a host of disorders associated with hormonal imbalances.

Unlike most other organs in the body the liver has a defined regenerative capacity following hepatic tissue damage or cell death. Specifically, while hepatocytes do not proliferate actively following fetal and post natal liver growth, normally quiescent hepatocytes do divide in response to cell death or loss of liver tissue. However, where tissue damage is extensive and/or chronic, permanent tissue damage can result, reducing the organ's viability and functional capacity. Permanent hepatic tissue damage typically is characterized by extensive necrosis and/or fibrogenesis or scarring (cirrhosis). Another source of nonreparative damage results from hepatic neoplasms and metastatic carcinomas.

Where either the mass of liver cells is sufficiently diminished or their function sufficiently impaired, hepatic failure ensues. The etiology of hepatic failure may be metabolic (e.g., altered bilirubin metabolism or fatty acid storage), infectious (e.g., induced by viral hepatitis, hepatic schistomiasis, syphilis, or ascariaris), toxic (e.g., induced by ethanol, ammonia, phenol, and other environmental toxins, fatty acids, drugs and/or their metabolites), autoimmune, ischemic or nutritional (e.g., alcoholic liver disease).

Another source of hepatic failure results from malignant tumors. The tumor cells may be derived from hepatic tissue cells (as in hepatocellular carcinoma, bileduct carcinomas, hepatoblastomas or hemangiosarcoma) or may be derived from distant tissue as part of a metastatic cancer. In fact, metastatic cancers are by far the most common malignant neoplasms of the liver, most notably derived from cancers of the gastrointestinal tract, breast and lung.

Another source of diminished liver function arises from hepatic protein deficiencies, which may result from a genetic defect (so called "inborn errors of metabolism") or may be induced by, for example, a pharmaceutical, infectious agent byproduct, or the like. For example, hemophilia is believed to be associated with diminished Factor VIII production, Wilson's disease, a copper metabolism disorder, is associated with deficient ceruloplasmin production by the liver, altered albumin production affects bilirubin metabolism, and α₁-antitrypsin deficiency, normally produced in the liver, can result in fatal neonatal hepatitis.

To date, the only viable treatment for hepatic failure or for patients at risk for hepatic failure due to, for example, chronic acute hepatitis, biliary atresia, idiopathic cirrhosis, primary biliary cirrhosis, sclerosing cholangitis, inborn errors of metabolism or malignancy, is liver transplantation. To date, liver transplantation also is the only viable alternative for correcting significant liver function deficiencies that result from inborn errors of metabolism. Liver transplantation as a treatment method suffers from donor scarcity, particularly of pediatric livers, technical surgical complexity, postoperative complications including organ rejection, and continuing difficulties in maintaining organ viability throughout the transplant process.

Selective cell transplantation of only those parenchymal elements necessary to replace lost function has been proposed as an alternative to whole or partial organ transplantation that avoid major surgery with its attendant blood loss, anesthetic difficulties, and complications (P.S.Russell, Ann. Surg. 201(3), 255-262 (1985). Replacing only those cells which supply the needed function reduces problems with passenger leukocytes, antigen presenting cells, and other cell types which may promote the rejection process. The ability to expand cell numbers with proliferation of cells in culture, in theory, allows autotransplantation of one's own tissue. In addition, transplantable cells may be used as part of a gene therapy to correct a liver protein deficiency, and/or as in vivo drug delivery vehicles. WO88/03785 published June 2, 1988, and WO90/12640 published November 1, 1990, both describe methods for attaching hepatocytes to matrices and implanting the matrices at sites in vivo that are capable of providing the cells with adequate nutrition or gas exchange, such as within mesentery folds or the odentum. To date, the existing protocols suffer from a variety of limitations. Typically, partial hepatectomy is required to stimulate cell proliferation of the synthetic tissue in vivo. In addition, cell implantation typically is accompanied by significant cell loss, requiring a substantial seed cell population for implantation, which may further require lengthy in vitro incubation periods. The delay in in vivo integration of the implanted cell-matrix structure also places significant restrictions on the matrix scaffold composition. Finally, the implanted cell-matrix structures also are at risk for destruction by the implant host's immune response mechanisms.

It is an object of this invention to provide methods and compositions for regenerating lost or damaged hepatic tissue in vivo in an existing liver without requiring organ or tissue transplant. Another object is to provide means for maintaining normal liver function following hepatic tissue injury or in anticipation of such injury. Another object is to provide means for enhancing or increasing a depressed liver function level which may result from a tissue injury or disease. Still another object is to provide methods and compositions for correcting a liver function deficiency in a mammal. Yet another object is to provide gene therapy protocols and compositions useful for correcting a protein deficiency in a mammal. Yet another object is to enhance integration of a liver tissue implant. These and other objects and features of the invention will be apparent from the description, drawings and claims which follow.

As further background to the invention, WO 92/09301 describe growth factor-supplemented tissue sealant, in particular a fibrin glue that may be supplemented with bone morphogenic proteins. Furthermore, WO 89/10409 discloses purified 3MP-3 proteins and proposes their use in the treatment of bone and cartilage defects, in wound healing and in related tissue repair.

### Summary of the Invention

The present invention provides compositions, kits, methods and uses as defined in the claims.

Liver function deficiencies in a mammal may arise, for example, from an inborn metabolism defect. Described herein are means for enhancing the viability of a hepatic tissue or organ to be transplanted and means for enhancing the integration of the transplanted tissue. Also described herein are methods and compositions for providing to hepatic cells a therapeutically effective concentration of a morphogenic protein ("morphogen", as defined herein) upon hepatocellular injury, or in anticipation of such injury, or following diagnosis of a liver function defect in a mammal, for a time and at a concentration sufficient to maintain or regain liver function in vivo.

The compositions and therapeutic treatment methods described herein include administering to a mammal a therapeutically effective amount of a morphogenic protein ("morphogen"), as defined herein, upon hepatocellular injury, or in anticipation of such injury, or following diagnosis of a liver function deficiency, for a time and at a concentration sufficient to maintain normal and/or to regain lost liver function in vivo, including regenerating lost or damaged hepatic tissue, and/or inhibiting additional damage thereto. The morphogens described herein also are capable of enhancing the level of a liver function which may be depressed as a result of a tissue injury or disease.

Also described herein are compositions and therapeutic treatment methods for maintaining liver function in a mammal in vivo which include administering to the mammal, upon hepatocellular injury or in anticipation of such injury, or following diagnosis of a liver function deficiency, a compound that stimulates in vivo a therapeutically effective concentration of an endogenous morphogen within the body of the mammal sufficient to increase or enhance the level of a depressed liver function, and/or to maintain normal and/or regain lost liver function, including regenerating damaged or lost hepatic tissue and/or inhibiting additional damage thereto. These compounds are referred to herein as morphogen-stimulating agents, and are understood to include substances which, when administered to a mammal, act on cells of tissue(s) or organ(s) that normally are responsible for, or capable of, producing a morphogen and/or secreting a morphogen, and which cause the endogenous level of the morphogen to be altered. The agent may act, for example, by stimulating expression and/or secretion of an endogenous morphogen.

While the methods and compositions described herein are particularly related to liver organ therapies, as will be appreciated by those skilled in the art, the methods and compositions of this invention can be applied, without undue experimentation, to other organ applications, including but not limited to, the pancreas, lung, kidney and heart. Accordingly, the methods and compositions disclosed herein can be used to advantage in the repair, regeneration, transplantation and/or function level enhancement of damaged or lost tissue such as, for example, damaged lung tissue resulting from emphysema, cirrhotic kidney or pancreatic tissue, damaged heart or blood vessel tissue, as may result from cardiomyopathies and/or atherothrombotic or cardioembolic strokes, damaged stomach tissue resulting from ulceric perforations or their repair, damaged neural tissue as may result from physical injury, degenerative diseases such as Alzheimer's disease or multiple sclerosis or strokes, and damaged dental and/or periodental tissue as may result from disease or mechanical injury. The methods and compositions also may be used to protect these tissues from anticipated injury, including unavoidably or deliberately induced injury, as may occur in a surgical or other clinical procedure. In addition to the tissue regenerative properties provided herein, the gene therapy and drug delivery protocols described herein may be used to particular advantage in pancreatic tissue, renal tissue and lung tissue contexts.

As embodied herein, the expression "maintaining nomral liver function" means both regaining or restoring liver function lost due to a hepatocellular injury or inborn metabolic defect, as well as protecting the hepatic tissue at risk of damage from hepatocellular injury. "Depressed liver function" level refers to a diminished or deficient liver function as a result of a tissue injury or disease. The expression "enhance viability of" transplant hepatic tissue or organ, as used herein, means protection from, reduction of and/or elimination of reduced or lost tissue or organ function as a result of tissue necrosis and/or fibrosis associated with transplantation, particularly immune response-mediated tissue necrosis and/or fibrosis. "Alleviating" means protection from, reduction of and/or elimination of undesired tissue destruction, particularly immune response-mediated tissue destruction. "Transplanted" living tissue includes both tissue grafts and cellular transplants, as in the case of transplanted isolated progenitor or stem cells, for example, which may be implanted alone or in association with a temporary scaffolding. Tissues may be autologous or allogenic tissue and/or synthetic tissue created, for example, by culturing hepatic cells in the presence of an artificial matrix. "Morphogenically permissive environment" is understood to mean an environment competent to allow tissue morphogenesis to occur. Finally, "symptom alleviating cofactor" refers to one or more pharmaceuticals which may be administered together with the therapeutic agents of this invention and which alleviate or mitigate one or more of the symptoms typically associated with liver tissue and/or liver function loss. Exemplary cofactors include antibiotics, antiseptics, non-steroidal anti-inflammatory agents, and the like.

The described methods and compositions are useful in the replacement of diseased, damaged or lost hepatic tissue in a mammal, particularly when the damaged tissue interferes with normal tissue or organ function. Where hepatic tissue has been lost, remaining hepatocytes are capable only of compensatory cell division to return the organ volume essentially to its original size. As determined by extensive experimental partial hepatectomy studies wherein part of all of a liver lobe is excised, this compensatory growth does not involve true morphogenisis, and the lost tissue is not itself regenerated. Rather, the intact lobe is capable only of tissue augmentation to compensate for the lost mass. By contrast, recent studies on toxin-induced tissue damage does suggest that this repair involves morphogenesis, particularly the infiltration and proliferation of progenitor cells. As described in Example 3 and 4, below, endogenous morphogen expression is enhanced following toxin-induced hepatic tissue damage, and not following partial hepatectomy.

When the proteins described herein are provided to, or their expression stimulated at, a hepatic tissue locus, the developmental cascade of tissue morphogenesis is induced, capable of stimulating the migration, proliferation and differentiation of hepatic progenitor cells, to regenerate viable hepatic tissue, including inducing the necessary associated vascularization (see below). Thus, methods and compositions for regenerating lost or substantially irreparably damaged hepatic tissue are described. The morphogen preferably is provided directly to the locus of tissue regeneration, e.g. by injection of the morphogen dispersed in a biocompatible, injectable solution, or by topical administration, as by painting or spraying a morphogen-containing solution on the tissue. Preferably, the locus has been surgically prepared by removing existing necrotic or cirrhotic tissue. Alternatively, morphogen may be provided locally by means of an osmotic pump implanted in the peritoneal cavity. At least one morphogen (OP-1) is known to be expressed by hepatic tissue during liver formation. Accordingly, in the alternative, and/or in addition, an agent capable of stimulating expression and/or secretion of an endogenous morphogen may be administered. As yet another alternative, progenitor hepatocytic cells may be stimulated ex vivo by exposure to a morphogen or morphogen-stimulating agent, and the stimulated cells, now primed for proliferation and differentiation, then provided to the hepatic tissue locus. A morphogen or a morphogen-stimulating agent also may be implanted with the cells. Alternatively, a suitable local morphogen concentration may be maintained by means, for example, of an osmotic pump. In all these cases the existing tissue provides the necessary matrix requirements, providing a suitable substratum for the proliferating and differentiating cells in a morphogenically permissive environment, as well as providing the necessary signals for directing the tissue-specificity of the developing tissue.

When the morphogens (or progenitor cells stimulated by these morphogens) are provided at a tissue-specific locus (e.g., by systemic injection or by implantation or injection at a tissue-specific locus, or by administration of an agent capable of stimulating morphogen expression in vivo), the existing tissue at that locus, whether diseased or damaged, has the capacity of acting as a suitable matrix. Alternatively, a formulated matrix may be externally provided together with the stimulated progenitor cells or morphogen, as may be necessary when the extent of injury sustained by the damaged tissue is large. The matrix should be a biocompatible, suitably modified acellular matrix having dimensions such that it allows the influx, differentiation, and proliferation of migratory progenitor cells, and is capable of providing a morphogenically permissive environment (see infra). Currently preferred matrices also are biodegradable. Where morphogen and/or progenitor cells are to be implanted and the existing liver tissue is insufficient to provide the necessary matrix components, the formulated matrix preferably is tissue-specific.

Formulated matrices may be generated from a fibrin clot or dehydrated organ-specific tissue, prepared for example, by treating the tissue with solvents to substantially remove the non-structural components from the tissue. Alternatively, the matrix may be formulated synthetically using one or more biocompatible, preferably in vivo biodegradable, structural carrier materials such as collagen, laminin, and/or hyaluronic acid which also may be in association with suitable tissue-specific cell attachment factors. Other biocompatible, in vivo biodegradable components, including synthetic polymers, including polybutyric, polylactic, polyglycolic acids, polyanhydrides and/or copolymers thereof. Currently preferred structural materials contain collagens. Currently preferred cell attachment factors include glycosaminoglycans and proteoglycans. The matrix further may be treated with an agent or agents to increase the number of pores and/or micropits on its surfaces, so as to enhance the influx, proliferation and differentiation of migratory progenitor cells from the body of the mammal.

In many instances, the loss of hepatic tissue function results from fibrosis or scar tissue formation, formed in response to an initial or repeated injury to the tissue. The degree of scar tissue formation generally depends on the regenerative properties of the injured tissue, and on the degree and type of injury. In liver, repeated tissue damage results in liver cirrhosis which destroys normal hepatic architecture by fiborous septa, causing vascular disorganization and perfusion deficits that impair liver function and unchecked, lead to hepatic failure. Thus, in another aspect, the invention provides methods and compositions that may be used to prevent and/or substantially inhibit the formation of scar tissue in hepatic tissue by providing the morphogens, or morphogen-stimulated cells, to a newly injured tissue locus (see below).

The morphogens described herein ay also may be used to increase or regenerate a liver progenitor or stem cell population in a mammal. For example, progenitor cells may be isolated from an individual's bone marrow, stimulated ex vivo for a time and at a morphogen concentration sufficient to induce the cells to proliferate, and returned to the bone marrow. Other sources of progenitor cells that may be suitable include biocompatible cells obtained from a cultured cell line, stimulated in culture, and subsequently provided to the body. Alternatively, the morphogen may be provided systemically, or implanted, injected or otherwise provided to a progenitor cell population in an individual to induce its mitogenic activity in vivo. For example, an agent capable of stimulating morphogen expression in the progenitor cell population of interest may be provided to the cells in vivo, for example systemically, to induce mitogenic activity.

The described morphogens also may be used to support the growth and maintenance of differentiated cells, inducing existing differentiated cells to continue expressing their phenotype. It is anticipated that this activity will be particularly useful in the treatment of liver disorders where loss of liver function is caused by cells becoming metabolically senescent or quiescent. Application of the protein directly to the cells to be treated, or providing it by systemic injection, can be used to stimulate these cells to continue expressing their phenotype, thereby significantly reversing the effects of the dysfunction. Alternatively, administration of an agent capable of stimulating morphogen expression in vivo also may be used. In addition, the morphogens of this invention also may be used in gene therapy protocols to stimulate the growth of quiescent cells, thereby potentially enhancing the ability of these cells to incorporate exogenous DNA.

The methods disclosed are useful for redifferentiating transformed cells, particularly transformed cells of parenchymal origin, such that the morphogen-treated cells are induced to display a morphology characteristic of untransformed cells. As described in international application US92/01968 (WO92/15323) the morphogens previously have been found to induce redifferentiation of transformed embryonic cells and cells of neuronal origin to a morphology characteristic of untransformed cells. Morphogen treatment preferably induces cell rounding and cell aggregation (clumping), cell-cell adhesion, and CAM production. The methods described herein are anticipated to substantially inhibit or reduce hepatocytic cell tumor formation and/or proliferation in liver tissue. It is anticipated that the methods of this invention will be useful in substantially reducing the effects of various carcinomas and sarcomas of liver tissue origin, including hepatocellular carcinomas, bileduct carcinomas, hepatoblastomas, and hemangiosarcomas. In addition, the method also is anticipated to aid in inhibiting neoplastic lesions caused by metastatic tissue. Metastatic tumors are one of the most common neoplasms of the liver, as they can reaching the liver through the bloodstream or lymph nodes. Metastatic tumors may damage hepatic function for example, by distorting normal liver tissue architecture, blocking or inhibiting blood flow, and/or by stimulating the body's immune response.

The morphogens described herein are useful for providing hepatocellular protective effects to alleviate liver tissue damage associated with the body's immune/inflammatory response to an initial injury to the tissue. As described in detail in international application US92/07358 (WO93/04692), such a response may follow acute or chronic trauma to hepatic tissue, caused, for example, by an autoimmune dysfunction, neoplastic lesion, infection, chemical or mechanical trauma, disease or by partial or complete interruption of blood flow to hepatocytes, for example following ischemia or hypoxia, or by other trauma to the liver or surrounding material. For example, portal hypertension is a significant liver disease caused by reduced blood flow through the portal vein and is characterized by tissue necrosis and cirrhosis. Application of the morphogen directly to the cells to be treated, or providing the morphogen to the mammal systemically, for example, intravenously or indirectly by oral administration, may be used to alleviate and/or inhibit the immunologically related response to a hepatic tissue injury. Alternatively, administration of an agent capable of stimulating morphogen expression and/or secretion in vivo, preferably at the site of injury, also may be used. Where the injury is to be unavoidably or deliberately induced, as during surgery or other aggressive clinical treatment, the morphogen or agent may be provided prior to induction of the injury to provide a cytoprotective effect to the liver tissue at risk.

Similarly, hepatic tissues and organs for transplantation also are subject to the tissue destructive effects associated with the recipient host body's inflammatory response following transplantation. It is currently believed that the initial destructive response is due in large part to reperfusion injury to the transplanted organ after it has been transplanted to the organ recipient.

Accordingly, the success of liver or hepatic tissue transplantation depends greatly on the preservation of the tissue activity (e.g., tissue or organ viability) at the harvest of the organ, during storage of the harvested organ, and at transplantation. To date, preservation of organs such as lungs, pancreas, heart and liver remains a significant stumbling block to the successful transplantation of these organs. U.S. Patent No. 4,952,409 describes a superoxide dismutase-containing liposome to inhibit reperfusion injury. U.S. Patent No. 5,002,965 describes the use of ginkolides, known platelet activating factor antagonists, to inhibit reperfusion injury. Both of these factors are described as working primarily by inhibiting the release of and/or inhibiting the damaging effects of free oxygen radicals. A number of patents also have issued on the use of immunosuppressants for inhibiting graft rejection. A representative listing includes U.S. Patent Nos. 5,104,858, 5,008,246 and 5,068,323. A significant problem with many immunosuppressants is their low therapeutic index, requiring the administration of high doses that can have significant toxic side effects.

Where a partial or complete organ transplant is desired, the morphogen may be administered to transplant tissue to enhance the viability of the tissue, to alleviate the tissue damage associated with immune response-mediated tissue destruction and/or to provide a cytoprotective effect to tissue at risk for such damage. Exemplary transplant tissues include hepatic tissue grafts which may be allogenic, autologous and/or synthetic (e.g., cultured cells attached to an artificial matrix), and whole or partial livers. Where the transplant tissue (e.g., liver, lung, kidney, pancreas, heart, etc.) is to be harvested from a donor host, the morphogen also preferably is provided to the tissue prior to, or concommitant with the tissue harvest, e.g., as a prophylactic, to provide a cytoprotective effect to the tissue.

The morphogens described herein also may be used in a gene therapy protocol and/or as part of a drug delivery protocol to correct a protein deficiency in a mammal, resulting, for example, from a genetic disorder or other dysfunction to the protein-producing tissue. Specifically, the methods and compositions find application in providing to the mammal an in vitro protein-producing mechanism for correcting any protein deficiency in the mammal. These proteins include proteins normally expressed and/or secreted by hepatic tissue and which play a role in liver-related functions, proteins normally expressed and secreted by the liver and which function elsewhere in the body, and proteins not normally expressed by hepatic tissue. Cells competent for expressing one or more proteins necessary to overcome the protein deficiency in vivo may be stimulated to proliferate ex vivo, and then implanted at a morphogenically permissive site at a liver-specific tissue locus in vivo. The competent cells may be attached to a scaffold-like structure prior to implantation. Alternatively, the competent cells may be attached to a synthetic or formulated matrix and implanted together with a morphogen at an extra-hepatic site in vivo, such as within the folds of the mesentery, or other associated vascularized tissue locus capable of providing the necessary nutrients and gas exchange to the cells. A detailed description of useful extra-hepatic loci are described, for example, in WO90/12604, published November 1, 1990 to Vacanti et al.

Exposing primary hepatocytes to a morphogen stimulates their proliferation (see below), thereby enhancing their cellular viability upon implantation, accelerating tissue development, and reducing the original cell population required to seed the matrix. In addition, implantation with a morphogen eliminates the need for partial hepatectomy to stimulate proliferation, and enhances cellular viability by inhibiting the inflammatory/immune response typically associated with such a procedure, overcoming the significant hepatocyte cell loss typically seen in this procedure.

Cells competent for correcting a protein deficiency include allogenic primary hepatocytes, preferably from a serotypically compatible individual and competent for expressing the protein or proteins of interest, and autologous cells transfected with the genetic material necessary to express the protein of interest. For example, primary hepatocytes may be removed from the patient by biopsy, transfected using standard recombinant DNA technology, proliferated, attached to a matrix and reimplanted together with a morphogen. Preferably the morphogen is provided to the cells during transfection and proliferation to enhance the mitogenic activity (and nucleic acid uptake) of these cells. In a currently preferred embodiment, morphogen is adsorbed to the matrix surface to which the cells are attached and the complex implanted as a single entity ("cell-matrix structure".)

As used herein "administration of morphogen" refers to the administration of the morphogen, either alone or in combination with other molecules. For example, the mature form of the morphogen may be provided in association with its precursor "pro" domain, which is known to enhance the solubility of the protein. Alternatively, the pro form of the morphogen (e.g., defined, for example, by residues 30-431 of OP1, Seq. I.D. No. 16, see below) may be used. Other useful molecules known to enhance protein solubility include casein and other milk components, as well as various serum proteins. Additional useful molecules which may be associated with the morphogen or morphogen-stimulating agent include tissue targeting molecules capable of directing the morphogen or morphogen-stimulating agent to hepatic tissue. Tissue targeting molecules envisioned to be useful in the treatment protocols of this invention include antibodies, antibody fragments or other binding proteins which interact specifically with surface molecules on nerve tissue cells. Still another useful tissue targeting molecule may include part or all of the morphogen precursor "pro" domain.

Associated tissue targeting or solubility-enhancing molecules also may be covalently linked to the morphogen using standard chemical means, including acid-labile linkages, which likely will be preferentially cleaved in the acidic environment of bone remodeling sites.

The morphogens and morphogen-stimulating agents also may be provided to the liver tissue together with other molecules ("cofactors") known to have a beneficial effect in treating damaged hepatic tissue, particularly cofactors capable of mitigating or alleviating symptoms typically associated with hepatic tissue damage and/or loss. Examples of such cofactors include antiseptics, antibiotics, tetracycline, aminoglycosides, macrolides, penicillins and cephalosporins, and other, non-steroidal anti-inflammatory agents.

Among the morphogens useful in this invention are proteins originally identified as osteogenic proteins, such as the OP-1, OP-2 and CBMP2 proteins, as well as amino acid sequence-related proteins such as DPP (from Drosophila), Vgl (from Xenopus), Vgr-1 (from mouse, see U.S. 5,011,691 to Oppermann et al.), GDF-1 (from mouse, see Lee (1991) PNAS 88:4250-4254), all of which are presented in Table II and Seq. ID Nos.5-14), and the recently identified 60A protein (from Drosophila, Seq. ID No. 24, see Wharton et al. (1991) PNAS 88:9214-9218.) The members of this family, which include members of the TGF-β super-family of proteins, share substantial amino acid sequence homology in their C-terminal regions. The proteins are translated as a precursor, having an N-terminal signal peptide sequence, typically less than about 30 residues, followed by a "pro" domain that is cleaved to yield the mature sequence. The "pro" form of the protein, includes both the pro domain and the mature domain, and forms a soluble species that apprears to be the primary form secreted from cultured mammalian cells. The signal peptide is cleaved rapidly upon translation, at a cleavage site that can be predicted in a given sequence using the method of Von Heijne ((1986) Nucleic Acids Research 14:4683-4691.) Table I, below, describes the various morphogens identified to date, including their nomenclature as used herein, their Seq. ID references, and publication sources for the amino acid sequences for the full length proteins not included in the Seq. Listing.

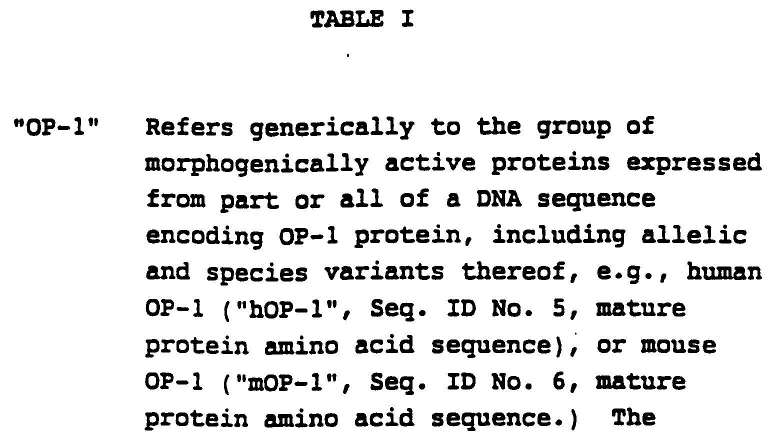

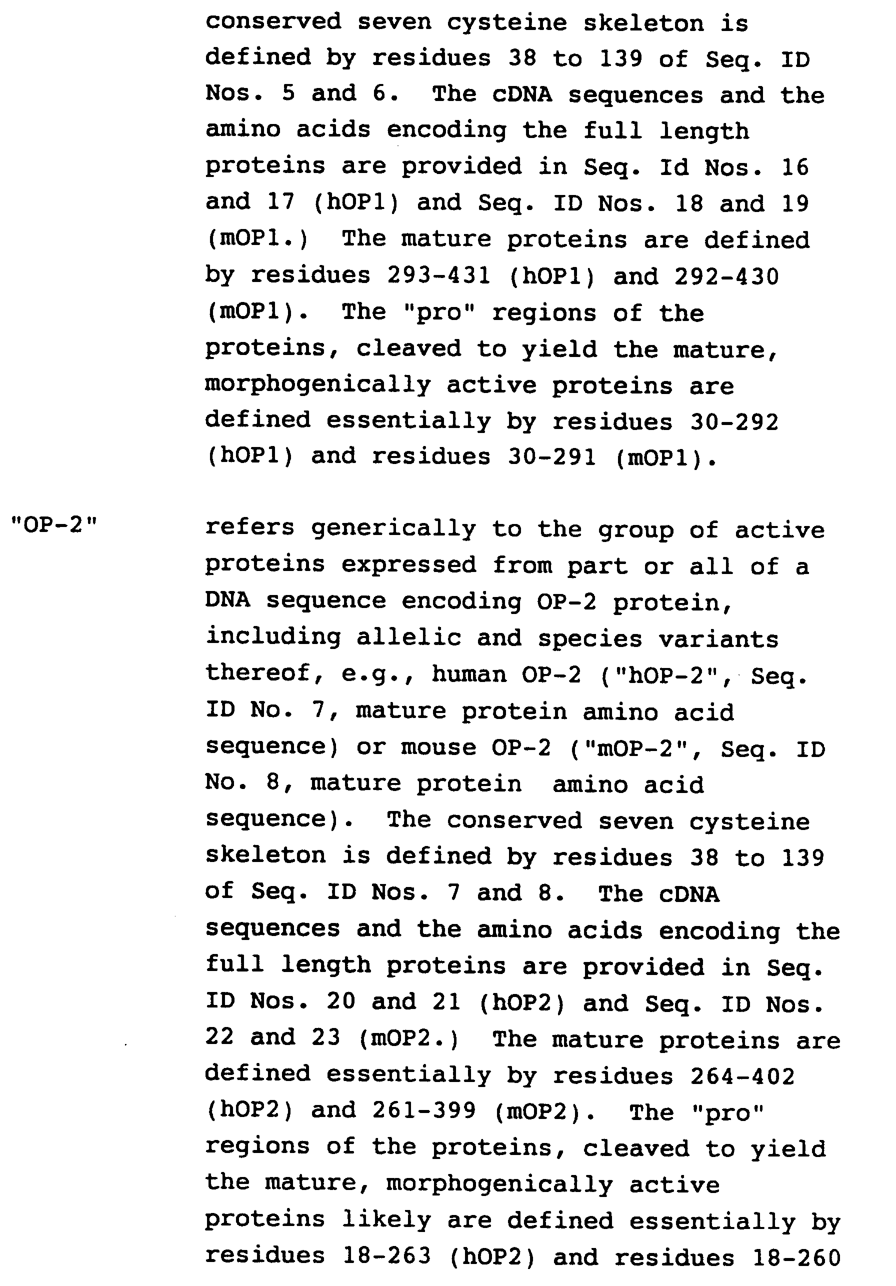

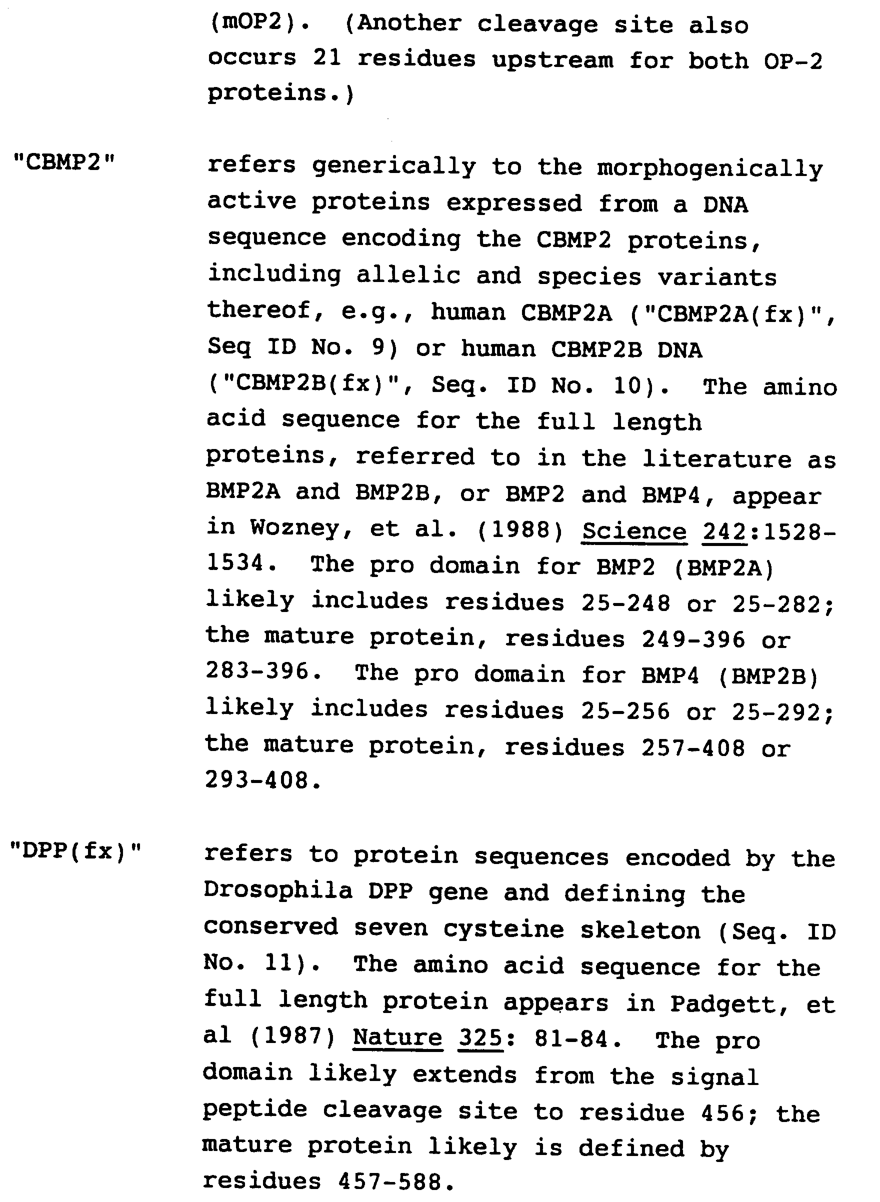

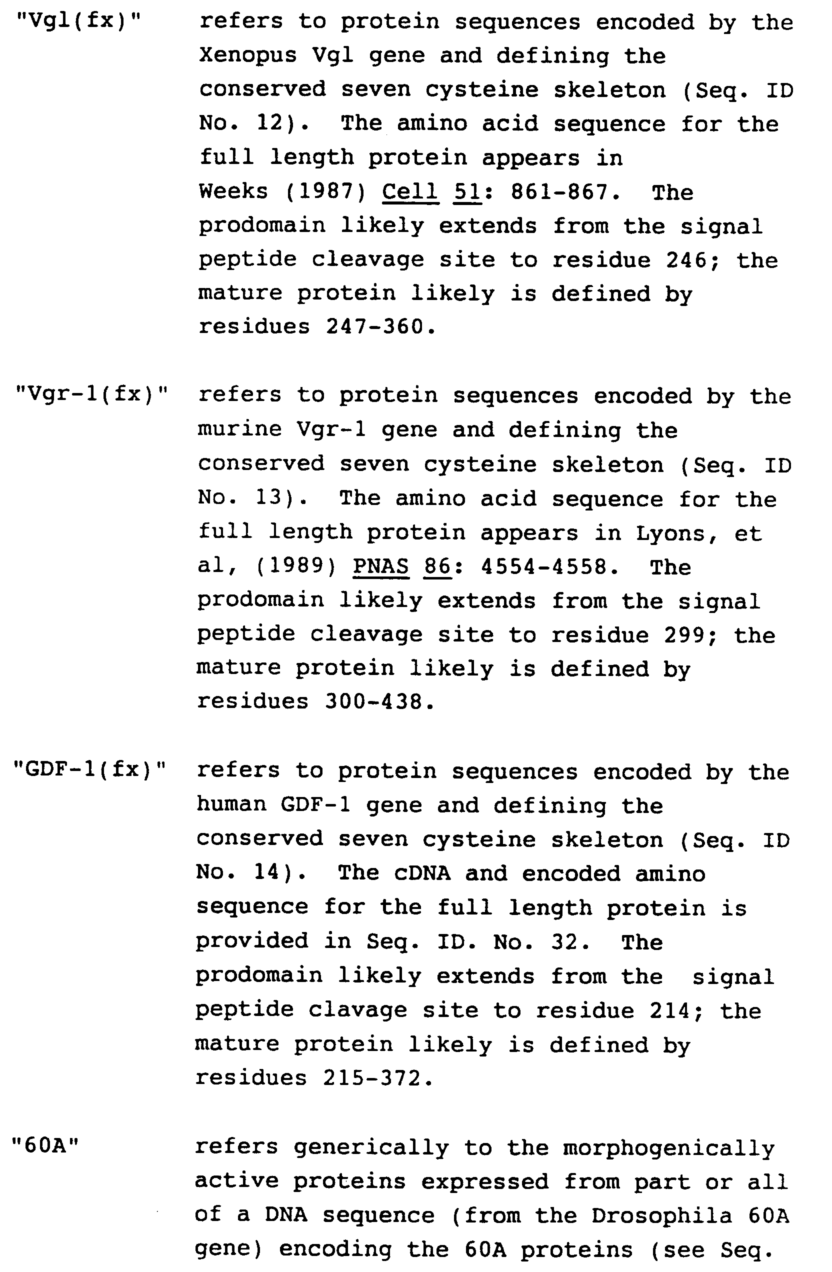

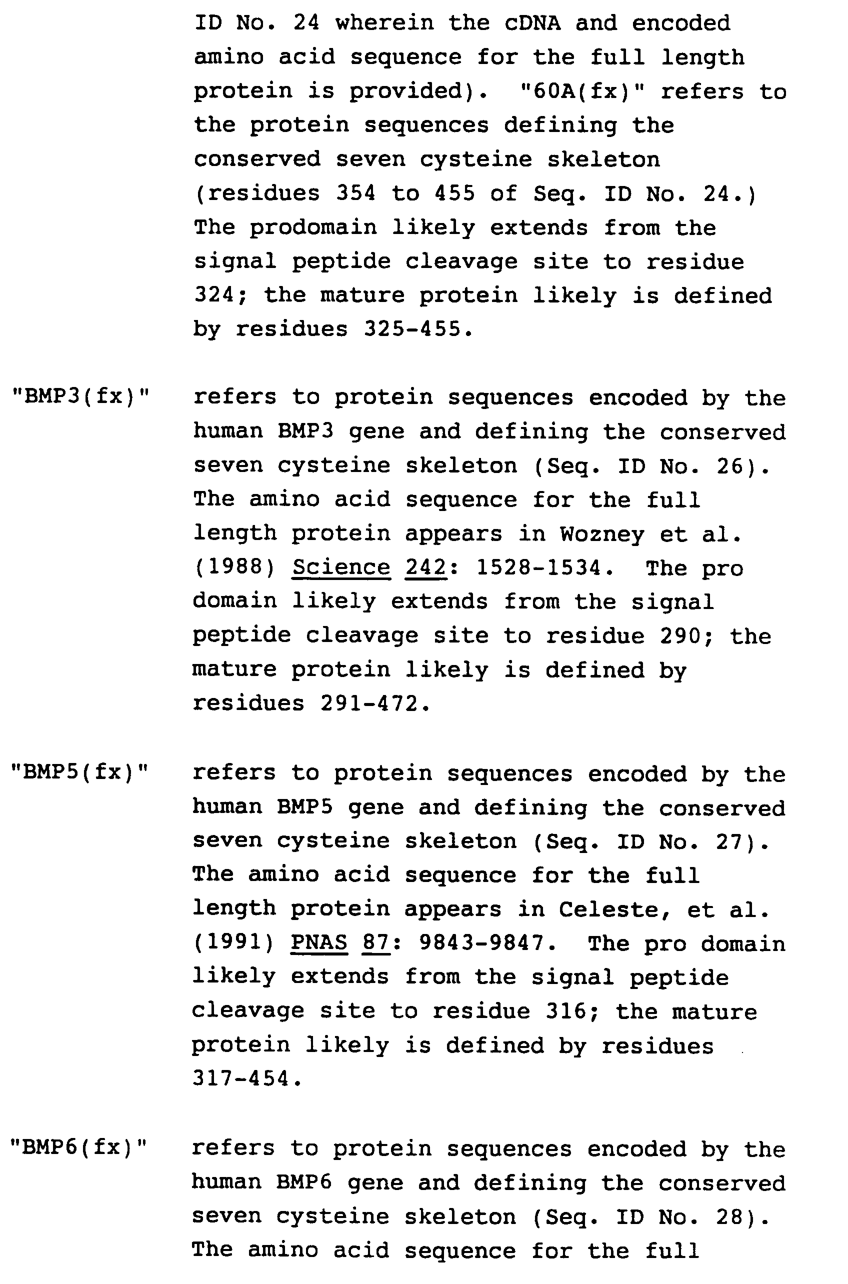

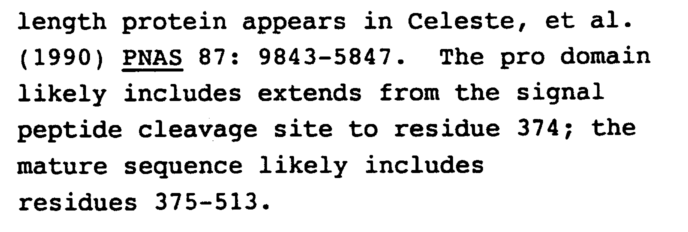

The OP-2 proteins have an additional cysteine residue in this region (e.g., see residue 41 of Seq. ID Nos. 7 and 8), in addition to the conserved cysteine skeleton in common with the other proteins in this family. The GDF-1 protein has a four amino acid insert within the conserved skeleton (residues 44-47 of Seq. ID No. 14) but this insert likely does not interfere with the relationship of the cysteines in the folded structure. In addition, the CBMP2 proteins are missing one amino acid residue within the cysteine skeleton.

The morphogens are inactive when reduced, but are active as oxidized homodimers and when oxidized in combination with other morphogens of this invention. Thus, as defined herein, a morphogen is a dimeric protein comprising a pair of polypeptide chains, wherein each polypeptide chain comprises at least the C-terminal six cysteine skeleton defined by residues 43-139 of Seq. ID No. 5, including functionally equivalent arrangements of these cysteines (e.g., amino acid insertions or deletions which alter the linear arrangement of the cysteines in the sequence but not their relationship in the folded structure), such that, when the polypeptide chains are folded, the dimeric protein species comprising the pair of polypeptide chains has the appropriate three-dimensional structure, including the appropriate intra- or inter-chain disulfide bonds such that the protein is capable of acting as a morphogen as defined herein. Specifically, the morphogens generally are capable of all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells, including the "redifferentiation" of transformed cells. In addition, it is also anticipated that these morphogens are capable of inducing redifferentiation of committed cells under appropriate environmental conditions.

In one preferred aspect, the morphogens of this invention comprise one of two species of generic amino acid sequences: Generic Sequence 1 (Seq. ID No. 1) or Generic Sequence 2 (Seq. ID No. 2); where each Xaa indicates one of the 20 naturally-occurring L-isomer, α-amino acids or a derivative thereof. Generic Sequence 1 comprises the conserved six cysteine skeleton and Generic Sequence 2 comprises the conserved six cysteine skeleton plus the additional cysteine identified in OP-2 (see residue 36, Seq. ID No. 2). In another preferred aspect, these sequences further comprise the following additional sequence at their N-terminus:

Cys Xaa Xaa Xaa Xaa (Seq. ID No. 15)
1 5

Preferred amino acid sequences within the foregoing generic sequences include: Generic Sequence 3 (Seq. ID No. 3), Generic Sequence 4 (Seq. ID No. 4), Generic Sequence 5 (Seq. ID No. 30) and Generic Sequence 6 (Seq. ID No. 31), listed below. These Generic Sequences accommodate the homologies shared among the various preferred members of this morphogen family identified in Table II, as well as the amino acid sequence variation among them. Specifically, Generic Sequences 3 and 4 are composite amino acid sequences of the following proteins presented in Table II and identified in Seq. ID Nos. 5-14: human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-22), CBMP2A (Seq. ID No. 9), CBMP2B (Seq. ID No. 10), DPP (from Drosophila, Seq. ID No. 11), Vgl, (from Xenopus, Seq. ID No. 12), Vgr-1 (from mouse, Seq. ID No. 13), and GDF-1 (from mouse, Seq. ID No. 14.) The generic sequences include both the amino acid identity shared by the sequences in Table II, as well as alternative residues for the variable positions within the sequence. Note that these generic sequences allow for an additional cysteine at position 41 or 46 in Generic Sequences 3 or 4, respectively, providing an appropriate cysteine skeleton where inter- or intramolecular disulfide bonds can form, and contain certain critical amino acids which influence the tertiary structure of the proteins. 
Generic Sequence 3

Leu Tyr Val Xaa Phe
1               5
Xaa Xaa Xaa Gly Trp Xaa Xaa Trp Xaa
                10
Xaa Ala Pro Xaa Gly Xaa Xaa Ala
15                  20
Xaa Tyr Cys Xaa Gly Xaa Cys Xaa
 
        25                  30
Xaa Pro Xaa Xaa Xaa Xaa Xaa
                35
Xaa Xaa Xaa Asn His Ala Xaa Xaa
        40                  45
Xaa Xaa Leu Xaa Xaa Xaa Xaa Xaa
                50
Xaa Xaa Xaa Xaa Xaa Xaa Xaa Cys
    55                  60
Cys Xaa Pro Xaa Xaa Xaa Xaa Xaa
            65
Xaa Xaa Xaa Leu Xaa Xaa Xaa
70                  75
Xaa Xaa Xaa Xaa Val Xaa Leu Xaa
            80
Xaa Xaa Xaa Xaa Met Xaa Val Xaa
85                  90
Xaa Cys Gly Cys Xaa
        95
 wherein each Xaa is independently selected from a group of one or more specified amino acids defined as follows: "Res." means "residue" and Xaa at res.4 = (Ser, Asp or Glu); Xaa at res.6 = (Arg, Gln, Ser or Lys); Xaa at res.7 = (Asp or Glu); Xaa at res.8 = (Leu or Val); Xaa at res.11 = (Gln, Leu, Asp, His or Asn); Xaa at res.12 = (Asp, Arg or Asn); Xaa at res.14 = (Ile or Val); Xaa at res.15 = (Ile or Val); Xaa at res.18 = (Glu, Gln, Leu, Lys, Pro or Arg); Xaa at res.20 = (Tyr or Phe); Xaa at res.21 = (Ala, Ser, Asp, Met, His, Leu or Gln); Xaa at res.23 = (Tyr, Asn or Phe); Xaa at res.26 = (Glu, His, Tyr, Asp or Gln); Xaa at res.28 = (Glu, Lys, Asp or Gln); Xaa at res.30 = (Ala, Ser, Pro or Gln); Xaa at res.31 = (Phe, Leu or Tyr); Xaa at res.33 = (Leu or Val); Xaa at res.34 = (Asn, Asp, Ala or Thr); Xaa at res.35 = (Ser, Asp, Glu, Leu or Ala); Xaa at res.36 = (Tyr, Cys, His, Ser or Ile); Xaa at res.37 = (Met, Phe, Gly or Leu); Xaa at res.38 = (Asn or Ser); Xaa at res.39 = (Ala, Ser or Gly); Xaa at res.40 = (Thr, Leu or Ser); Xaa at res.44 = (Ile or Val); Xaa at res.45 = (Val or Leu); Xaa at res.46 = (Gln or Arg); Xaa at res.47 = (Thr, Ala or Ser); Xaa at res.49 = (Val or Met); Xaa at res.50 = (His or Asn); Xaa at res.51 = (Phe, Leu, Asn, Ser, Ala or Val); Xaa at res.52 = (Ile, Met, Asn, Ala or Val); Xaa at res.53 = (Asn, Lys, Ala or Glu); Xaa at res.54 = (Pro or Ser); Xaa at res.55 = (Glu, Asp, Asn, or Gly); Xaa at res.56 = (Thr, Ala, Val, Lys, Asp, Tyr, Ser or Ala); Xaa at res.57 = (Val, Ala or Ile); Xaa at res.58 = (Pro or Asp); Xaa at res.59 = (Lys or Leu); Xaa at res.60 = (Pro or Ala); Xaa at res.63 = (Ala or Val); Xaa at res.65 = (Thr or Ala); Xaa at res.66 = (Gln, Lys, Arg or Glu); Xaa at res.67 = (Leu, Met or Val); Xaa at res.68 = (Asn, Ser or Asp); Xaa at res.69 = (Ala, Pro or Ser); Xaa at res.70 = (Ile, Thr or Val); Xaa at res.71 = (Ser or Ala); Xaa at res.72 = (Val or Met); Xaa at res.74 = (Tyr or Phe); Xaa at res.75 = (Phe, Tyr or Leu); Xaa at res.76 = (Asp or Asn); Xaa at res.77 = (Asp, Glu, Asn or Ser); Xaa at res.78 = (Ser, Gln, Asn or Tyr); Xaa at res.79 = (Ser, Asn, Asp or Glu); Xaa at res.80 = (Asn, Thr or Lys); Xaa at res.82 = (Ile or Val); Xaa at res.84 = (Lys or Arg); Xaa at res.85 = (Lys, Asn, Gln or His); Xaa at res.86 = (Tyr or His); Xaa at res.87 = (Arg, Gln or Glu); Xaa at res.88 = (Asn, Glu or Asp); Xaa at res.90 = (Val, Thr or Ala); Xaa at res.92 = (Arg, Lys, Val, Asp or Glu); Xaa at res.93 = (Ala, Gly or Glu); and Xaa at res.97 = (His or Arg); 
Generic Sequence 4

Cys Xaa Xaa Xaa Xaa Leu Tyr Val Xaa Phe
1               5                   10
Xaa Xaa Xaa Gly Trp Xaa Xaa Trp Xaa
                15
Xaa Ala Pro Xaa Gly Xaa Xaa Ala
20                  25
Xaa Tyr Cys Xaa Gly Xaa Cys Xaa
        30                  35
Xaa Pro Xaa Xaa Xaa Xaa Xaa
                40
Xaa Xaa Xaa Asn His Ala Xaa Xaa
        45                  50
Xaa Xaa Leu Xaa Xaa Xaa Xaa Xaa
                55
Xaa Xaa Xaa Xaa Xaa Xaa Xaa Cys
    60                  65
Cys Xaa Pro Xaa Xaa Xaa Xaa Xaa
            70
Xaa Xaa Xaa Leu Xaa Xaa Xaa
75                  80
Xaa Xaa Xaa Xaa Val Xaa Leu Xaa
            85
Xaa Xaa Xaa Xaa Met Xaa Val Xaa
90                  95
Xaa Cys Gly Cys Xaa
        100
 wherein each Xaa is independently selected from a group of one or more specified amino acids as defined by the following: "Res." means "residue" and Xaa at res.2 = (Lys or Arg); Xaa at res.3 = (Lys or Arg); Xaa at res.4 = (His or Arg); Xaa at res.5 = (Glu, Ser, His, Gly, Arg or Pro); Xaa at res.9 = (Ser, Asp or Glu); Xaa at res.11 = (Arg, Gln, Ser or Lys); Xaa at res.12 = (Asp or Glu); Xaa at res.13 = (Leu or Val); Xaa at res.16 = (Gln, Leu, Asp, His or Asn); Xaa at res.17 = (Asp, Arg, or Asn); Xaa at res.19 = (Ile or Val); Xaa at res.20 = (Ile or Val); Xaa at res.23 = (Glu, Gln, Leu, Lys, Pro or Arg); Xaa at res.25 = (Tyr or Phe); Xaa at res.26 = (Ala, Ser, Asp, Met, His, Leu, or Gln); Xaa at res.28 = (Tyr, Asn or Phe); Xaa at res.31 = (Glu, His, Tyr, Asp or Gln); Xaa at res.33 = Glu, Lys, Asp or Gln); Xaa at res.35 = (Ala, Ser or Pro); Xaa at res.36 = (Phe, Leu or Tyr); Xaa at res.38 = (Leu or Val); Xaa at res.39 = (Asn, Asp, Ala or Thr); Xaa at res.40 = (Ser, Asp, Glu, Leu or Ala); Xaa at res.41 = (Tyr, Cys, His, Ser or Ile); Xaa at res.42 = (Met, Phe, Gly or Leu); Xaa at res.44 = (Ala, Ser or Gly); Xaa at res.45 = (Thr, Leu or Ser); Xaa at res.49 = (Ile or Val); Xaa at res.50 = (Val or Leu); Xaa at res.51 = (Gln or Arg); Xaa at res.52 = (Thr, Ala or Ser); Xaa at res.54 = (Val or Met); Xaa at res.55 = (His or Asn); Xaa at res.56 = (Phe, Leu, Asn, Ser, Ala or Val); Xaa at res.57 = (Ile, Met, Asn, Ala or Val); Xaa at res.58 = (Asn, Lys, Ala or Glu); Xaa at res.59 = (Pro or Ser); Xaa at res.60 = (Glu, Asp, or Gly); Xaa at res.61 = (Thr, Ala, Val, Lys, Asp, Tyr, Ser or Ala); Xaa at res.62 = (Val, Ala or Ile); Xaa at res.63 = (Pro or Asp); Xaa at res.64 = (Lys or Leu); Xaa at res.65 = (Pro or Ala); Xaa at res.68 = (Ala or Val); Xaa at res.70 = (Thr or Ala); Xaa at res.71 = (Gln, Lys, Arg or Glu); Xaa at res.72 = (Leu, Met or Val); Xaa at res.73 = (Asn, Ser or Asp); Xaa at res.74 = (Ala, Pro or Ser); Xaa at res.75 = (Ile, Thr or Val); Xaa at res.76 = (Ser or Ala); Xaa at res.77 = (Val or Met); Xaa at res.79 = (Tyr or Phe); Xaa at res.80 = (Phe, Tyr or Leu); Xaa at res.81 = (Asp or Asn); Xaa at res.82 = (Asp, Glu, Asn or Ser); Xaa at res.83 = (Ser, Gln, Asn or Tyr); Xaa at res.84 = (Ser, Asn, Asp or Glu); Xaa at res.85 = (Asn, Thr or Lys); Xaa at res.87 = (Ile or Val); Xaa at res.89 = (Lys or Arg); Xaa at res.90 = (Lys, Asn, Gln or His); Xaa at res.91 = (Tyr or His); Xaa at res.92 = (Arg, Gln or Glu); Xaa at res.93 = (Asn, Glu or Asp); Xaa at res.95 = (Val, Thr or Ala); Xaa at res.97 = (Arg, Lys, Val, Asp or Glu); Xaa at res.98 = (Ala, Gly or Glu); and Xaa at res.102 = (His or Arg).

Similarly, Generic Sequence 5 (Seq. ID No. 30) and Generic Sequence 6 (Seq. ID No. 31) accommodate the homologies shared among all the morphogen protein famiiy members identified in Table II. Specifically, Generic Sequences 5 and 6 are composite amino acid sequences of human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-22), CBMP2A (Seq. ID No. 9), CBMP2B (Seq. ID No. 10), DPP (from Drosophila, Seq. ID No. 11), Vgl, (from Xenopus, Seq. ID No. 12), Vgr-1 (from mouse, Seq. ID No. 13), and GDF-1 (from mouse, Seq. ID No. 14), human BMP3 (Seq. ID No. 26), human BMP5 (Seq. ID No. 27), human BMP6 (Seq. ID No. 28) and 60(A) (from Drosophila, Seq. ID Nos. 24-25). The generic sequences include both the amino acid identity shared by these sequences in the C-terminal domain, defined by the six and seven cysteine skeletons (Generic Sequences 5 and 6, respectively), as well as alternative residues for the variable positions within the sequence. As for Generic Sequences 3 and 4, Generic sequences 5 and 6 allow for an additional cysteine at position 41 (Generic sequence 5) or position 46 (Generic sequence 6), providing an appropriate cysteine skeleton where inter- or intramolecular disulfide bonds can form, and containing certain critical amino acids which influence the tertiary structure of the proteins. 
Generic Sequence 5

Leu Xaa Xaa Xaa Phe
1               5
Xaa Xaa Xaa Gly Trp Xaa Xaa Trp Xaa
                10
Xaa Xaa Pro Xaa Xaa Xaa Xaa Ala
15                  20
Xaa Tyr Cys Xaa Gly Xaa Cys Xaa
        25                  30
Xaa Pro Xaa Xaa Xaa Xaa Xaa
                35
Xaa Xaa Xaa Asn His Ala Xaa Xaa
        40                  45
Xaa Xaa Xaa Xaa Xaa Xaa Xaa Xaa
                50
Xaa Xaa Xaa Xaa Xaa Xaa Xaa Cys
    55                  60
Cys Xaa Pro Xaa Xaa Xaa Xaa Xaa
            65
Xaa Xaa Xaa Leu Xaa Xaa Xaa
 
70                          75
Xaa Xaa Xaa Xaa Val Xaa Leu Xaa
                80
Xaa Xaa Xaa Xaa Met Xaa Val Xaa
85                          90
Xaa Cys Xaa Cys Xaa
            95
 wherein each Xaa is independently selected from a group of one or more specified amino acids defined as follows: "Res." means "residue" and Xaa at res.2 = (Tyr or Lys); Xaa at res.3 = Val or Ile); Xaa at res.4 = (Ser, Asp or Glu); Xaa at res.6 = (Arg, Gln, Ser, Lys or Ala); Xaa at res.7 = (Asp, Glu or Lys); Xaa at res.8 = (Leu, Val or Ile); Xaa at res.11 = (Gln, Leu, Asp, His, Asn or Ser); Xaa at res.12 = (Asp, Arg, Asn or Glu); Xaa at res.14 = (Ile or Val); Xaa at res.15 = (Ile or Val); Xaa at res.16 (Ala or Ser); Xaa at res.18 = (Glu, Gln, Leu, Lys, Pro or Arg); Xaa at res.19 = (Gly or Ser); Xaa at res.20 = (Tyr or Phe); Xaa at res.21 = (Ala, Ser, Asp, Met, His, Gln, Leu or Gly); Xaa at res.23 = (Tyr, Asn or Phe); Xaa at res.26 = (Glu, His, Tyr, Asp, Gln or Ser); Xaa at res.28 = (Glu, Lys, Asp, Gln or Ala); Xaa at res.30 = (Ala, Ser, Pro, Gln or Asn); Xaa at res.31 = (Phe, Leu or Tyr); Xaa at res.33 = (Leu, Val or Met); Xaa at res.34 = (Asn, Asp, Ala, Thr or Pro); Xaa at res.35 = (Ser, Asp, Glu, Leu, Ala or Lys); Xaa at res.36 = (Tyr, Cys, His, Ser or Ile); Xaa at res.37 = (Met, Phe, Gly or Leu); Xaa at res.38 = (Asn, Ser or Lys); Xaa at res.39 = (Ala, Ser, Gly or Pro); Xaa at res.40 = (Thr, Leu or Ser); Xaa at res.44 = (Ile, Val or Thr); Xaa at res.45 = (Val, Leu or Ile); Xaa at res.46 = (Gln or Arg); Xaa at res.47 = (Thr, Ala or Ser); Xaa at res.48 = (Leu or Ile); Xaa at res.49 = (Val or Met); Xaa at res.50 = (His, Asn or Arg); Xaa at res.51 = (Phe, Leu, Asn, Ser, Ala or Val); Xaa at res.52 = (Ile, Met, Asn, Ala, Val or Leu); Xaa at res.53 = (Asn, Lys, Ala, Glu, Gly or Phe); Xaa at res.54 = (Pro, Ser or Val); Xaa at res.55 = (Glu, Asp, Asn, Gly, Val or Lys); Xaa at res.56 = (Thr, Ala, Val, Lys, Asp, Tyr, Ser, Ala, Pro or His); Xaa at res.57 = (Val, Ala or Ile); Xaa at res.58 = (Pro or Asp); Xaa at res.59 = (Lys, Leu or Glu); Xaa at res.60 = (Pro or Ala); Xaa at res.63 = (Ala or Val); Xaa at res.65 = (Thr, Ala or Glu); Xaa at res.66 = (Glu, Lys, Arg or Glu); Xaa at res.67 = (Leu, Met or Val); Xaa at res.68 = (Asn, Ser, Asp or Gly); Xaa at res.69 = (Ala, Pro or Ser); Xaa at res.70 = (Ile, Thr, Val or Leu); Xaa at res.71 = (Ser, Ala or Pro); Xaa at res.72 = (Val, Met or Ile); Xaa at res.74 = (Tyr or Phe); Xaa at res.75 = (Phe, Tyr, Leu or His); Xaa at res.76 = (Asp, Asn or Leu); Xaa at res.77 = (Asp, Glu, Asn or Ser); Xaa at res.78 = (Ser, Gln, Asn, Tyr or Asp); Xaa at res.79 = (Ser, Asn, Asp, Glu or Lys); Xaa at res.80 = (Asn, Thr or Lys); Xaa at res.82 = (Ile, Val or Asn); Xaa at res.84 = (Lys or Arg); Xaa at res.85 = (Lys, Asn, Gln, His or Val); Xaa at res.86 = (Tyr or His); Xaa at res.87 = (Arg, Gln, Glu or Pro); Xaa at res.88 = (Asn, Glu or Asp); Xaa at res.90 = (Val, Thr, Ala or Ile); Xaa at res.92 = (Arg, Lys, Val, Asp or Glu); Xaa at res.93 = (Ala, Gly, Glu or Ser); Xaa at res.95 = (Gly or Ala) and Xaa at res.97 = (His or Arg). 
Generic Sequence 6

Cys Xaa Xaa Xaa Xaa Leu Xaa Xaa Xaa Phe
1               5                   10
Xaa Xaa Xaa Gly Trp Xaa Xaa Trp Xaa
                15
Xaa Xaa Pro Xaa Xaa Xaa Xaa Ala
 
20                          25
Xaa Tyr Cys Xaa Gly Xaa Cys Xaa
          30                      35
Xaa Pro Xaa Xaa Xaa Xaa Xaa
                    40
Xaa Xaa Xaa Asn His Ala Xaa Xaa
          45                      50
Xaa Xaa Xaa Xaa Xaa Xaa Xaa Xaa
                    55
Xaa Xaa Xaa Xaa Xaa Xaa Xaa Cys
     60                      65
Cys Xaa Pro Xaa Xaa Xaa Xaa Xaa
               70
Xaa Xaa Xaa Leu Xaa Xaa Xaa
 75                      80
Xaa Xaa Xaa Xaa Val Xaa Leu Xaa
               85
Xaa Xaa Xaa Xaa Met Xaa Val Xaa
 90                      95
Xaa Cys Xaa Cys Xaa
          100
 wherein each Xaa is independently selected from a group of one or more specified amino acids as defined by the following: "Res." means "residue" and Xaa at res.2 = (Lys, Arg, Ala or Gln); Xaa at res.3 = (Lys, Arg or Met); Xaa at res.4 = (His, Arg or Gln); Xaa at res.5 = (Glu, Ser, His, Gly, Arg, Pro, Thr, or Tyr); Xaa at res.7 = (Tyr or Lys); Xaa at res.8 = (Val or Ile); Xaa at res.9 = (Ser, Asp or Glu); Xaa at res.11 = (Arg, Gln, Ser, Lys or Ala); Xaa at res.12 = (Asp, Glu, or Lys); Xaa at res.13 = (Leu, Val or Ile); Xaa at res.16 = (Glu, Leu, Asp, His, Asn or Ser); Xaa at res.17 = (Asp, Arg, Asn or Glu); Xaa at res.19 = (Ile or Val); Xaa at res.20 = (Ile or Val); Xaa at res.21 = (Ala or Ser); Xaa at res.23 = (Glu, Gln, Leu, Lys, Pro or Arg); Xaa at res.24 = (Gly or Ser); Xaa at res.25 = (Tyr or Phe); Xaa at res.26 = (Ala, Ser, Asp, Met, His, Gln, Leu, or Gly); Xaa at res.28 = (Tyr, Asn or Phe); Xaa at res.31 = (Glu, His, Tyr, Asp, Gln or Ser); Xaa at res.33 = Glu, Lys, Asp, Gln or Ala); Xaa at res.35 = (Ala, Ser, Pro, Gln or Asn); Xaa at res.36 = (Phe, Leu or Tyr); Xaa at res.38 = (Leu, Val or Met); Xaa at res.39 = (Asn, Asp, Ala, Thr or Pro); Xaa at res.40 = (Ser, Asp, Glu, Leu, Ala or Lys); Xaa at res.41 = (Tyr, Cys, His, Ser or Ile); Xaa at res.42 = (Met, Phe, Gly or Leu); Xaa at res.43 = (Asn, Ser or Lys); Xaa at res.44 = (Ala, Ser, Gly or Pro); Xaa at res.45 = (Thr, Leu or Ser); Xaa at res.49 = (Ile, Val or Thr); Xaa at res.50 = (Val, Leu or Ile); Xaa at res.51 = (Gln or Arg); Xaa at res.52 = (Thr, Ala or Ser); Xaa at res.53 = (Leu or Ile); Xaa at res.54 = (Val or Met); Xaa at res.55 = (His, Asn or Arg); Xaa at res.56 = (Phe, Leu, Asn, Ser, Ala or Val); Xaa at res.57 = (Ile, Met, Asn, Ala, Val or Leu); Xaa at res.58 = (Asn, Lys, Ala, Glu, Gly or Phe); Xaa at res.59 = (Pro, Ser or Val); Xaa at res.60 = (Glu, Asp, Gly, Val or Lys); Xaa at res.61 = (Thr, Ala, Val, Lys, Asp, Tyr, Ser, Ala, Pro or His); Xaa at res.62 = (Val, Ala or Ile); Xaa at res.63 = (Pro or Asp); Xaa at res.64 = (Lys, Leu or Glu); Xaa at res.65 = (Pro or Ala); Xaa at res.68 = (Ala or Val); Xaa at res.70 = (Thr, Ala or Glu); Xaa at rest.71 = (Gln, Lys, Arg or Glu); Xaa at res.72 = (Leu, Met or Val); Xaa at res.73 = (Asn, Ser, Asp or Gly); Xaa at res.74 = (Ala, Pro or Ser); Xaa at rest.75= (Ile, Thr, Val or Leu); Xaa at res.76 = (Ser, Ala or Pro); Xaa at res.77 = (Val, Met or Ile); Xaa at res.79 = (Tyr or Phe); Xaa at res.80 = (Phe, Tyr, Leu or His); Xaa at res.81 = (Asp, Asn or Leu); Xaa at res.82 = (Asp, Glu, Asn or Ser); Xaa at res.83 = (Ser, Gln, Asn, Tyr or Asp); Xaa at res.84 = (Ser, Asn, Asp, Glu or Lys); Xaa at res.85 = (Asn, Thr or Lys); Xaa at res.87 = (Ile, Val or Asn); Xaa at res.89 = (Lys or Arg); Xaa at res.90 = (Lys, Asn, Gln, His or Val); Xaa at res.91 = (Tyr or His); Xaa at res.92 = (Arg, Gln, Glu or Pro); Xaa at res.93 = (Asn, Glu or Asp); Xaa at res.95 = (Val, Thr, Ala or Ile); Xaa at res.97 = (Arg, Lys, Val, Asp or Glu); Xaa at res.98 = (Ala, Gly, Glu or Ser); Xaa at res.100 = (Gly or Ala); and Xaa at res.102 = (His or Arg).

Particularly useful sequences for use as morphogens in this invention include the C-terminal domains, e.g., the C-terminal 96-102 amino acid residues of Vgl, Vgr-1, DPP, OP-1, OP-2, CBMP-2A, CBMP-2B, GDF-1 (see Table II, below, and Seq. ID Nos. 5-14), as well as proteins comprising the C-terminal domains of 60A, BMP3, BMP5 and BMP6 (see Seq. ID Nos. 24-28), all of which include at least the conserved six or seven cysteine skeleton. In addition, biosynthetic constructs designed from the generic sequences, such as COP-1, 3-5, 7, 16, disclosed in U.S. Pat. No. 5,011,691, also are useful. Other sequences include the inhibins/activin proteins (see, for example, U.S. Pat. Nos. 4,968,590 and 5,011,691). Accordingly, other useful sequences are those sharing at least 70% amino acid sequence homology or "similarity", and preferably 80% homology or similarity with any of the sequences above. These are anticipated to include allelic, species variants and other sequence variants (e.g., including "muteins" or "mutant proteins"), whether naturally-occurring or biosynthetically produced, as well as novel members of this morphogenic family of proteins. As used herein, "amino acid sequence homology" is understood to mean amino acid sequence similarity, and homologous sequences share identical or similar amino acids, where similar amino acids are conserved amino acids as defined by Dayoff et al., Atlas of Protein Sequence and Structure; vol.5, Suppl.3, pp.345-362 (M.O. Dayoff, ed., Nat'l BioMed. Research Fdn., Washington D.C. 1978.) Thus, a candidate sequence sharing 70% amino acid homology with a reference sequence requires that, following alignment of the candidate sequence with the reference sequence, 70% of the amino acids in the candidate sequence are identical to the corresponding amino acid in the reference sequence, or constitute a conserved amino acid change thereto. "Amino acid sequence identity" is understood to require identical amino acids between two aligned sequences. Thus, a candidate sequence sharing 60% amino acid identity with a reference sequence requires that, following alignment of the candidate sequence with the reference sequence, 60% of the amino acids in the candidate sequence are identical to the corresponding amino acid in the reference sequence.

As used herein, all homologies and identities calculated use OP-1 as the reference sequence. Also as used herein, sequences are aligned for homology and identity calculations using the method of Needleman et al. (1970) J.Mol. Biol. 48:443-453 and identities calculated by the Align program (DNAstar, Inc.) In all cases, internal gaps and amino acid insertions in the candidate sequence as aligned are ignored when making the homology/identity calculation.

The currently most preferred protein sequences useful as morphogens in this invention include those having greater than 60% identity, preferably greater than 65% identity, with the amino acid sequence defining the conserved six cysteine skeleton of hOP1 (e.g., residues 43-139 of Seq. ID No. 5). These most preferred sequences include both allelic and species variants of the OP-1 and OP-2 proteins, including the Drosophila 60A protein. Accordingly, in another preferred aspect of the invention, useful morphogens include active proteins comprising species of polypeptide chains having the generic amino acid sequence herein referred to as "OPX", which accommodates the homologies between the various identified species of OP1 and OP2 (Seq. ID No. 29).

In still another preferred aspect of the invention, useful morphogens include dimeric proteins comprising amino acid sequences encoded by nucleic acids that hybridise to DNA or RNA sequences encoding the C-terminal sequences defining the conserved seven cysteine domain of OP1 or OP2, e.g., nucleotides 1036-1341 and nucleotides 1390-1695 of Seq. ID No. 16 and 20, respectively, under stringent hybridization conditions. As used herein, stringent hybridization conditions are defined as hybridisation in 40% formamide, 5 X SSPE, 5 X Denhardt's Solution, and 0.1% SDS at 37°C overnight, and washing in 0.1 X SSPE, 0.1% SDS at 50°C.

The morphogens useful in the methods, composition and devices of this invention include proteins comprising any of the polypeptide chains described above, whether isolated from naturally-occurring sources, or produced by recombinant DNA or other synthetic techniques, and includes allelic and species variants of these proteins, naturally-occurring or biosynthetic mutants thereof, as well as various truncated and fusion constructs. Deletion or addition mutants also are envisioned to be active, including those which may alter the conserved C-terminal cysteine skeleton, provided that the alteration does not functionally disrupt the relationship of these cysteines in the folded structure. Accordingly, such active forms are considered the equivalent of the specifically described constructs disclosed herein. The proteins may include forms having varying glycosylation patterns, varying N-termini, a family of related proteins having regions of amino acid sequence homology, and active truncated, chimeric and/or mutated forms of native or biosynthetic proteins, produced by expression of recombinant DNA in host cells.

The morphogenic proteins can be expressed from intact, chimeric and/or truncated cDNA or from synthetic DNAs in procaryotic or eucaryotic host cells, and purified, cleaved, refolded, and dimerized to form morphogenically active compositions. Currently preferred host cells include E. coli or mammalian cells, such as CHO, COS or BSC cells.

Thus, in view of this disclosure, skilled genetic engineers can isolate genes from cDNA or genomic libraries of various different species which encode appropriate amino acid sequences, or construct DNAs from oligonucleotides, and then can express them in various types of host cells, including both procaryotes and eucaryotes, to produce large quantities of active proteins capable of maintaining liver function in a mammal, including correcting liver function deficiencies and stimulating hepatic tissue regeneration and repair in a variety of mammals, including humans.

The foregoing and other objects, features and advantages of the present invention will be made more apparent from the following detailed description of the invention.

### Brief Description of the Drawings:

The foregoing and other objects and features of this invention, as well as the invention itself, may be more fully understood from the following description, when read together with the accompanying drawings, in which:
FIGURE 1 is a representation of a Northern blot identifying OP-1-specific mRNA expression in developing liver tissue in embryonic and postnatal mouse, wherein lanes 2 and 3 contained RNA from 15- and 20-day embryo tissue, respectively; lanes 4-8, RNA from 3, 7, 14, 21 and 28 days post natal animals, respectively; and lanes 1 and 9 were molecular weight marker ladders;
FIGURE 2 is a photomicrograph showing the effect of phosphate buffered saline (PBS, animal 1) or morphogen (OP-1, animal 2) on partially hepatectomized rats (arrow indicates the treated lobe in both animals);
FIGURE 3 is a representation of a Northern blot of mRNA isolated from sham-operated (lanes 3, 5, 7, 9, 11, 13 and 15) and partially hepatectomized rats (lanes 2, 4, 6, 8, 10, 12, 14) at 6 hr intervals between 12-96 hours post surgery, probed with an mOP-1-specific probe, and lanes 1 and 16 are molecular weight marker lanes;
FIGURE 4 is a representation of a Northern blot of mRNA isolated from galactosamine-treated rats and probed with mOP-1-specific probe on days 0-7, 10 (lanes 1-9, respectively, and lane 10 contains molecular weight markers);
FIGURE 5 (A and B) are schematic representations of morphogen inhibition of early mononuclear phagocytic cell multinuclearization in vivo; and
FIGURE 6 (A-D) graphs the effects of a morphogen (e.g., OP-1, Figs. 6A and 6C) and TGF-B (Fig. 6B and 6D) on collagen (6A and 6B) and hyaluronic acid (6C and 6D) production in primary fibroblast cultures.

### Detailed Description of the Invention

It now has been discovered that the proteins described herein are effective agents for maintaining liver function in a mammal. As described herein, these proteins ("morphogens") are capable of inducing hepatic tissue regeneration and repair under conditions where true tissue morphogenesis typically does not occur, including stimulating the proliferation and differentiation of hepatocytic progenitor cells. The proteins also are capable of providing a cytoprotective effect to alleviate the tissue destructive effects associated with immunologically-related hepatic tissue damage. Accordingly, the proteins may be used as part of a protocol for regenerating damaged or lost hepatic tissue, correcting a liver function deficiency, and enhancing the viability of a tissue or organ to be transplanted in a mammal. The morphogens also may be used in a gene therapy protocol to correct a protein deficiency in a mammal.

Provided below are detailed descriptions of suitable morphogens useful in the methods, compositions and devices of this invention, as well as methods for their administration and application, and numerous, nonlimiting examples which 1) illustrate the suitability of the morphogens and morphogen-stimulating agents described herein as therapeutic agents for maintaining liver function in a mammal; and 2) provide assays with which to test candidate morphogens and morphogen-stimulating agents for their efficacy. Specifically, the examples demonstrate the expression distribution of endogenous morphogen (Example 1), the expression of endogenous morphogen during liver formation in a developing embryo (Example 2), the ability of morphogens to induce proliferation of primary hepatocytes (Example 3), morphogen-induced liver tissue morphogenesis following partial hepatectomy (Example 4); endogenous morphogen expression during hepatic tissue repair following toxin-induced tissue damage (Examples 5); the inhibitory effect of morphogens on the body's cellular and humoral immune response (Example 6); effect of morphogen on fibrogenesis (Example 7); morphogen utility in liver diagnostic procedures (Example 8), and a screening assay for testing candidate morphogen-stimulating agents (Example 9).

### I. Useful Morphogens

As defined herein a protein is morphogenic if it is capable of inducing the developmental cascade of cellular and molecular events that culminate in the formation of new, organ-specific tissue and comprises at least the conserved C-terminal six cysteine skeleton or its functional equivalent (see supra). Specifically, the morphogens generally are capable of all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells.

As disclosed therein, the morphogens may be purified from naturally-sourced material or recombinantly produced from procaryotic or eucaryotic host cells, using the genetic sequences disclosed therein. Alternatively, novel morphogenic sequences may be identified following the procedures disclosed therein.

Particularly useful proteins include those which comprise the naturally derived sequences disclosed in Table II. Other useful sequences include biosynthetic constructs such as those disclosed in U.S. Pat. 5,011,691, the disclosure of which is incorporated herein by reference (e.g., COP-1, COP-3, COP-4, COP-5, COP-7, and COP-16).

Accordingly, the morphogens useful in the methods and compositions of this invention also may be described by morphogenically active proteins having amino acid sequences sharing 70% or, preferably, 80% homology (similarity) with any of the sequences described above, where "homology" is as defined herein above.

The morphogens useful in the method of this invention also can be described by any of the 6 generic sequences described herein (Generic sequences 1, 2, 3, 4, 5 and 6). Generic sequences 1 and 2 also may include, at their N-terminus, the sequence

Cys Xaa Xaa Xaa Xaa (Seq. ID No. 15)
1               5

Table II, set forth below, compares the amino acid sequences of the active regions of native proteins that have been identified as morphogens, including human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-23), CBMP2A (Seq. ID No. 9), CBMP2B (Seq. ID No. 10), BMP3 (Seq. ID No. 26), DPP (from Drosophila, Seq. ID No. 11), Vgl, (from Xenopus, Seq. ID No. 12), Vgr-1 (from mouse, Seq. ID No. 13), GDF-1 (from mouse, Seq. ID Nos. 14, 32 and 33), 60A protein (from Drosophila, Seq. ID Nos. 24 and 25), BMP5 (Seq. ID No. 27) and BMP6 (Seq. ID No. 28). The sequences are aligned essentially following the method of Needleman et al. (1970) J. Mol. Biol., 48:443-453, calculated using the Align Program (DNAstar, Inc.) In the table, three dots indicates that the amino acid in that position is the same as the amino acid in hOP-1. Three dashes indicates that no amino acid is present in that position, and are included for purposes of illustrating homologies. For example, amino acid residue 60 of CBMP-2A and CBMP-2B is "missing". Of course, both these amino acid sequences in this region comprise Asn-Ser (residues 58, 59), with CBMP-2A then comprising Lys and Ile, whereas CBMP-2B comprises Ser and Ile.

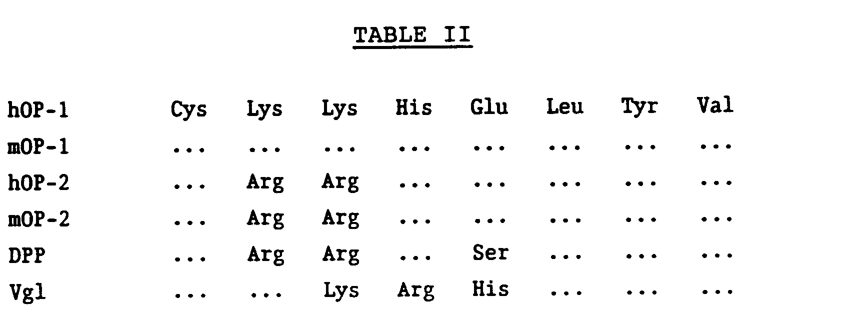

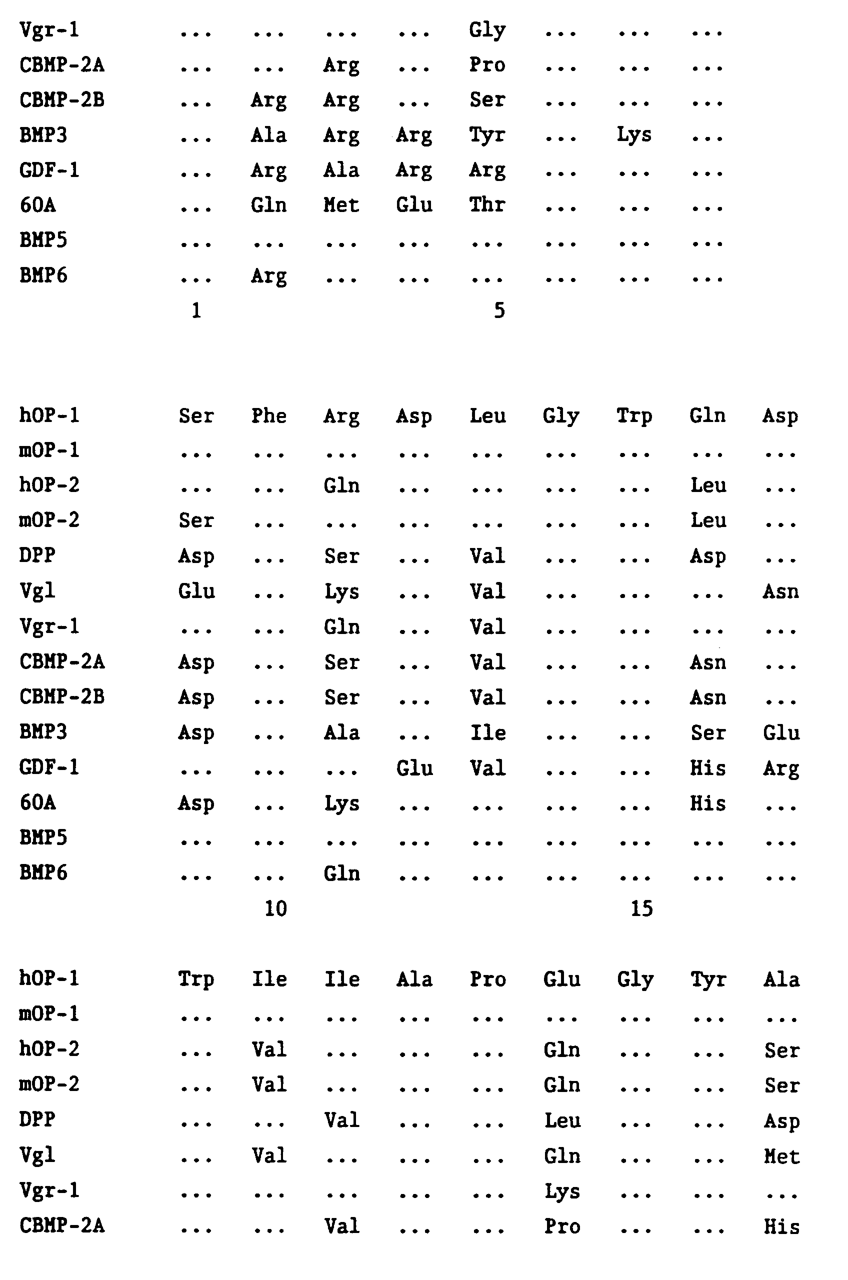

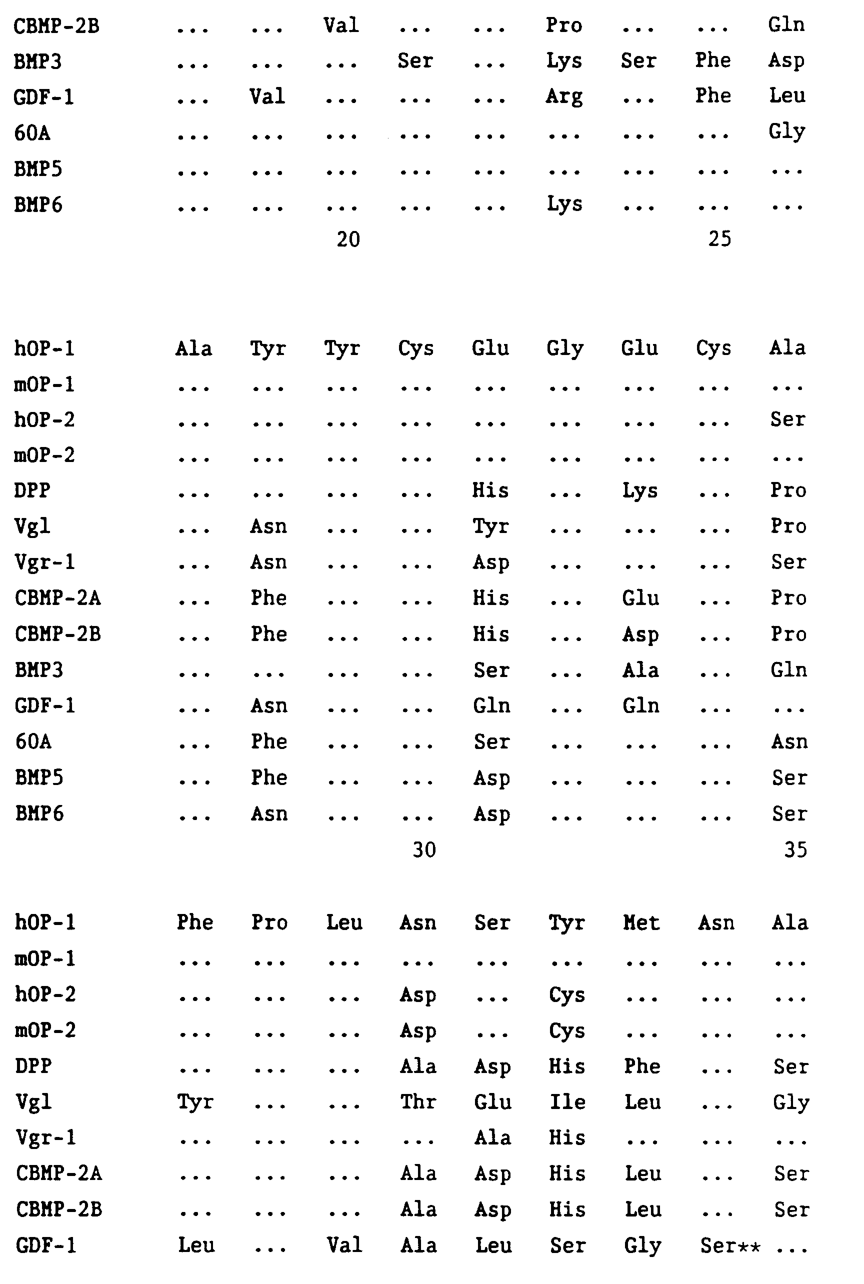

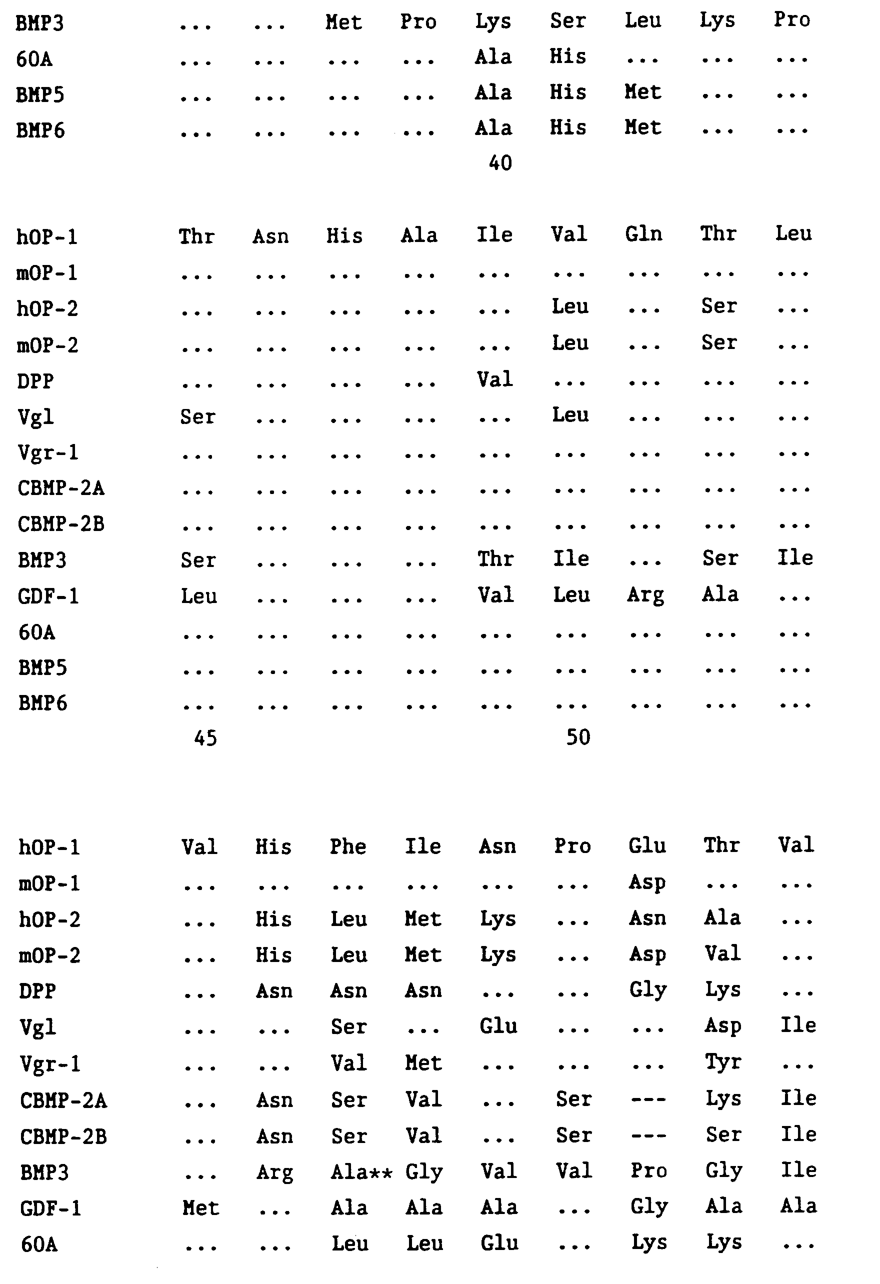

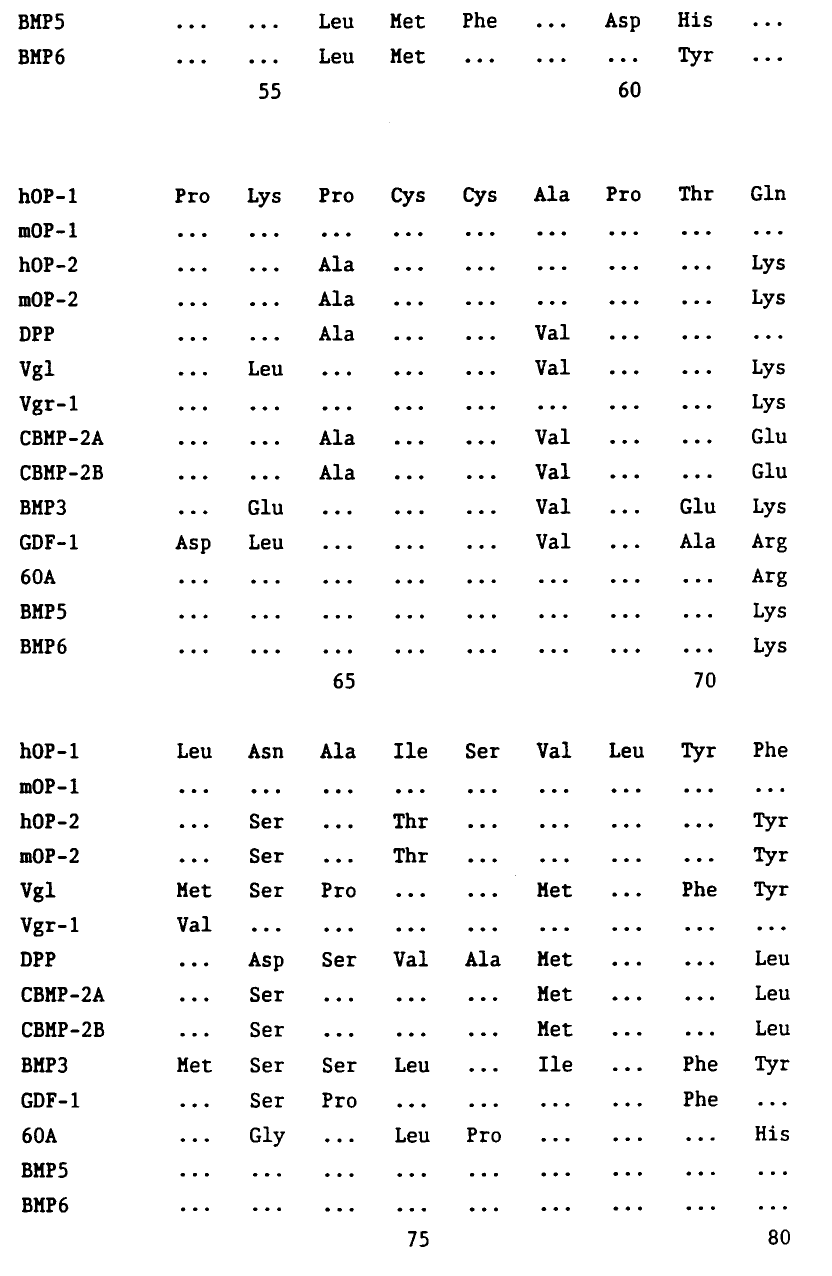

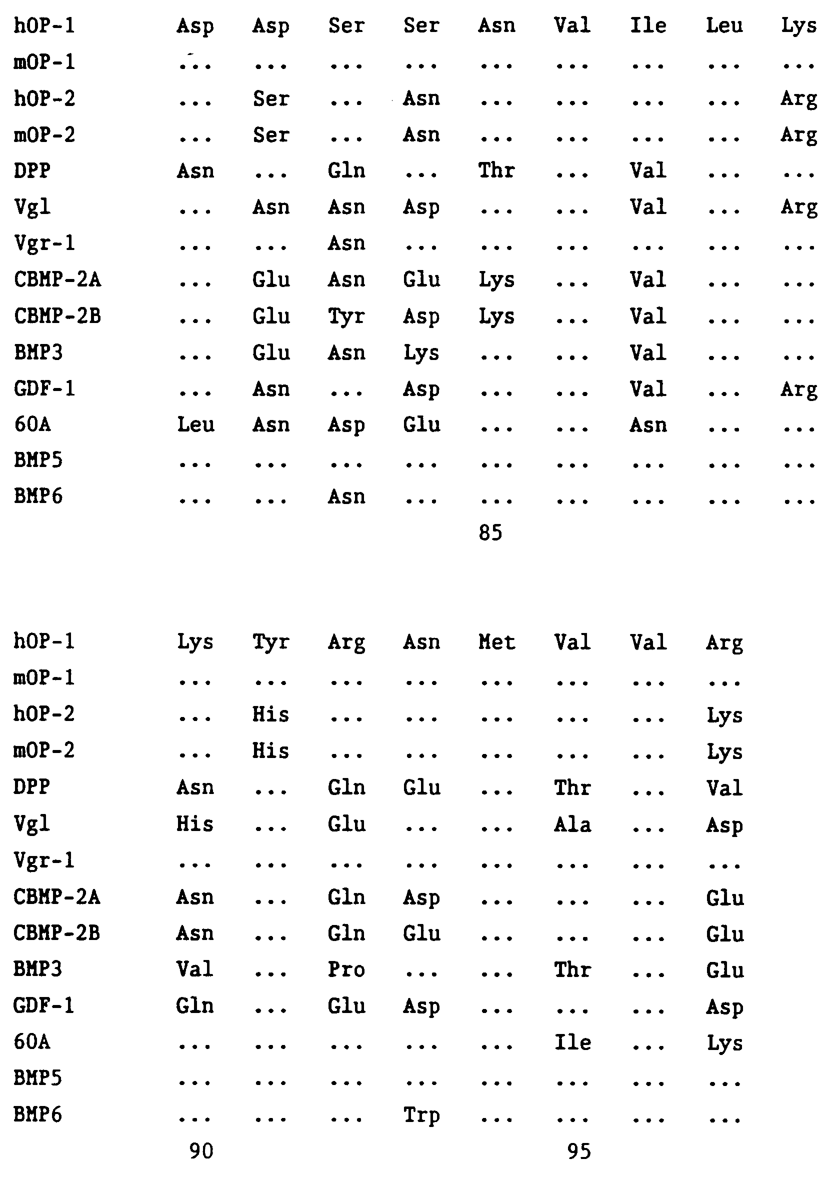

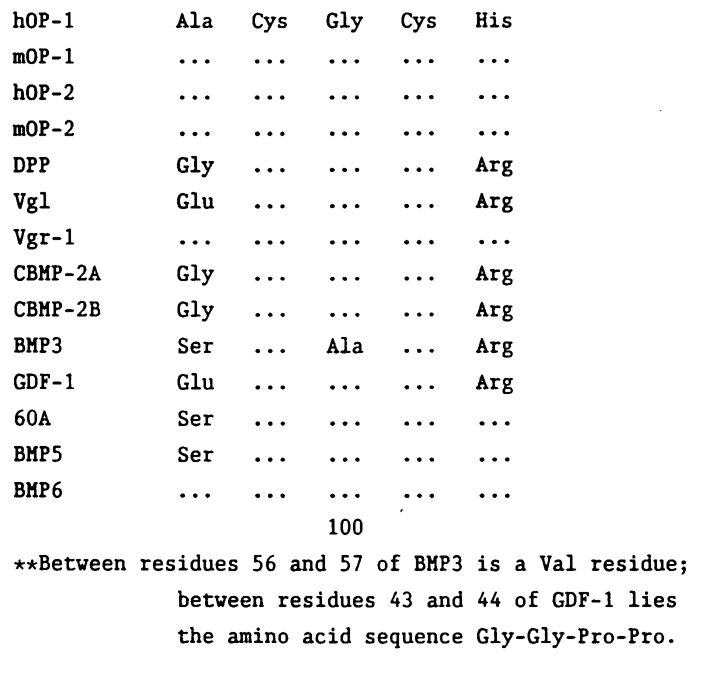

As is apparent from the foregoing amino acid sequence comparisons, significant amino acid changes can be made within the generic sequences while retaining the morphogenic activity. For example, while the GDF-1 protein sequence depicted in Table II shares only about 50% amino acid identity with the hOP1 sequence described therein, the GDF-1 sequence shares greater than 70% amino acid sequence homology (or "similarity") with the hOP1 sequence, where "homology" or "similarity" includes allowed conservative amino acid changes within the sequence as defined by Dayoff, et al., Atlas of Protein Sequence and Structure vol.5, supp.3, pp.345-362, (M.O. Dayoff, ed., Nat'l BioMed. Res. Fd'n, Washington D.C. 1979.)

The currently most preferred protein sequences useful as morphogens in this invention include those having greater than 60% identity, preferably greater than 65% identity, with the amino acid sequence defining the conserved six cysteine skeleton of hOP1 (e.g., residues 43-139 of Seq. ID No. 5). These most preferred sequences include both allelic and species variants of the OP-1 and OP-2 proteins, including the Drosophila 60A protein. Accordingly, in still another preferred aspect, the invention includes morphogens comprising species of polypeptide chains having the generic amino acid sequence referred to herein as "OPX", which defines the seven cysteine skeleton and accommodates the identities between the various identified mouse and human OP1 and OP2 proteins. OPX is presented in Seq. ID No. 29. As described therein, each Xaa at a given position independently is selected from the residues occurring at the corresponding position in the C-terminal sequence of mouse or human OP1 or OP2 (see Seq. ID Nos. 5-8 and/or Seq. ID Nos. 16-23).

### II. Matrix Considerations

The morphogens of this invention may be implanted surgically dispersed in a biocompatible, preferably in vivo biodegradable matrix appropriately modified to provide a structure in which the morphogen may be dispersed and which allows the influx, differentiation and proliferation of migrating progenitor cells. Alternatively, or, in addition, differentiated hepatocytes and/or hepatocytic progenitor cells, stimulated by exposure to the morphogen, may be disposed in and attached to a matrix structure and implanted surgically. In certain applications, such as where tissue morphogenesis is to be induced in the absence of endogenous tissue-specificity directing signals, the matrix preferably also provides signals capable of directing the tissue specificity of the differentiating cells, and provides a morphogenically permissive environment, being essentially free of growth inhibiting signals.

Where the matrix is to be incorporated into a surgically prepared liver, or provided to a biocompatible, associated site, the formulated matrix on which the morphogen is disposed may be shaped as desired in anticipation of surgery or may be shaped by the physician or technician during surgery. Where cells are to be attached to the matrix before implantation, the matrix preferably is shaped before cells are attached thereto. The matrix preferably is biodegradable in vivo, being slowly absorbed by the body and replaced by new tissue growth, in the shape or very nearly in the shape of the implant.

Details of how to make and how to use preferred matrices useful in this invention are disclosed below. In addition to these matrices, WO 88/03785, published June 2, 1988, and WO90/12604, published November 1, 1990, describe additional polymeric materials and matrix scaffold considerations.

### A. Tissue-derived Matrices

Suitable biocompatible, in vivo biodegradable acellular matrices may be prepared from naturally-occurring tissue. The tissue is treated with suitable agents to substantially extract the cellular, nonstructural components of the tissue. The agents also should be capable of extracting any growth inhibiting components associated with the tissue. The resulting material is a porous, acellular matrix, substantially depleted in nonstructurally-associated components, and preferably containing structural molecules such as collagen, laminin, hyaluronic acid, and the like.

The matrix also may be further treated with agents that modify the matrix, increasing the number of pores and micropits on its surfaces. Those skilled in the art will know how to determine which agents are best suited to the extraction of nonstructural components for different tissues. For example, soft tissues such as liver and lung may be thin-sectioned and exposed to a nonpolar solvent such as, for example, 100% ethanol, to destroy the cellular structure of the tissue and extract nonstructural components. The material then is dried and pulverised to yield nonadherent porous particles. Structural tissues such as cartilage and dentin where collagen is the primary component may be demineralized and extracted with guanidine, essentially following the method of Sampath et al. (1983) PNAS 80:6591-6595. For example, pulverized and demineralized dentin is extracted with five volumes of 4M guanidine-HCl, 50mM Tris-HCl, pH 7.0 for 16 hours at 4°C. The suspension then is filtered. The insoluble material that remains is collected and used to fabricate the matrix. The material is mostly collagenous in manner. It is devoid of morphogenic activity. The matrix particles may further be treated with a collagen fibril-modifying agent that extracts potentially unwanted components from the matrix, and alters the surface structure of the matrix material. Useful agents include acids, organic solvents or heated aqueous media. A detailed description of these matrix treatments are disclosed in U.S. Patent No. 4,975,526 and PCT publication US90/00912, published September 7, 1990 (WO90/10018).

The currently most preferred agent is a heated aqueous fibril-modifying medium such as water, to increase the matrix particle surface area and porosity. The currently most preferred aqueous medium is an acidic aqueous medium having a pH of less than about 4.5, e.g., within the range of about pH 2 - pH 4 which may help to "swell" the collagen before heating. 0.1% acetic acid, which has a pH of about 3, currently is most preferred. O.1 M acetic acid also may be used.

Various amounts of delipidated, demineralized guanidine-extracted collagen matrix are heated in the aqueous medium (1g matrix/30ml aqueous medium) under constant stirring in a water jacketed glass flask, and maintained at a given temperature for a predetermined period of time. Preferred treatment times are about one hour, although exposure times of between about 0.5 to two hours appear acceptable. The temperature employed is held constant at a temperature within the range of about 37°C to 65°C. The currently preferred heat treatment temperature is within the range of about 45°C to 60°C.

After the heat treatment, the matrix is filtered, washed, lyophilized and used for implant. Where an acidic aqueous medium is used, the matrix also is preferably neutralized prior to washing and lyophilization. A currently preferred neutralization buffer is a 200mM sodium phosphate buffer, pH 7.0. To neutralize the matrix, the matrix preferably first is allowed to cool following thermal treatment, the acidic aqueous medium (e.g., 0.1% acetic acid) then is removed and replaced with the neutralization buffer and the matrix agitated for about 30 minutes. The neutralization buffer then may be removed and the matrix washed and lyophilized.

Other useful fibril-modifying treatments include acid treatments (e.g., trifluoroacetic acid and hydrogen fluoride) and solvent treatments such as dichloromethane, acetonitrile, isopropanol and chloroform, as well as particular acid/solvent combinations.

After contact with the fibril-modifying agent, the treated matrix may be washed to remove any extracted components, following a form of the procedure set forth below:
1. Suspend matrix preparation in TBS (Tris-buffered saline) 1g/200 ml and stir at 4°C for 2 hrs; or in 6 M urea, 50 mM Tris-HCl, 500 mM NaCl, pH 7.0 (UTBS) or water and stir at room temperature (RT) for 30 minutes (sufficient time to neutralize the pH);
2. Centrifuge and repeat wash step; and
3. Centrifuge; discard supernatant; water wash residue; and then lyophilize.

### B. Synthetic Matrices

Suitable matrix scaffolds may be created from biocompatible, preferably in vivo biodegradable synthetic polymers, including polylactic acid, polyglycolic acid, polyanhydride, polybutyric acid, and copolymers thereof, and/or synthetic-inorganic materials, such as hydroxyapatite, tricalcium phosphate, and other calcium phospates. These polymers are well described in the art and are available commercially. For example, polymers composed of polyactic acid (e.g., MW 100 kDa), 80% polylactide/20% glycoside or poly 3-hydroxybutyric acid (e.g., MW 30 kDa) all may be purchased from PolySciences, Inc. The polymer compositions generally are obtained in particulate form and the osteogenic devices preferably fabricated under nonaqueous conditions (e.g., in an ethanol-trifluoroacetic acid solution, EtOH/TFA) to avoid hydrolysis of the polymers. In addition, one can alter the morphology of the particulate polymer compositions, for example to increase porosity, using any of a number of particular solvent treatments known in the art.

For example, osteogenic devices fabricated with morphogenic protein, solubilized in EtOH/TFA as described below, and a matrix composed of polylactic acid, poly 3-hydroxybutyric acid, or 80% polylactide/20% glycoside are all osteogenically active when implanted in the rat model and bioassayed as described in U.S. Pat. No. 4,968,590 (e.g., as determined by calcium content, alkaline phosphatase levels and histology of 12-day implants).

### C. Synthetic Tissue-Specific Matrices

In addition to the naturally-derived tissue-specific matrices described above, useful tissue-specific matrices may be formulated synthetically if appropriately modified. These porous biocompatible, in vivo biodegradable synthetic matrices are disclosed in PCT publication US91/03603, published December 12, 1991 (WO91/18558). Briefly the matrix comprises a porous crosslinked structural matrix comprises a porous crosslinked structural polymer of biocompatible, biodegradable collagen and appropriate, tissue-specific glycosaminoglycans as tissue-specific cell attachment factors. Collagen derived from a number of sources may be suitable for use in these synthetic matrices, including insoluble collagen, acid-soluble collagen, collagen soluble in neutral or basic aqueous solutions, as well as those collagens which are commercially available.

Glycosaminoglycans (GAGs) or mucopolysaccharides are hexosamine-containing polysaccharides of animal origin that have a tissue specific distribution, and therefore may be used to help determine the tissue specificity of the morphogen-stimulated differentiating cells. Reaction with the GAGs also provides collagen with another valuable property, i.e., inability to provoke an immune reaction (foreign body reaction) from an animal host.

Chemically, GAGs are made up of residues of hexoseamines glycosidically bound and alternating in a more-or-less regular manner with either hexouronic acid or hexose moieties (see, e.g., Dodgson et al. in Carbohydrate Metabolism and its Disorders (Dickens et al., eds.) Vol. 1, Academic Press (1968)). Useful GAGs include hyaluronic acid, heparin, heparin sulfate, chondroitin 6-sulfate, chondroitin 4-sulfate, dermatan sulfate, and keratin sulfate. Other GAGs are suitable for forming the matrix described herein, and those skilled in the art will either know or be able to ascertain other suitable GAGS using no more than routine experimentation. For a more detailed description of mucopolysaccharides, see Aspinall, Polysaccharides, Pergamon Press, Oxford (1970). For example chondroitin-6-sulfate can be used here endochondral bone formation is desired. Heparin sulfate, on the other hand, may be used to formulate synthetic matrices for use in lung tissue repair.

Collagen can be reacted with a GAG in aqueous acidic solutions, preferably in diluted acetic acid solutions. By adding the GAG dropwise into the aqueous collagen dispersion, coprecipitates of tangled collagen fibrils coated with GAG results. This tangled mass of fibers then can be homogenized to form a homogeneous dispersion of fine fibers and then filtered and dried.

Insolubility of the collagen-GAG products can be raised to the desired degree by covalently cross-linking these materials, which also serves to raise the resistance to resorption of these materials. In general, any covalent cross-linking method suitable for cross-linking collagen also is suitable for crosslinking these composite materials, although crosslinking by a dehydrothermal process is preferred.

When dry, the crosslinked particles are essentially spherical, with diameters of about 500 µm. Scanning electron miscroscopy shows pores of about 20 µm on the surface and 40 µm on the interior. The interior is made up of both fibrous and sheet-like structures, providing surfaces for cell attachment. The voids interconnect, providing access to the cells throughout the interior of the particle. The material appears to be roughly 99.5% void volume, making the material very efficient in terms of the potential cell mass that can be grown per gram of microcarrier.

### D. Morphogen Adsorption to Matrix Surfaces

The morphogens described herein can be combined and dispersed in a suitable matrix using any of the methods described below:

### 1. Ethanol Precipitation

Matrix is added to the morphogen dissolved in guanidine-HCl. Samples are vortexed and incubated at a low temperature. Samples are then further vortexed. Cold absolute ethanol is added to the mixture which is then stirred and incubated. After centrifugation (microfuge, high speed) the supernatant is discarded. The matrix is washed with cold concentrated ethanol in water and then lyophilized.

### 2. Acetonitrile Trifluoroacetic Acid Lyophilization

In this procedure, morphogen in an acetonitrile trifluroacetic acid (ACN/TFA solution is added to the carrier material. Samples are vigorously vortexed many times and then lyophilized.

### 3. Buffered Saline Lyophilization

Morphogen preparations in physiological saline may also be vortexed with the matrix and lyophilized to produce morphogenically active material.

### III. Hepatocytic Cell Considerations

Primary hepatocytes or progenitor cells may be implanted in the mammal in one embodiment of the invention. For example, implanted hepatocytes may act as gene therapy tools capable of correcting a protein deficiency in vivo by expressing and/or secreting the deficient protein when implanted at a liver tissue or associated locus in a mammal. The liver functions in part as a protein-synthesizing organ, responsible for the production of myriad proteins which are secreted from the liver and transported, e.g., via the circulatory system, to function elsewhere in the body. Accordingly, hepatic tissue, like renal and pancreatic tissue, provides an endogenous system having the necessary mechanisms in place to act as a vector for the in vivo production of (including secretion of) any protein, including proteins not normally expressed by hepatic tissue. Thus, protein deficiencies that can be treated by this method include proteins involved in normal liver functions, proteins normally produced and secreted by the liver to function elsewhere in the body, and proteins not normally produced by hepatic tissue. Where the proteins to be produced are not normally expressed by hepatic tissue, the hepatocytes must be provided with means for expressing that protein. For example, the cell may be genetically engineered as described below to induce expression of the endogenous genetic sequence encoding the protein. Alternatively, a nucleic acid encoding the protein and under control of a suitable promoter (and enhancer), may be provided to the cell as described below. In addition, the cell may be provided with one or more regulatory elements so that expression of the protein of interest mimics that of the endogenously produced protein, particularly where normal protein expression depends on changes in the physiological concentration of a molecule. For example, insulin production is regulated by blood glucose levels in the body.

The protein deficiency to be corrected may result from defective endogenous protein production, including protein expression and/or secretion, or the protein's efficacy may be reduced due to a preexisting condition in the individual. The defect may be genetic or may be induced by, for example, damage to the protein-synthesizing tissue. Exemplary hepatic proteins that may be used in a gene therapy include, but are not limited to, albumin and albumin synthesis proteins, blood clotting factors, including fibrinogen and thrombin, Factor VIII, iron or copper binding proteins, and vitamin A binding proteins. Exemplary non-hepatic proteins that may be used in a gene therapy include, but are not limited to, insulin, tissue plasminogen activator (TPA), erythropoietin, growth hormones, and the like. Similarly, the cells also may act as in vivo drug delivery vehicles, capable of producing and secreting one or more therapeutic drugs when implanted at a suitable locus in a mammal. The cells further may be manipulated to modify antigen expression on the cell surface, and limit the in vivo immune response typically induced by foreign material.

Where cells act as gene therapy tools, the cells may be obtained from a donor competent for providing the protein of interest. Cells can be obtained by biopsy or surgical excision from a donor, or from established cell lines. Preferably, allogenic cells are obtained from a biocompatible donor. Alternatively, autologous cells may be obtained from the patient and modified by recombinant DNA technology to incorporate genetic sequences sufficient to allow the cells to produce the protein or proteins of interest in vivo when the cells are reimplanted in the patient. Protocols and detailed discussions of considerations for introducing foreign genetic material into cells, particularly human cells, are well described in the art. A representative, but by no means exhaustive list, includes US Pat.No. 4,868,116, issued September 19, 1989, US Pat. No. 4,980,286, issued December 25, 1990, both to Morgan et al., and US Pat. No. 4,396,601, issued August 2, 1983, to Salser et al., Anderson, WF (1992) Science 256:808-813, Karson et al., (1992) J. Reprod Med 37:508-514, and Hoeg et al., (1990) Trans Assoc. Am Physicians 103:73-79,

A currently preferred protocol for isolating primary hepatocytes from liver tissue is described in Example 3 below. Other methods known in the art also are envisioned to be useful, such as those described, for example, in WO 88/03785. Where pluripotential hemopoietic stem cells are to be used, a useful method for their isolation is described in international application US92/01968 (WO92/15323). Briefly, and as described in detail therein, a biocompatible matrix material able to allow the influx of migratory progenitor cells may be implanted at an in vivo site long enough to allow the influx of migratory progenitor cells. For example, a bone-derived, guanidine-extracted matrix, formulated as disclosed for example in Sampath et al. ((1983) PNAS 80:6591-6595), or U.S. Patent No. 4,975,526, may be implanted into a rat, essentially following the method of Sampath et al. (ibid). After three days the implant is removed, and the progenitor cells associated with the matrix dispersed and cultured. Another method is described, for example, in US Pat. No. 5,061,620, issued 10/29/91, to Tsukamoto et al.

Isolated cells may be stimulated in vitro by morphogen exposure, essentially as described in Example 3. Stimulation is performed under sterile conditions, using an appropriate morphogen concentration and incubation period to stimulate the cells. Preferred times and concentration for a given procedure may be determined empirically by the clinician without undue experimentation. In general, a period of from about 10 minutes to 72 hours should be sufficient. Cells may be attached to a matrix by incubating the cells in the presence of matrix for at least a number of hours, e.g., 3-5 hours, or, preferably overnight. An efficient technique for attaching cells to a matrix surface is to place a concentrated suspension of cells on the surface of the matrix material and allow the cells to infiltrate and adsorb to the material. Cells typically attach individually or in small groups. In the absence of added morphogen cells begin rearranging into clusters within 24 hours and within 3 days cells have almost completely infiltrated the support and have organized into large clusters.

In a particularly preferred embodiment, the morphogen first is adsorbed to the matrix surface and cells subsequently attached thereto. The cell-matrix structure may be maintained in vitro and to allow the cells to proliferate (preferably by exposure to a morphogen or morphogen-stimulting agent) or, alternatively, the complex may be implanted in the animal and the cells allowed to proliferate (and differentiate) in vivo.

As with morphogen administrations, where implanted cells are to replace damaged or lost tissue at a liver-specific locus, the cells preferably are provided to a surgically prepared locus where from which necrotic or cirrhotic tissue has been removed, e.g., by surgical, chemical, ablating, or other means known in the medical art. The cells then are provided to the prepared site, preferably attached to a matrix and associated with a morphogen or morphogen-stimulating agent.

The cells may be provided to a morphogenically permissive site in a liver-specific locus, e.g., following removal of necrotic and/or cirrhotic tissue, or following excision of sufficient tissue to provide a morphogenically permissive site. Alternatively, the cell-matrix structure may be implanted together with a morphogen or morphogen-stimulating agent at a suitable, vascularized liver-associated locus, such as within the folds of the mesentery.

As described above, implanting cells together with a morphogen or morphogen-stimulating agent enhances their proliferation and their viability in vivo, such that the new tissue is formed without the significant associated cell loss or delay which characterizes existing protocols and which currently require the use of substantial initial seed cell populations. In addition, hepatic tissue growth can be stimulated using the methods described herein without the need of a partial hepatectomy as described in the art. Finally, the morphogens described herein functionally inhibit the tissue damage associated with the body's immune response, reducing the need for associated treatments with immunosuppressive drugs.

### IV. Bioassy Considerations

The following sets forth various procedures for evaluating the in vivo morphogenic utility of the morphogens and morphogenic compositions of this invention. The proteins and compositions may be injected or surgically implanted in a mammal, following any of a number of procedures well known in the art.

### Histological Evaluation

Histological sectioning and staining is preferred to determine the extent of morphogenesis in vivo, particularly in tissue repair procedures. Excised implants are fixed in Bouins Solution, embedded in paraffin, and cut into 6-8 µm sections. Staining with toluidine blue or hemotoxylin/eosin demonstrates clearly the ultimate development of the new tissue. Twelve day implants are usually sufficient to determine whether the implants contain newly induced tissue.

Successful implants exhibit a controlled progression through the stages of induced tissue development allowing one to identify and follow the tissue-specific events that occur. For example, in endochondral bone formation the stages include:
(1) leukocytes on day one; (2) mesenchymal cell migration and proliferation on days two and three;
(3) chondrocyte appearance on days five and six;
(4) cartilage matrix formation on day seven;
(5) cartilage calcification on day eight; (6) vascular invasion, appearance of osteoblasts, and formation of new bone on days nine and ten; (7) appearance of osteoclasts and bone remodeling and dissolution of the implanted matrix on days twelve to eighteen; and
(8) hematopoietic bone marrow differentiation in the ossicle on day twenty-one. Similarly, in hepatic tissue formation the stages include leukocytes on day one, mesenchymal cell migration and proliferation on days two and three, hepatocyte appearance on days five and six, followed by matrix formation and vascularization.

### Biological Markers

In addition to histological evaluation, biological markers may be used as a marker for tissue morphogenesis. Useful markers include tissue-specific enzymes whose activities may be assayed (e.g., spectrophotometrically) after homogenization of the implant. These assays may be useful for quantitation and for obtaining an estimate of tissue formation quickly after the implants are removed from the animal. For example, alkaline phosphatase activity may be used as a marker for osteogenesis.

Incorporation of systemically provided morphogens may be followed using tagged morphogens (e.g., radioactively labelled) and determining their localization in new tissue, and/or by monitoring their disappearance from the circulatory system using a standard pulse-chase labeling protocol. The morphogen also may be provided with a tissue-specific molecular tag, whose uptake may be monitored and correlated with the concentration of morphogen provided.

### V. Formulations and Methods for Parenteral Administration of Therapeutic Agents

The morphogens of this invention may be used to repair diseased or damaged mammalian tissue. The tissue to be repaired is preferably assessed, and excess necrotic or interfering scar tissue removed as needed, by surgical, chemical, ablating or other methods known in the medical arts.

The morphogen then may be provided directly to the tissue locus as part of a sterile, biocompatible composition, either by surgical implantation or injection. Alternatively, a sterile, biocompatible composition containing morphogen-stimulated progenitor cells may be provided to the tissue locus. The existing tissue at the locus, whether diseased or damaged, provides the appropriate matrix to allow the proliferation and tissue-specific differentiation of progenitor cells. In addition, a damaged or diseased tissue locus, particularly one that has been further assaulted by surgical means, provides a morphogenically permissive environment. For some tissues, it is envisioned that systemic provision of the morphogen will be sufficient.

In some circumstances, particularly where tissue damage is extensive, the tissue may not be capable of providing a sufficient matrix for cell influx and proliferation. In these instances, it may be necessary to provide the morphogen or morphogen-stimulated progenitor cells to the tissue locus in association with a suitable, biocompatible formulated matrix, prepared by any of the means described below. The matrix preferably is tissue-specific, in vivo biodegradable, and comprises particles having dimensions within the range of 70-850µm, most preferably 150-420µm.

The morphogens may be provided to an individual by any suitable means. Preferably, the morphogen or morphogen-stimulating agent (collectively described herein below as the "therapeutic agent") is provided directly to the liver tissue (e.g., locally, as by injection to the tissue locus or by periodic release from a locally implanted osmotic pump). While not currently preferred for most liver tissue regenerative applications, oral administration or systemic injection also may be viable administration routes for certain applications, such as part of a protocol to enhance viabilty of a tissue to be transplanted, or as part of a protocol to maintain liver function during a surgical or other therapeutic procedure, or for maintaining liver function in aged or immuno-suppressed individuals, or others at risk for hepatic tissue damage. It should be noted that morphogenically active protein is present in milk, including mammary gland extract, colostrum and 57-day milk, and also is present in human serum, indicating that systemic and, in particular, oral administration are viable administrative routes for morphogens.

Where the morphogen or morphogen-stimulatig agent is provided by local injection, the morphogen preferably comprises part of an aqueous solution. The solution is physiologically acceptable so that in addition to delivery of the desired morphogen to the patient, the solution does not otherwise adversely affect the patient's electrolyte and volume balance. The aqueous medium for the morphogen thus may comprise normal physiologic saline (0.85-0.9% NaCl, 0.15M), pH 7-7.4. The aqueous solution containing the morphogen can be made, for example, by dissolving the protein in 50% ethanol containing acetonitrile in 0.1% trifluoroacetic acid (TFA) or 0.1% HCl, or equivalent solvents. One volume of the resultant solution then is added, for example, to ten volumes of phosphate buffered saline (PBS), which further may include 0.1-0.2% human serum albumin (HSA). The resultant solution preferably is vortexed extensively. If desired, a given morphogen may be made more soluble by association with a suitable molecule. For example, the pro form of the morphogenic protein comprises a species that is soluble in physiologically buffered solutions. In fact, the endogenous protein is thought to be transported in this form. This soluble form of the protein may be obtained from the culture medium of morphogen-secreting mammalian cells. Alternatively, a soluble species may be formulated by complexing the mature dimer (or an active fragment thereof) with part or all of a pro domain. Another molecule capable of enhancing solubility and particularly useful for oral administrations, is casein. For example, addition of 0.2% casein increases solubility of the mature active form of OP-1 by 80%. Other components found in milk and/or various serum proteins also may be useful.

Useful solutions for parenteral administration may be prepared by any of the methods well known in the pharmaceutical art, described, for example, in Remington's Pharmaceutical Sciences (Gennaro, A., ed.), Mack Pub., 1990. Formulations may include, for example, polyalkylene glycols such as polyethylene glycol, oils of vegetable origin, hydrogenated naphthalenes, and the like. Formulations for direct administration, in particular, may include glycerol and other compositions of high viscosity. Biocompatible, preferably bioresorbable, polymers, including, for example, hyaluronic acid, collagen, polybutyrate, tricalcium phosphate, lactide and lactide/glycolide copolymers, may be useful excipients to control the release of the morphogen in vivo. Other potentially useful parenteral delivery systems for these morphogens include ethylene-vinyl acetate copolymer particles, osmotic pumps, implantable infusion systems, and liposomes.

In addition, while the mature forms of certain morphogens described herein typically are sparingly soluble, the morphogen form found in milk (and mammary gland extract and colostrum) is readily soluble, probably by noncovalent association of the mature, morphogenically active form with part or all of the pro domain of the intact sequence as described below, (see Section V.1) and/or by association with one or more milk components. Accordingly, the compounds provided herein also may be associated with molecules capable of enhancing their solubility in vitro or in vivo.

The compounds provided herein also may be associated with molecules capable of targeting the morphogen or morphogen-stimulating agent to liver tissue. For example, an antibody, antibody fragment, or other binding protein that interacts specifically with a surface molecule on liver tissue cells, including hepatocytes or epithelial cells, may be used. Useful targeting molecules may be designed, for example, using the single chain binding site technology disclosed, for example, in U.S. Pat. No. 5,091,513.

As described above, the morphogens provided herein share significant sequence homology in the C-terminal active domains. By contrast, the sequences typically diverge significantly in the sequences which define the pro domain. Accordingly, the pro domain is thought to be morphogen-specific. As described above, it is also known that the various morphogens identified to date are differentially expressed in the different tissues. Accordingly, without being limited to any given theory, it is likely that, under natural conditions in the body, selected morphogens typically act on a given tissue. Accordingly, part or all of the pro domains which have been identified associated with the active form of the morphogen in solution, may serve as targeting molecules for the morphogens described herein. For example, the pro domains may interact specifically with one or more molecules at the target tissue to direct the morphogen associated with the pro domain to that tissue. Accordingly, another useful targeting molecule for targeting morphogen to hepatic tissue may include part or all of a morphogen pro domain. As described above, morphogen species comprising the pro domain may be obtained from culture medium of morphogen-secreting cells. Alternatively, a tissue-targeting species may be formulated by complexing the mature dimer (or an active fragment thereof) with part or all of a pro domain.

Finally, the morphogens or morphogen-stimulating agents provided herein may be administered alone or in combination with other molecules ("cofactors") known to be beneficial in maintaining liver function, particularly symptom-alleviating cofactors, such as other, non-steroidal anti-inflammatory agents, antiseptics and antibiotics.

The compounds provided herein can be formulated into pharmaceutical compositions by admixture with pharmaceutically acceptable nontoxic excipients and carriers. As noted above, such compositions may be prepared for direct, or local or systemic administration, particularly in the form of liquid solutions or suspensions; for oral administration, particularly in the form of tablets or capsules; or intranasally, particularly in the form of powders, nasal drops, or aerosols.

The compositions can be formulated for administration to humans or other mammals in therapeutically effective amounts, e.g., amounts which provide appropriate concentrations for a time sufficient to substantially eliminate or reduce the patient's pathological condition, including stimulating regeneration of damaged or lost hepatic tissue following hepatocellular injury including inhibiting additional damage thereto, to provide therapy for the liver diseases and disorders described above, and amounts effective to protect hepatic tissue in anticipation of injury to the tissue.

As will be appreciated by those skilled in the art, the concentration of the compounds described in a therapeutic composition will vary depending upon a number of factors, including the dosage of the drug to be administered, the chemical characteristics (e.g., hydrophobicity) of the compounds employed, and the route of administration. The preferred dosage of therapeutic agent to be administered also is likely to depend on such variables as the type and extent of progression of the hepatic disorder, the overall health status of the particular patient, the relative biological efficacy of the compound selected, the formulation of the compound excipients, and its route of administration. In general terms, the compounds of this invention may be provided in an aqueous physiological buffer solution containing about 0.001 to 10% w/v compound for liquid administration. Typical dose ranges are from about 10 ng/kg to about 1 g/kg of body weight per day; a preferred dose range is from about 0.1 µg/kg to 100 mg/kg of body weight per day. Optimally, the morphogen dosage given is between 0.1-100 µg of protein per kilogram weight of the patient. No obvious morphogen induced pathological lesions are induced when mature morphogen (e.g., OP-1, 20 µg) is administered daily to normal growing rats for 21 consecutive days. Moreover, 10 µg systemic injections of morphogen (e.g., OP-1) injected daily for 10 days into normal newborn mice does not produce any gross abnormalties.

Where morphogens are administered systemically, in the methods of the present invention, preferably a large volume loading dose is used at the start of the treatment. The treatment then is continued with a maintenance dose. Further administration then can be determined by monitoring at intervals the levels of the morphogen in the blood.

Where injury to hepatic tissue is induced deliberately as part of, for example, a surgical or other medical procedure, the morphogen preferably is provided just prior to, or concomitant with induction of the trauma. Preferably, the morphogen is administered prophylactically in a surgical setting. Optimally, the morphogen dosage given in all cases is between 1-100 µg of protein per kilogram weight of the patient.

As described above, as an alternative or, in addition, an effective amount of an agent capable of stimulating endogenous morphogen levels may be administered by any of the routes described above. For example, an agent capable of stimulating morphogen production and/or secretion from liver tissue cells or cells at a distant which then is targeted to the liver, may be provided to a mammal, e.g., by direct administration of the morphogen to glial cells associated with the nerve tissue to be treated. A method for identifying and testing agents capable of modulating the levels of endogenous morphogens in a given tissue is described generally herein in Example 9, and in detail in international application US92/07359 (WO 93/05/72). Briefly, candidate compounds can be identified and tested by incubating the compound in vitro with a test tissue or cells thereof, for a time sufficient to allow the compound to affect the production, i.e., the expression and/or secretion, of a morphogen produced by the cells of that tissue. Here, suitable tissue or cultured cells of a tissue preferably would comprise hepatic tissue cells.

A currently preferred detection means for evaluating the level of the morphogen in culture upon exposure to the candidate compound comprises an immunoassay utilizing an antibody or other suitable binding protein capable of reacting specifically with a morphogen and being detected as part of a complex with the morphogen. Immunoassays may be performed using standard techniques known in the art and antibodies raised against a morphogen and specific for that morphogen. Agents capable of stimulating endogenous morphogens then may formulated into pharmaceutical preparations and administered as described herein.

### V.A Soluble Morphogen Complexes

A currently preferred form of the morphogen useful in therapeutic formulations, having improved solubility in aqueous solutions and consisting essentially of amino acids, is a dimeric morphogenic protein comprising at least the 100 amino acid peptide sequence having the pattern of seven or more cysteine residues characteristic of the morphogen family complexed with a peptide comprising part or all of a pro region of a member of the morphogen family, or an allelic, species or other sequence variant thereof. Preferably, the dimeric morphogenic protein is complexed with two peptides. Also, the dimeric morphogenic protein preferably is noncovalently complexed with the pro region peptide or peptides. The pro region peptides also preferably comprise at least the N-terminal eighteen amino acids that define a given morphogen pro region. In a most preferred embodiment, peptides defining substantially the full length pro region are used.

Other soluble forms of morphogens include dimers of the uncleaved pro forms of these proteins, as well as "hemi-dimers" wherein one subunit of the dimer is an uncleaved pro form of the protein, and the other subunit comprises the mature form of the protein, including truncated forms thereof, preferably noncovalently associated with a cleaved pro domain peptide.

As described above, useful pro domains include the full length pro regions, as well as various truncated forms hereof, particularly truncated forms cleaved at proteolytic Arg-Xaa-Xaa-Arg cleavage sites. For example, in OP-1, possible pro sequences include sequences defined by residues 30-292 (full length form); 48-292; and 158-292. Soluble OP-1 complex stability is enhanced when the pro region comprises the full length form rather than a truncated form, such as the 48-292 truncated form, in that residues 30-47 show sequence homology to the N-terminal portions of other morphogens, and are believed to have particular utility in enhancing complex stability for all morphogens. Accordingly, currently preferred pro sequences are those encoding the full length form of the pro region for a given morphogen. Other pro sequences contemplated to have utility include biosynthetic pro sequences, particularly those that incorporate a sequence derived from the N-terminal portion of one or more morphogen pro sequences.

As will be appreciated by those having ordinary skill in the art, useful sequences encoding the pro region may be obtained from genetic sequences encoding known morphogens. Alternatively, chimeric pro regions can be constructed from the sequences of one or more known morphogens. Still another option is to create a synthetic sequence variant of one or more known pro region sequences.

In another preferred aspect, useful pro region peptides include polypeptide chains comprising an amino acid sequence encoded by a nucleic acid that hybridizes under stringent conditions with a DNA or RNA sequence encoding at least the N-terminal eighteen amino acids of the pro region sequence for OP1 or OP2, e.g., nucleotides 136-192 and 152-211 of Seq. ID No. 16 and 20, respectively.

### V.A.1 Isolation of Soluble morphogen complex from conditioned media or body fluid

Morphogens are expressed from mammalian cells as soluble complexes. Typically, however the complex is disassociated during purification, generally by exposure to denaturants often added to the purification solutions, such as detergents, alcohols, organic solvents, chaotropic agents and compounds added to reduce the pH of the solution. Provided below is a currently preferred protocol for purifying the soluble proteins from conditioned media (or, optionally, a body fluid such as serum, cerebro-spinal or peritoneal fluid), under non-denaturing conditions. The method is rapid, reproducible and yields isolated soluble morphogen complexes in substantially pure form.

Soluble morphogen complexes can be isolated from conditioned media using a simple, three step chromatographic protocol performed in the absence of denaturants. The protocol involves running the media (or body fluid) over an affinity column, followed by ion exchange and gel filtration chromatographies. The affinity column described below is a Zn-IMAC column. The present protocol has general applicability to the purification of a variety of morphogens, all of which are anticipated to be isolatable using only minor modifications of the protocol described below. An alternative protocol also envisioned to have utility an immunoaffinity column, created using standard procedures and, for example, using antibody specific for a given morphogen pro domain (complexed, for example, to a protein A-conjugated Sepharose column.) Protocols for developing immunoaffinity columns are well described in the art, (see, for example, Guide to Protein Purification, M. Deutscher, ed., Academic Press, San Diego, 1990, particularly sections VII and XI.)

In this experiment OP-1 was expressed in mammalian CHO (chinese hamster ovary) cells as described in the art (see, for example, international application US90/05903 (WO91/05802).) The CHO cell conditioned media containing 0.5% FBS was initially purified using Immobilized Metal-Ion Affinity Chromatography (IMAC). The soluble OP-1 complex from conditioned media binds very selectively to the Zn-IMAC resin and a high concentration of imidazole (50 mM imidazole, pH 8.0) is required for the effective elution of the bound complex. The Zn-IMAC step separates the soluble OP-1 from the bulk of the contaminating serum proteins that elute in the flow through and 35 mM imidazole wash fractions. The Zn-IMAC purified soluble OP-1 is next applied to an S-Sepharose cation-exchange column equilibrated in 20 mM NaPO₄ (pH 7.0) with 50 mM NaCl. This S-Sepharose step serves to further purify and concentrate the soluble OP-1 complex in preparation for the following gel filtration step. The protein was applied to a Sephacryl S-200HR column equilibrated in TBS. Using substantially the same protocol, soluble morphogens also may be isolated from one or more body fluids, including serum, cerebro-spinal fluid or peritoneal fluid.

IMAC was performed using Chelating-Sepharose (Pharmacia) that had been charged with three column volumes of 0.2 M ZnSO₄. The conditioned media was titrated to pH 7.0 and applied directly to the ZN-IMAC resin equilibrated in 20 mM HEPES (pH 7.0) with 500 mM NaCl. The Zn-IMAC resin was loaded with 80 mL of starting conditioned media per mL of resin. After loading, the column was washed with equilibration buffer and most of the contaminating proteins were eluted with 35 mM imidazole (pH 7.0) in equilibration buffer. The soluble OP-1 complex then is eluted with 50 mM imidazole (pH 8.0) in 20 mM HEPES and 500 mM NaCl.

The 50 mM imidazole eluate containing the soluble OP-1 complex was diluted with nine volumes of 20 mM NaPO₄ (pH 7.0) and applied to an S-Sepharose (Pharmacia) column equilibrated in 20 mM NaPO₄ (pH 7.0) with 50 mM NaCl. The S-Sepharose resin was loaded with an equivalent of 800 mL of starting conditioned media per mL of resin. After loading the S-Sepharose column was washed with equilibration buffer and eluted with 100 mM NaCl followed by 300 mM and 500 mM NaCl in 20 mM NaPO₄ (pH 7.0). The 300 mM NaCl pool was further purified using gel filtration chromatography. Fifty mls of the 300 mm NaCl eluate was applied to a 5.0 X 90 cm Sephacryl S-200HR (Pharmacia) equilibrated in Tris buffered saline (TBS), 50 mM Tris, 150 mM NaCl (pH 7.4). The column was eluted at a flow rate of 5 mL/minute collecting 10 mL fractions. The apparent molecular of the soluble OP-1 was determined by comparison to protein molecular weight standards (alcohol dehydrogenase (ADH, 150 kDa), bovine serum albumin (BSA, 68 kDa), carbonic anhydrase (CA, 30 kDa) and cytochrome C (cyt C, 12.5 kDa). The purity of the S-200 column fractions was determined by separation on standard 15% polyacrylamide SDS gels stained with coomassie blue. The identity of the mature OP-1 and the pro-domain was determined by N-terminal sequence analysis after separation of the mature OP-1 from the pro-domain using standard reverse phase C18 HPLC.

The soluble OP-1 complex elutes with an apparent molecular weight of 110 kDa. This agrees well with the predicted composition of the soluble OP-1 complex with one mature OP-1 dimer (35-36 kDa) associated with two pro-domains (39 kDa each). Purity of the final complex can be verified by running the appropriate fraction in a reduced 15% polyacrylamide gel.

The complex components can be verified by running the complex-containing fraction from the S-200 or S-200HR columns over a reverse phase C18 HPLC column and eluting in an acetonitrile gradient (in 0.1% TFA), using standard procedures. The complex is dissociated by this step, and the pro domain and mature species elute as separate species. These separate species then can be subjected to N-terminal sequencing using standard procedures (see, for example, Guide to Protein Purification, M. Deutscher, ed., Academic Press, San Diego, 1990, particularly pp. 602-613), and the identity of the isolated 36kD, 39kDa proteins confirmed as mature morphogen and isolated, cleaved pro domain, respectively. N-terminal sequencing of the isolated pro domain from mammalian cell produced OP-1 revealed 2 forms of the pro region, the intact form (beginning at residue 30 of Seq. ID No. 16) and a truncated form, (beginning at residue 48 of Seq. ID No. 16.) N-terminal sequencing of the polypeptide subunit of the isolated mature species reveals a range of N-termini for the mature sequence, beginning at residues 293, 300, 313, 315, 316, and 318, of Seq. ID No. 16, all of which are active as demonstrated by the standard bone induction assay.

### V.A.2. In Vitro Soluble Morphogen Complex Formation

As an alternative to purifying soluble complexes from culture media or a body fluid, soluble complexes may be formulated from purified pro domains and mature dimeric species. Successful complex formation apparently requires association of the components under denaturing conditions sufficient to relax the folded structure of these molecules, without affecting disulfide bonds. Preferably, the denaturing conditions mimic the environment of an intracellular vesicle sufficiently such that the cleaved pro domain has an opportunity to associate with the mature dimeric species under relaxed folding conditions. The concentration of denaturant in the solution then is decreased in a controlled, preferably step-wise manner, so as to allow proper refolding of the dimer and pro regions while maintaining the association of the pro domain with the dimer. Useful denaturants include 4-6M urea or guanidine hydrochloride (GuHCl), in buffered solutions of pH 4-10, preferably pH 6-8. The soluble complex then is formed by controlled dialysis or dilution into a solution having a final denaturant concentration of less than 0.1-2M urea or GuHCl, preferably 1-2 M urea of GuHCl, which then preferably can be diluted into a physiological buffer. Protein purification/renaturing procedures and considerations are well described in the art, and details for developing a suitable renaturing protocol readily can be determined by one having ordinary skill in the art. One useful text one the subject is Guide to Protein Purification, M. Deutscher, ed., Academic Press, San Diego, 1990, particularly section V. Complex formation also may be aided by addition of one or more chaperone proteins.

### V.A.3 Stability of Soluble Morphogen Complexes

The stability of the highly purified soluble morphogen complex in a physiological buffer, e.g., tris-buffered saline (TBS) and phosphate-buffered saline (PBS), can be enhanced by any of a number of means. Currently preferred is by means of a pro region that comprises at least the first 18 amino acids of the pro sequence (e.g., residues 30-47 of Seq. ID NO. 16 for OP-1), and preferably is the full length pro region. Residues 30-47 show sequence homology to the N-terminal portion of other morphogens and are believed to have particular utility in enhancing complex stability for all morphogens. Other useful means for enhancing the stability of soluble morphogen complexes include three classes of additives. These additives include basic amino acids (e.g., L-arginine, lysine and betaine); nonionic detergents (e.g., Tween 80 or NonIdet P-120); and carrier proteins (e.g., serum albumin and casein). Useful concentrations of these additives include 1-100 mM, preferably 10-70 mM, including 50 mM, basic amino acid;, 0.01-1.0%, preferably 0.05-0.2%, including 0.1% (v/v) nonionic detergent;, and 0.01-1.0%, preferably 0.05-0.2%, including 0.1% (w/v) carrier protein.

### VI. Examples

### Example 1. Identification of Morphogen-Expressing Tissue

Determining the tissue distribution of morphogens may be used to identify different morphogens expressed in a given tissue, as well as to identify new, related morphogens. Tissue distribution also may be used to identify useful morphogen-producing tissue for use in screening and identifying candidate morphogen-stimulating agents. The morphogens (or their mRNA transcripts) readily are identified in different tissues using standard methodologies and minor modifications thereof in tissues where expression may be low. For example, protein distribution may be determined using standard Western blot analysis or immunofluorescent techniques, and antibodies specific to the morphogen or morphogens of interest. Similarly, the distribution of morphogen transcripts may be determined using standard Northern hybridization protocols and transcript-specific probes.

Any probe capable of hybridizing specifically to a transcript, and distinguishing the transcript of interest from other, related transcripts may be used. Because the morphogens described herein share such high sequence homology in their active, C-terminal domains, the tissue distribution of a specific morphogen transcript may best be determined using a probe specific for the pro region of the immature protein and/or the N-terminal region of the mature protein. Another useful sequence is the 3' non-coding region flanking and immediately following the stop codon. These portions of the sequence vary substantially among the morphogens described herein, and accordingly, are specific for each protein. For example, a particularly useful Vgr-1-specific probe sequence is the PvuII-SacI fragment, a 265 bp fragment encoding both a portion of the untranslated pro region and the N-terminus of the mature sequence (see Lyons et al. (1989) PNAS 86:4554-4558 for a description of the cDNA sequence). Similarly, particularly useful mOP-1-specific probe sequences are the BstX1-BglI fragment, a 0.68 Kb sequence that covers approximately two-thirds of the mOP-1 pro region; a StuI-StuI fragment, a 0.2 Kb sequence immediately upstream of the 7-cysteine domain; and the Ear1-Pst1 fragment, an 0.3 Kb fragment containing a portion of the 3'untranslated sequence (See Seq. ID No. 18, where the pro region is defined essentially by residues 30-291.) Similar approaches may be used, for example, with hOP-1 (Seq. ID No. 16) or human or mouse OP-2 (Seq. ID Nos. 20 and 22.)

Using these morphogen-specific probes, which may be synthetically engineered or obtained from cloned sequences, morphogen transcripts can be identified in mammalian tissue, using standard methodologies well known to those having ordinary skill in the art. Briefly, total RNA is prepared from various adult murine tissues (e.g., liver, kidney, testis, heart, brain, thymus and stomach) by a standard methodology such as by the method of Chomczyaski et al. ((1987) Anal. Biochem 162:156-159) and described below. Poly (A)+ RNA is prepared by using oligo (dT)-cellulose chromatography (e.g., Type 7, from Pharmacia LKB Biotechnology, Inc.). Poly (A)+ RNA (generally 15 µg) from each tissue is fractionated on a 1% agarose/formaldehyde gel and transferred onto a Nytran membrane (Schleicher & Schuell). Following the transfer, the membrane is baked at 80°C and the RNA is cross-linked under UV light (generally 30 seconds at 1 mW/cm²). Prior to hybridization, the appropriate probe is denatured by heating. The hybridization is carried out in a lucite cylinder rotating in a roller bottle apparatus at approximately 1 rev/min for approximately 15 hours at 37°C using a hybridization mix of 40% formamide, 5 x Denbardts, 5 x SSPE, and 0.1% SDS. Following hybridization, the non-specific counts are washed off the filters in 0.1 x SSPE, 0.1% SDS at 50°C.

Examples demonstrating the tissue distribution of various morphogens, including Vgr-1, OP-1, BMP2, BMP3, BMP4, BMP5, GDF-1, and OP-2 in developing and adult tissue are disclosed international application US92/01968 (WO92/15323), and in Ozkaynak, et al., (1991) Biochem. Biophys. Res. Commn. 179:116-123, and Ozkaynak, et al. (1992) (J. Biol. Chem. 267: 25220-25227. Using the general probing methodology described herein, northern blot hybridizations using probes specific for these morphogens to probe brain, spleen, lung, heart, liver and kidney tissue indicate that kidney-related tissue appears to be the primary expression source for OP-1, with brain, heart and lung tissues being secondary sources. Lung tissue appears to be the primary tissue expression source for Vgr-1, BMP5, BMP4 and BMP3. Lower levels of Vgr-1 also are seen in kidney and heart tissue, while the liver appears to be a secondary expression source for BMP5, and the spleen appears to be a secondary expression source for BMP4. GDF-1 appears to be expressed primarily in brain tissue. To date, OP-2 appears to be expressed primarily in early embryonic tissue. Specifically, northern blots of murine embryos and 6-day post-natal animals shows abundant OP2 expression in 8-day embryos. Expression is reduced significantly in 17-day embryos and is not detected in post-natal animals.

### Example 2. Morphogen Localization in Developing Hepatic Tissue

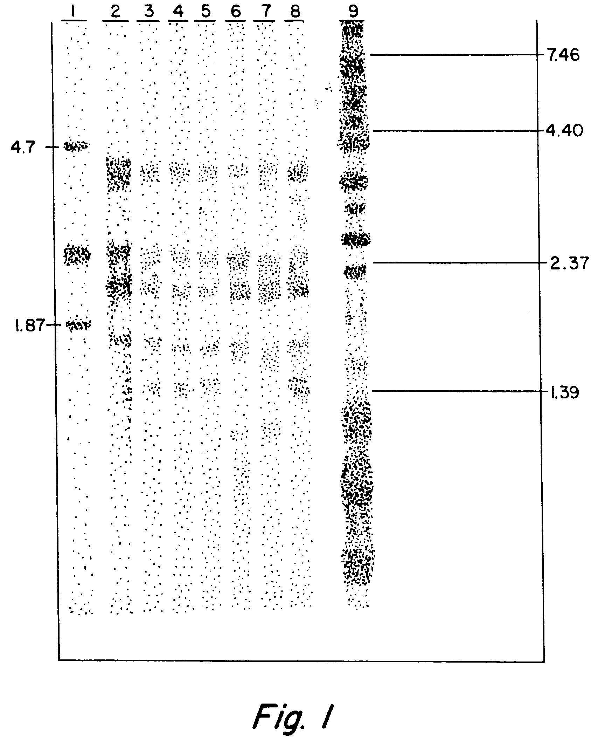

The onset of liver formation in a developing embryo occurs at day 14. Using the hybridization protocol described in Example 1, morphogen expression was identified at the onset of liver formation during embryo development. Specifically, northern blots of mRNA isolated from murine embryo liver tissue (probed at 15 days and 20 days) and post natal mouse liver tissue (probed at 7, 14, 21 and 28 days past birth) show mOP-1 expression in developing liver tissue only during the time of liver formation. Specifically, as illustrated, in Fig. 1, mOP-1 RNA is expressed significantly in the 15 day embryo, and is present at much lower amounts at later times in healthy hepatic tissue. In the figure, lanes 2 and 3 contain RNA from 15- and 20-day embryo tissue, respectively; lanes 4-8, RNA from 3, 7, 14, 21 and 28 days post natal animals, respectively; and lane 9 is a molecular weight ladder. Lanes 1 and 9 are markers. In the Northern blot mOP-1 RNA appears as a discrete band running at about 4kb and 2.2 or 2.4 kb, as well as a shorter band at 1.8kb (see, for example, Ozkaynak, et al. (1991) Biochem. Biophys Res. 179: 116-123.)

### Example 3. Mitogenic Effect of Morphogen on Rat Hepatocytes

The ability of a morphogen to induce proliferation of primary hepatocytes may be demonstrated in vitro using the following assay using primary hepatocytes isolated from rat liver. Unless otherwise indicated, all chemicals referenced are standard, commercially available reagents, readily available from a number of sources, including Sigma Chemical, Co., St. Louis; Calbiochem, Corp., San Diego, and Aldrich Chemical Co., Milwaukee.

Rat primary hepatocyte cultures were prepared by a two-step collagenase digestion essentially as described by Fausto et al. (1987) Cell Separation: Methods and Selected Applications 4:45-77. Briefly, the liver of a male rat (e.g., CD strain, Charles River Laboratories, Wilmington, MA) was perfused via the portal vein with Ca²⁺ free and Mg²⁺ free Hank's balanced salt solution for 10 min at a flow of 30-40 ml/min, followed by perfusion with 0.05% collagenase in Ca²⁺-containing medium (Hepes buffer) for 10 min. The liver capsule was removed, the cells shaken loose from the tissue and filtered hepatocytes were collected by repeated centrifugation of the cell suspension at 50 xg for 25 min. Hepatocyte suspensions were virtually free of non-parenchymal cell contamination. Cells (2x10⁵ per dish) were plated on 35-mm dishes coated with rat tail collagen in MEM (modified Eagle's Medium, Gibco, Long Island) containing 5% fetal bovine serum (FBS), 1mM pyuvate, 0.2mM aspartate, 1mM proline, 0.2mM serine, 2mM glutamine, and 0.5 µg of hydrocotisone and 1 µg of insulin per ml. The cells were incubated for 24 hours under standard at 37°C, at which time the growth medium was replaced with serum-free MEM.

The cell culture then was divided into two groups:
(1) wells which received morphogen within the dose range of 1-100 ng of morphogen per ml medium; and (2) the control group, which received no additional factors. In this example, OP-1 was the morphogen tested. The cells then were incubated for an additional 18-24 hours after which the wells were pulsed with 2µCi/well of ³H-thymidine and incubated for six more hours. The excess label then was washed off with a cold solution of 0.15 M NaCl. 250 µl of 10% tricholoracetic acid then was added to each well and the wells incubated at room temperature for 30 minutes. The cells then were washed three times with cold distilled water, and lysed by the addition of 250 µl of 1% sodium dodecyl sulfate (SDS) for a period of 30 minutes at 37°C. The cell lysates then were harvested using standard means well known in the art, and the incorporation of ³H-thymidine into cellular DNA was determined by liquid scintillation as an indication of mitogenic activity of the cells.

Morphogen treatment of primary hepatocyte cultures significantly stimulates ³ H-thymidine incorporation into DNA, and thus promotes their cell proliferation. The mitogenesis stimulated by 20 ng of OP-1 in 1 ml serum-free medium was equivalent to the mitogenic effect of 10% fresh serum alone. By contrast, other local-acting growth factors, such as TGF-β do not stimulate proliferation of primary hepatocytes (see Fausto et al. (1991) Ciba Found Symp 157:165-174.)

### Example 4. Morphogen-Induced Liver Regeneration

While hepatocytes have a remarkable capacity to undergo compensatory growth following tissue loss, the reparative properties of liver differ significantly from embryonic morphogenesis. Specifically, following a partial hepatectomy wherein a liver lobe is partially or completely removed, the remaining intact lobes grow rapidly and double in weight due to the ability of the differentiated hepatocytes in the intact lobe to undergo limited proliferation. However, the excised lobe itself is not regenerated. The following example demonstrates the ability of morphogens to regenerate lost hepatic tissue following a partial hepatectomy, including regenerating the excised tissue lobe. The protocol described below is a variation on a standard partial hepatectomy protocol, described, for example, by Higgins et al. (1931) Arch. Pathol. 12:136-202 and Braun et al. (1989) PNAS 86:1558-1562.

Morphogen, e.g., purified recombinant human OP-1, mature form, was solubilized (1 mg/ml) in 50% ethanol (or compatible solvent) containing 0.1% trifluoroacetic acid (or compatible acid). The injectable OP-1 solution was prepared by diluting one volume of OP-1/solvent-acid stock solution with 9 volumes of 0.2% rat serum albumin in sterile PBS (phosphate-buffered saline).

Growing rats or aged rats were anesthetized by using ketamine. Two of the liver lobes (left and right) were cut out (approximately 1/3 of the lobe) and the morphogen was injected locally at multiple sites along the cut ends. The amount of OP-1 injected was 100 µg in 100 of PBS/RSA (phosphate-buffered saline/rat serum albumin) injection buffer. Placebo samples were injection buffer without OP-1. Five rats in each group were used. The wound was closed using standard surgical procedures and the rats were allowed to eat normal food and drink tap water.

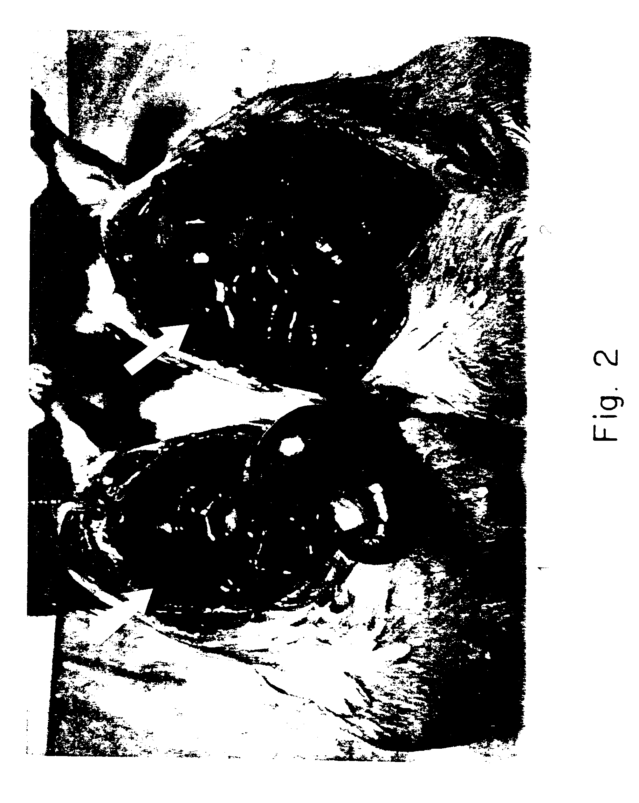

After 12 days, the rats were sacrificed and liver regeneration was observed visually. The photomigraph in Fig. 2 illustrates dramatically the regenerative effects of OP-1 on liver tissue formation. In the figure, the arrow indicates the treated lobe. The OP-1-injected group showed complete liver tissue regeneration including reformation of the excised lobe tissue, and showed no sign of any cut in the liver (animal 2). By contrast, in the control group into which only PBS was injected, the excised lobe tissue was not regenerated (animal 1). The original incision remains in this sample.

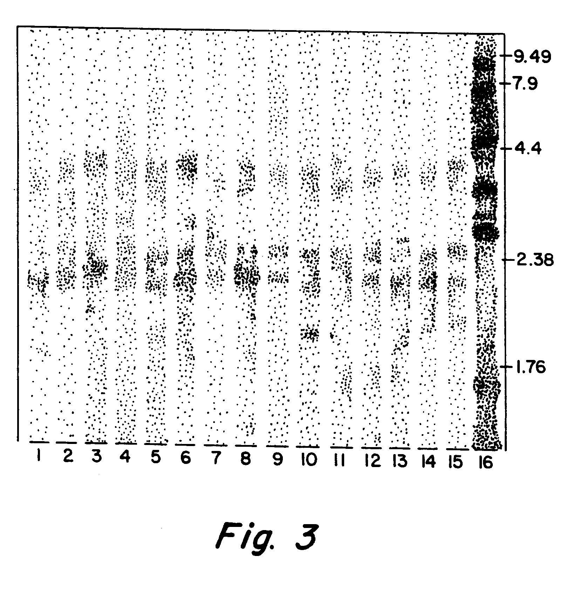

In a related experiment, animals were partially hepatectomized or sham-operated and Northern blot analysis performed on RNA isolated from the liver tissue. None of the animals were morphogen-treated. As determined by Northern blot analysis (probed with mOP-1-specific labeled oligonucleotide, see Fig.3), in the absence of morphogen treatment, the level of endogenous morphogen is not enhanced significantly following partial hepatectomy. In the figure lanes 2, 4, 6, 8, 10, 12, and 14, are samples from partially hepatectomized rats and lanes 3, 5, 7, 9, 11, 13, and 15 are samples from sham-operated rats, and lanes 1 and 16 are markers. Samples were taken at 6 hour intervals between 12 and 96 hours post surgery.

### Example 5. Morphogen Expression in Regenerating Liver Tissue Following Toxin-Induced Tissue Damage

Hepatic tissue repair following toxic agent-induced damaged tissue involves proliferation and differentiation of hepatocyte precursor cells. This tissue reparation apparently mimics the tissue morphogenesis cascade that occurs during embryogenesis (Fausto, et al.(1989) Lab.Investigation 60:4-13). As demonstrated in the example below, morphogen expression is enhanced significantly during hepatic tissue regeneration following galactosamine or carbon tetrachloride (CCl₄)-induced liver damage. Experiments were performed essentially as described in Kuhlmann et al., (1980) Virchows Arch 387:47-57.

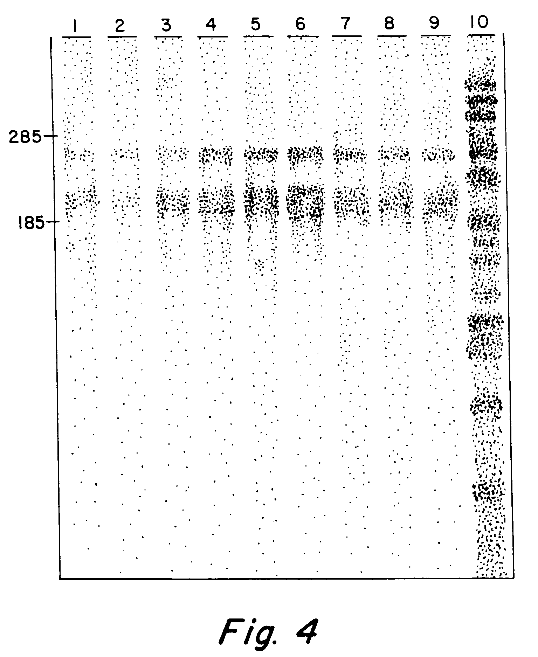

In this experiment, male rats were provided with a single intraperitoneal injection of galactosamine-HCl 0.75 g/.kg body weight on day 0, and morphogen expression monitored by standard Northern blot of liver tissue samples taken on days 1-7 and day 10. OP-1 expression was significantly enhanced during this hepatic tissue regenerative period, indicating that morphogens play a significant role in tissue regeneration. A representation of the Northern blot is presented in Fig. 4. In Fig. 4, lanes 1-8 are samples taken on days 0-7; lane 9 is a sample taken on day 10, and lane 10 contains molecular weight markers. OP-1 mRNA shows a significant expression spike on days 3-7. Similar results were seen with tissue regeneration stimulated following CCl₄-induced tissue, wherein CCl₄ intoxication is induced by orally administering 1.5g CCl₄/kg body weight. Significant morphogen expression (mOP-1 mRNA, as determined by standard Northern blot) is identified by a hybridization spike at 12 hours and continuing through at least 72 hours.

### Example 6. Morphogen Inhibition of Cellular and Humoral Inflammatory Response

The morphogens described herein may be used to alleviate tissue damage associated with immune response-mediated damage to liver tissue. Details of this damage and the use of morphogens to alleviate this injury as well as to provide a cytoprotective effect in anticipation of this injury for example, during a transplant procedure, are disclosed in international application US92/07358 (WO93/04672). A primary source of such damage to hepatic tissue results, for example, from reduced perfusion of the hepatic blood supply and/or from partial or complete occlusion of the portal vein. As described in international application US92/07358 (WO93/04672) morphogens have been shown to alleviate damage to myocardial tissue following ischemia-reperfusion injury. The morphogens also alleivate analogous tissue damage to hepatic tissue.

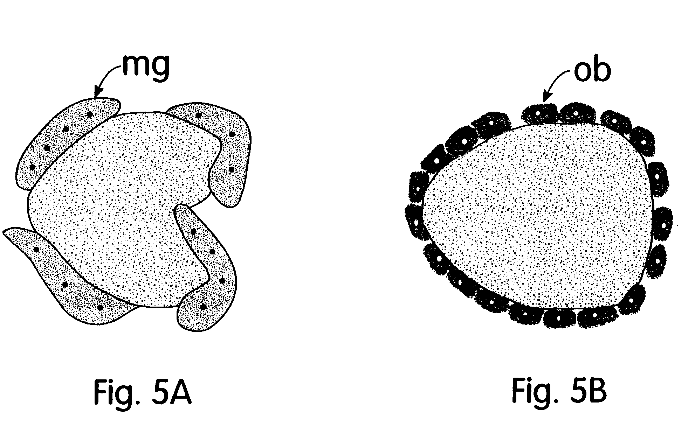

Morphogens described herein inhibit multinucleation of mononuclear phagocytic cells under conditions where these cells normally would be activated, e.g., in response to a tissue injury or the presence of a foreign substance. For example, in the absence of morphogen, an implanted substrate material (e.g., implanted subcutaneously) composed of, for example, mineralized bone, a ceramic such as titanium oxide or any other substrate that provokes multinucleated giant cell formation, rapidly becomes surrounded by multinucleated giant cells, e.g., activated phagocytes stimulated to respond and destroy the foreign object. In the presence of morphogen however, the recruited cells remain in their mononuclear precursor form and the matrix material is undisturbed. Figure 5 illustrates this effect of morphogens, in a schematic representation of histology results of a titanium oxide substrate implanted subcutaneously. In the figure, "mg" means multinucleated giant cells and "ob" means osteoblasts. The substrate represented in Fig. 5B was implanted together with morphogen (OP-1) and newly formed osteoblasts are evident surrounding the substrate. By contrast, the substrate represented in Fig. 5A was implanted without morphogen and extensive multinucleated giant cell formation is evident surrounding the substrate. Accordingly, the morphogens' effect in inhibiting excessive bone mass loss in a mammal also may include inhibiting activation of these giant cells.

In addition, the morphogens described herein also suppress antibody production stimulated in response to a foreign antigen in a mammal. Specifically, when bovine bone collagen matrix alone was implanted in a bony site in a rat, a standard antibody response to the collagen is stimulated in the rat as determined by standard anti-bovine collagen ELISA experiments performed on blood samples taken at four week intervals following implantation (e.g., between 12 and 20 weeks.) Serum anti-collagen antibody titers, measured by ELISA essentially following the procedure described by Nagler-Anderson et al, (1986) PNAS 83:7443-7446, increased consistently throughout the experiment. However, when the matrix was implanted together with a morphogen (e.g., OP-1, dispersed in the matrix and adsorbed thereto, essentially as described in U.S. Pat. No. 4,968,590) anti-bovine collagen antibody production was suppressed significantly. This ability of morphogen to suppress the humoral response is further evidence of morphogen utility in alleviating tissue damage associated with autoimmune diseases, including autoantibody diseases, such as rheumatoid arthritis.

### Example 7. Morphogen Effect on Fibrogenesis and Scar Tissue Formation

The morphogens described herein induce tissue morphogenesis of damaged or lost tissue. The ability of these proteins to regenerate new tissue also is enhanced by the anti-inflammatory effect of these proteins. Provided below are a series of in vitro experiments demonstrating the ability of morphogens to induce migration and accumulation of mesenchymal cells. In addition, the experiments demonstrate that morphogens, unlike TGF-β, do not stimulate fibrogenesis or scar tissue formation. Specifically, morphogens do not stimulate production of collagen, hyaluronic acid (HA) or metalloproteinases in primary fibroblasts, all of which are required for fibrogenesis or scar tissue formation. By contrast, TGF-β, a known inducer of fibrosis, but not of tissue morphogenesis as described herein, does stimulate production of these fibrosis markers.

Chemotaxis and migration of mesenchymal progenitor cells were measured in modified Boyden chambers essentially as described by Fava, R.A. et al (1991) J. Exp. Med. 173: 1121-1132 using polycarbonate filters of 2, 3 and 8 micron ports to measure migration of progenitor neutrophils, monocytes and fibroblasts. Chemotaxis was measured over a range of morphogen concentrations, e.g., 10⁻²⁰ M to 10⁻¹²M OP-1. For progenitor neutrophils and monocytes, 10⁻¹⁸-10⁻¹⁷M OP-1 consistently induced maximal migration, and 10⁻¹⁴ to 10⁻¹³M OP-1 maximally induced migration of progenitor fibroblasts. In all cases the chemotactic activity could be inhibited with anti-OP-1 antibody. Similar migration activities also were measured and observed with TGF-β.

The effect of morphogen on fibrogenesis was determined by evaluating fibroblast production of hyaluronic acid (HA), collagen, collagenese and tissue inhibitor of metalloproteinases (TIMP).

Human fibroblasts were established from explants of infant foreskins and maintained in monolayer culture using standard culturing procedures. (See, for example, (1976) J. Exp. Med. 144: 1188-1203.) Briefly, fibroblasts were grown in maintenance medium consisting of Eagle's MEM, supplemented with nonessential amino acids, ascorbic acid (50 µg/ml), NaHCO₃ and HEPES buffers (pH 7.2), penicillin (100 U/ml), streptomycin (100 µg/ml), amphotericin B (1 µg/ml) and 9% heat inactivated FCS. Fibroblasts used as target cells to measure chemotaxis were maintained in 150 mm diameter glass petri dishes. Fibroblasts used in assays to measure synthesis of collagen, hyaluronic acid, collagenase and tissue inhibitors of metalloproteinases (TIMP) were grown in 100 mm diameter plastic tissue culture petri dishes.

The effects of morphogen on fibroblast production of hyaluronic acid, collagens, collagenase and TIMP were determined by standard assays (See, for example, Posttethwaite et al. (1989) J. Clin. Invest. 83: 629-636, Posttethwaithe (1988) J./ Cell Biol. 106: 311-318 and Clark et al (1985) Arch. Bio-chem Biophys. 241: 36-44.

For these assays, fibroblasts were transferred to 24-well tissue culture plates at a density of 8 x 10⁴ cells per well. Fibroblasts were grown confluency in maintenance medium containing 9% FCS for 72 h and then grown in serum-free maintenance medium for 24 h. Medium was then removed from each well and various concentrations of OP-1 (recombinantly produced mature or soluble form) or TGF-β-1 (R&D Systems, Minneapolis) in 50 µl PBS were added to triplicate wells containing the confluent fibroblast monolayers. For experiments that measured production of collagenase and TIMP, maintenance medium (450 µl) containing 5% FCS was added to each well, and culture supernatants were harvested from each well 48 h later and stored at -70°C until assayed. For experiments that assessed HA production, maintenance medium (450 µl) containing 2.5% FCS was added to each well, and cultures grown for 48 h. For experiments that measured fibroblast production of collagens, serum-free maintenance medium (450 µl) without non-essential amino acids was added to each well and cultures grown for 72 h. Fibroblast production of HA was measured by labeling newly synthesized glycosaminoglycans (GAG) with [³H]-acetate the last 24 h of culture and quantitating released radioactivity after incubation with hyaluronidase from Streptomyces hyalurolyticus (ICN Biochemicals, Cleveland, OH) which specifically degrades hyaluronic acid. Production of total collagen by fibroblasts was measured using a collagenase-sensitive protein assay that reflects [³H]-proline incorporation the last 24 h of culture into newly synthesized collagens. Collagenase and TIMP protein levels in fibroblast cultures supernatants was measured by specific ELISAs.

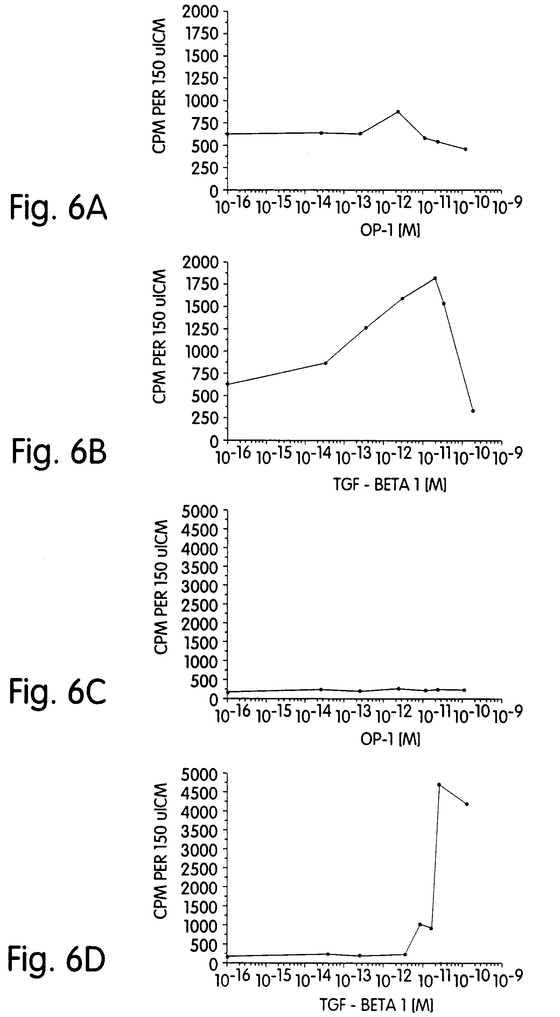

As shown in Fig. 6, OP1 does not stimulate significant collagen or HA production, as compared with TGF-β. In the figure, panel A shows OP-1 effect on collagen production, panel B shows TGF-β effect on collagen production, and panels C and D show OP-1 (panel C) and TGF-β (panel D) effect on HA production. The morphogen results were the same whether the soluble or mature form of OP1 was used. By contrast, the latent form of TGF-β (e.g., pro domain-associated form of TGF-β) was not active.

### Example 8. Liver Tissue Diagnostics

Morphogen localization in developing and regenerating liver tissue can be used as part of a method for diagnosing a liver function disorder in vivo. The method may be particularly advantageous for diagnosing early stages of a liver dysfunction associated with a hepatocellular injury. Specifically, a biopsy of liver tissue is performed on a patient at risk, using standard procedures known in the medical art. Morphogen expression associated with the biopsied tissue then is assessed using standard methodologies, as by immunolocalization, using standard immunofluorescence techniques in concert with morphogen-specific antisera or monoclonal antibodies. Specifically, the biopsied tissue is thin sectioned using standard methodologies known in the art, and fluorescently labelled (or otherwise detectable) antibodies having specificity for the morphogen are incubated with the tissue under conditions sufficient to allow specific antigen-antibody complex formation. The presence and quantity of complex formed then is detected and compared with a predetermined standard or reference value. Detection of altered levels of morphogen present in the tissue then may be used as an indicator of tissue dysfunction. Alternatively, fluctuation in morphogen levels may be assessed by monitoring morphogen transcription levels, either by standard Northern blot analysis or by in situ hybridization, using a labelled probe capable of hybridizing specifically to morphogen RNA and standard RNA hybridization protocols well described in the art and as described in Examples 1, 2, 5 and 6.

Fluctuations in morphogen levels present in the bloodstream or peritoneal fluid also may be used to evaluate liver tissue viability. For example, morphogens are detected associated with regenerating liver tissue and/or may be released from dying cells into surrounding peritoneal fluid. OP-1 recently has been identified in human blood, which also may be a means of morphogen transport.

Serum samples may be obtained by standard venipuncture and serum prepared by centrifugation at 3,000 RPM for ten minutes. Similarly, peritoneal fluid samples may be obtained by a standard fluid extraction methodology. The presence of morphogen in the serum or peritoneal fluid then may be assessed by standard Western blot (immunoblot), ELISA or RIA procedures. Briefly, for example, with the ELISA, samples may be diluted in an appropriate buffer, such as phosphate-buffered saline, and 50 µl aliquots allowed to absorb to flat bottomed wells in microtitre plates pre-coated with morphogen-specific antibody, and allowed to incubate for 18 hours at 4°C. Plates then may be washed with a standard buffer and incubated with 50 µl aliquots of a second morphogen-specific antibody conjugated with a detecting agent, e.g., biotin, in an appropriate buffer, for 90 minutes at room temperature. Morphogen-antibody complexes then may be detected using standard procedures.

Alternatively, a morphogen-specific affinity column may be created using, for example, morphogen-specific antibodies adsorbed to a column matrix, and passing the fluid sample through the matrix to selectively extract the morphogen of interest. The morphogen then is eluted. A suitable elution buffer may be determined empirically by determining appropriate binding and elution conditions first with a control (e.g., purified, recombinantly-produced morphogen.) Fractions then are tested for the presence of the morphogen by standard immunoblot. Morphogen concentrations in serum or other fluid samples then may be determined using standard protein quantification techniques, including by spectrophotometric absorbance or by quantitation by ELISA or RIA antibody assays. Using this procedure, OP-1 has been identified in serum.

OP-1 was detected in human serum using the following assay. A monoclonal antibody raised against mammalian, recombinantly produced OP-1 using standard immunology techniques well described in the art and described generally in Example 13, was immobilized by passing the antibody over an activated agarose gel (e.g., Affi-Gel™, from Bio-Rad Laboratories, Richmond, CA, prepared following manufacturer's instructions), and used to purify OP-1 from serum. Human serum then was passed over the column and eluted with 3M K-thiocyanate. K-thiocyanante fractions then were dialyzed in 6M urea, 20mM PO₄, pH 7.0, applied to a C8 HPLC column, and eluted with a 20 minute, 25-50% acetonitrile/0.1% TFA gradient. Mature, recombinantly produced OP-1 homodimers elute between 20-22 minutes. Accordingly, these fractions from the affinity-purified human serum sample were collected and tested for the presence of OP-1 by standard immunoblot using an OP-1-specifc antibody, and the protein identity confirmed by N-terminal sequencing.

Morphogens may be used in diagnostic applications by comparing the quantity of morphogen present in a body fluid sample with a predetermined reference value, with fluctuations in fluid morphogen levels indicating a change in the status of liver tissue. Alternatively, fluctuations in the level of endogenous morphogen antibodies may be detected by this method, most likely in serum, using an antibody or other binding protein capable of interacting specifically with the endogenous morphogen antibody. Detected fluctuations in the levels of the endogenous antibody may be used as indicators of a change in tissue status.

### Example 9. Screening Assay for Candidate Compounds which Alter Endogenous Morphogen Levels

Candidate compound(s) which may be administered to affect the level of a given morphogen may be found using the following screening assay, in which the level of morphogen production by a cell type which produces measurable levels of the morphogen is determined with and without incubating the cell in culture with the compound, in order to assess the effects of the compound on the cell's production of morphogen. This can be accomplished by detection of the morphogen either at the protein or RNA level. A more detailed description also may be found in international application US92/07359 (WO93/05172).

### 9.1 Growth of Cells in Culture

Cell cultures of kidney, adrenals, urinary bladder, brain, or other organs, may be prepared as described widely in the literature. For example, kidneys may be explanted from neonatal or new born or young or adult rodents (mouse or rat) and used in organ culture as whole or sliced (1-4 mm) tissues. Primary tissue cultures and established cell lines, also derived from kidney, adrenals, urinary, bladder, brain, mammary, or other tissues may be established in multiwell plates (6 well or 24 well) according to conventional cell culture techniques, and are cultured in the absence or presence of serum for a period of time (1-7 days). Cells may be cultured, for example, in Dulbecco's Modified Eagle medium (Gibco, Long Island, NY) containing serum (e.g., fetal calf serum at 1%-10%, Gibco) or in serum-deprived medium, as desired, or in defined medium (e.g., containing insulin, transferrin, glucose, albumin, or other growth factors).

Samples for testing the level of morphogen production includes culture supernatants or cell lysates, collected periodically and evaluated for OP-1 production by immunoblot analysis (Sambrook et al., eds., 1989, Molecular Cloning, Cold Spring Harbor Press, Cold Spring Harbor, NY), or a portion of the cell culture itself, collected periodically and used to prepare polyA+ RNA for mRNA analysis. To monitor de novo OP-1 synthesis, some cultures are labeled according to conventional procedures with an ³⁵S-methionine/³⁵S-cysteine mixture for 6-24 hours and then evaluated to OP-1 synthesis by conventional immunoprecipitation methods.

### 9.2 Determination of Level of Morphogenic Protein

In order to quantitate the production of a morphogenic protein by a cell type, an immunoassay may be performed to detect the morphogen using a polyclonal or monoclonal antibody specific for that protein. For example, OP-1 may be detected using a polyclonal antibody specific for OP-1 in an ELISA, as follows.

1 µg/100 µl of affinity-purified polyclonal rabbit IgG specific for OP-1 is added to each well of a 96-well plate and incubated at 37°C for an hour. The wells are washed four times with 0.167M sodium borate buffer with 0.15 M NaCl (BSB), pH 8.2, containing 0.1% Tween 20. To minimize non-specific binding, the wells are blocked by filling completely with 1% bovine serum albumin (BSA) in BSB and incubating for 1 hour at 37°C. The wells are then washed four times with BSB containing 0.1% Tween 20. A 100 µl aliquot of an appropriate dilution of each of the test samples of cell culture supernatant is added to each well in triplicate and incubated at 37°C for 30 min. After incubation, 100 µl biotinylated rabbit anti-OP-1 serum (stock solution is about 1 mg/ml and diluted 1:400 in BSB containing 1% BSA before use) is added to each well and incubated at 37°C for 30 min. The wells are then washed four times with BSB containing 0.1% Tween 20. 100 µl strepavidin-alkaline (Southern Biotechnology Associates, Inc. Birmingham, Alabama, diluted 1:2000 in BSB containing 0.1% Tween 20 before use) is added to each well and incubated at 37°C for 30 min. The plates are washed four times with 0.5M Tris buffered Saline (TBS), pH 7.2. 50µl substrate (ELISA Amplification System Kit, Life Technologies, Inc., Bethesda, MD) is added to each well and incubated at room temperature for 15 min. Then, 50 µl amplifier (from the same amplification system kit) is added and incubated for another 15 min at room temperature. The reaction is stopped by the addition of 50 µl 0.3 M sulphuric acid. The OD at 490 nm of the solution in each well is recorded. To quantitate OP-1 in culture media, a OP-1 standard curve is performed in parallel with the test samples.

Polyclonal antibody may be prepared as follows. Each rabbit is given a primary immunization of 100 ug/500 µl E. coli produced OP-1 monomer (amino acids 328-431 in SEQ ID NO:5) in 0.1% SDS mixed with 500 µl Complete Freund's Adjuvant. The antigen is injected subcutaneously at multiple sites on the back and flanks of the animal. The rabbit is boosted after a month in the same manner using incomplete Freund's Adjuvant. Test bleeds are taken from the ear vein seven days later. Two additional boosts and test bleeds are performed at monthly intervals until antibody against OP-1 is detected in the serum using an ELISA assay. Then, the rabbit is boosted monthly with 100 µg of antigen and bled (15 ml per bleed) at days seven and ten after boosting.

Monoclonal antibody specific for a given morphogen may be prepared as follows. A mouse is given two injections of E. coli produced OP-1 monomer. The first injection contains 100µg of OP-1 in complete Freund's adjuvant and is given subcutaneously. The second injection contains 50 µg of OP-1 in incomplete adjuvant and is given intraperitoneally. The mouse then receives a total of 230 µg of OP-1 (amino acids 307-431 in SEQ ID NO:5) in four intraperitoneal injections at various times over an eight month period. One week prior to fusion, the mouse is boosted intraperitoneally with 100 µg of OP-1 (307-431) and 30 µg of the N-terminal peptide (Ser₂₉₃-Asn₃₀₉-Cys) conjugated through the added cysteine to bovine serum albumin with SMCC crosslinking agent. This boost was repeated five days (IP), four days (IP), three days (IP) and one day (IV) prior to fusion. The mouse spleen cells are then fused to myeloma (e.g., 653) cells at a ratio of 1:1 using PEG 1500 (Boeringer Mannheim), and the cell fusion is plated and screened for OP-1-specific antibodies using OP-1 (307-431) as antigen. The cell fusion and monoclonal screening then are according to standard procedures well described in standard texts widely available in the art.

The invention may be embodied in other specific forms without departing from the spirit or essential characteristics thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

## Claims

1. A composition for correcting a liver function deficiency or a protein deficiency in a mammal, comprising:
(a) hepatocytic or hepatic progenitor cells capable of expressing one or more proteins in vivo to correct said deficiency;
(b) a biocompatible, acellular matrix having a three-dimensional structure suitable for the attachment, infiltration, differentiation and proliferation of said cells; and
(c) a morphogen, either adsorbed on said matrix or provided to said cells at a concentration effective for inducing said cells to correct said deficiency when said cells are attached to said matrix and implanted in said mammal, said morphogen comprising a dimeric protein that induces hepatic tissue morphogenesis, said dimeric protein comprising a pair of folded polypeptides, the amino acid sequence of each of which comprises a sequence sharing at least 70% homology with the C-terminal seven cysteine domain of human OP-1, residues 38-139 of Seq. ID No. 5.

2. The composition of claim 1 wherein the progenitor cells are allogenic cells.

3. The composition of claim 1 or claim 2 wherein said cells comprise foreign genetic material.

4. The composition of claim 3 wherein the foreign genetic material encodes said one or more proteins.

5. The composition of any one of claims 1-4 wherein said matrix is in vivo biodegradable or comprises a synthetic polymeric material.

6. The composition of claim 5 wherein said polymeric material comprises polylactic acid, polybutyric acid, polyglycolic acid, polyanhydride, or copolymers thereof.

7. The composition of any one of claims 5 or 6 wherein said polymeric material further comprises a structural molecule selected from collagen, laminin or hyaluronic acid, or a cell attachment factor selected from glycosaminoglycans or proteoglycans.

8. The composition of any one of claims 5-7 wherein the matrix is derived from hepatic tissue.

9. The composition of any one of the proceding claims wherein:
(i) said morphogen comprises a polypeptide comprising an amino acid sequence sharing at least 80% homology with the C-terminal seven cysteine domain of human OP-1, residues 38-139 of Seq. ID No. 5, or
(ii) said morphogen comprises a polypeptide comprising an amino acid sequence having greater than 60% identity, preferably greater than 65% identity, with the amino acid sequence defining the conserved six cysteine skeleton of hOP1 residues 43-139 of Seq. ID No. 5, or
(iii) said morphogen comprises a polypeptide comprising an amino acid sequence defined by OPX Seq. ID No. 29, or
(iv) said morphogen comprises a polypeptide comprising the sequence of the C-terminal seven cysteine domain of human OP-1, residues 38-139 of Seq. ID No. 5, or allelic or species variant thereof, or
(v) said morphogen is homodimeric or heterodimeric.

10. A kit for detecting reduced liver function or hepatocellular injury in a mammal, or for evaluating efficacy of a therapy for treating a malady associated with reduced liver function or hepatocellular injury in a mammal, comprising:
(a) means for capturing a cell or body fluid sample obtained from said mammal;
(b) a binding protein that interacts specifically with a morphogen in said sample so as to form a binding protein-morphogen complex, said morphogen comprising a dimeric protein that induces hepatic tissue morphogenesis, said dimeric protein comprising a pair of folded polypeptides, the amino acid sequence of each of which comprises a sequence sharing at least 70% homology with the C-terminal seven cysteine domain of human OP-1, residues 38-139 of Seq. ID No. 5; and
(c) means for detecting said complex.

11. A method for detecting reduced liver function or hepatocellular injury in a mammal, or for evaluating efficacy of a therapy for treating a malady associated with reduced liver function or hepatocellular injury in said mammal, said method comprising the step of detecting, in a sample of serum or peritoneal fluid obtained from said mammal, a change in the concentration of a morphogen, or of the titer of an antibody that binds specifically thereto, said change being indicative of an increase in hepatic cell death, said morphogen comprising a dimeric protein that induces hepatic tissue morphogenesis, said dimeric protein comprising a pair of folded polypeptides, the amino acid sequence of each of which comprises a sequence sharing at least 70% homology with the C-terminal seven cysteine domain of human OP-1, residues 38-139 of Seq. ID No. 5.

12. Use of a morphogen for the manufacture of an implantable, proliferating cellular device to correct a liver function deficiency or protein deficiency in a mammal, said device comprising proliferating hepatocytic or hepatic progenitor cells and a biocompatible, acelluar matrix having a three-dimensional structure suitable for the attachment, infiltration and differentiation in vivo of said proliferating cells, said morphogen comprising a dimeric protein that induces hepatic tissue morphogenesis, said dimeric protein comprising a pair of folded ploypeptides, the amino acid sequence of each of which comprises a sequence sharing at least 70% homology with the C-terminal seven cysteine domain of human OP-1, residues 38-139 of Seq. ID No. 5.

13. The use of claim 12 wherein the proliferating cells are allogenic to said mammal.

14. The use of claim 12 or claim 13 wherein the proliferating cells comprise foreign genetic material.

15. Use according to any one of claims 12-14 wherein:
(a) said matrix is a defined in claims 5, 6, 7 or 8, or
(b) wherein the amino acid sequences of said morphogen polypeptides are as defined in claim 9.

## Patentansprüche

1. Zusammensetzung zur Korrektur eines Leberfunktionsdefektes oder eines Proteinmangels in einem Säuger, enthaltend:
(a) hepatozytische oder hepatische Vorstufen-Zellen, die die Fähigkeit besitzen, ein oder mehrere Proteine in vivo zur Mangelkorrektur auszuprägen;
(b) eine biokompatible azelluläre Matrix mit einer dreidimensionalen Struktur, die zur Anheftung, Infiltration, Differenzierung und Proliferation solcher Zellen geeignet ist; und
(c) ein Morphogen, das entweder auf der Matrix adsorbiert ist oder den Zellen zur Verfügung gestellt wird, bei einer effektiven Konzentration zum Anregen der Zellen zur Korrektur des Mangels wenn die Zellen an der Matrix angeheftet und im Säuger implantiert sind, wobei das Morphogen ein dimeres Protein enthält, das hepatische Gewebe-Morphogenese induziert, wobei das dimere Protein ein Paar gefalteter Polypeptide enthält, deren jeweilige Aminosäure-Sequenz eine Sequenz enthält, die mindestens eine 70 %-ige Homologie mit der C-terminalen sieben Cystein-Domäne von menschlichem OP-1, Gruppen 38 - 139 der Sequenz ID Nr. 5, aufweist.

2. Zusammensetzung nach Anspruch 1, wobei die Vorstufen-Zellen allogene Zellen sind.

3. Zusammensetzung nach Anspruch 1 oder 2, wobei die Zellen fremdes genetisches Material enthalten.

4. Zusammensetzung nach Anspruch 3, wobei das fremde genetische Material das eine oder mehrere Proteine kodiert.

5. Zusammensetzung nach einem der Ansprüche 1 - 4, wobei die Matrix in vivo biologisch abbaubar ist oder ein synthetisches Polymer-Material enthält.

6. Zusammensetzung nach Anspruch 5, wobei das Polymer-Material Poly-Milchsäure, Poly-Buttersäure, Poly-Glykolsäure, Poly-Anhydrid oder co-Polymere davon enthält.

7. Zusammensetzung nach einem der Ansprüche 5 oder 6, wobei das Polymer-Material zusätzlich ein Struktur-Molekül, ausgewählt aus Kollagen, Laminin oder Hyaluronsäure, oder einen Zellanheftungslaktor, ausgewählt aus Glykosaminoglykan oder Proteoglykan, enthält.

8. Zusammensetzung nach einem der Ansprüche 5 - 7, wobei die Matrix aus hepatischem Gewebe stammt.

9. Zusammensetzung nach einem der vorhergehenden Ansprüche, wobei:
(i) das Morphogen ein Polypeptid enthält, das eine Aminosäure-Sequenz enthält, die mindestens eine 80%-ige Homologie mit der C-terminalen sieben Cystein-Domäne von menschlichem OP-1, Gruppen 38 - 139 der Sequenz ID Nr. 5, aufweist, oder
(ii) das Morphogen ein Polypeptid enthält, das eine Aminosäure-Sequenz enthält, die eine größere als 60 %-ige Identität, vorzugsweise eine größere als 65%-ige Identität, mit der Aminosäure-Sequenz aufweist, die das erhaltene sechs Cystein-Skelett von menschlichem OP-1, Gruppen 43 - 139 der Sequenz ID Nr. 5, definiert, oder
(iii) das Morphogen ein Polypeptid enthält, das eine Aminosäure-Sequenz enthält, die durch OPX Seq. ID Nr. 29 definiert wird, oder
(iv) das Morphogen ein Polypeptid enthält, das die Sequenz der C-terminalen sieben Cystein-Domäne von menschlichem OP-1, Gruppen 38 - 139 der Seq. ID Nr. 5, oder eine allelische oder Spezies-Variante davon enthält, oder
(v) das Morphogen homo- oder heterodimerisch ist.

10. Kit zur Detektion einer reduzierten Leberfunktion oder hepatozellulären Verletzung in einem Säuger, oder zur Bestimmung der Wirksamkeit einer Therapie zur Behandlung einer Krankheit, die mit einer reduzierten Leberfunktion oder einer hepatozellulären Verletzung in einem Säuger verbunden ist, enthaltend:
(a) Mittel zum Einfangen einer von einem Säuger erhaltenen Zell- oder Körperfluid-Probe`;
(b) ein Bindungsprotein, das spezitisch mit einem Morphogen in der Probe interagiert, um auf diese Weise einen Bindungsproteinmorphogenkomplex zu bilden, wobei das Morphogen ein dimeres Protein enthält, das hepatische Gewebe-Morphogenese induziert, wobei das dimere Protein ein Paar gefalteter Polypeptide enthält, deren jeweilige Aminosäure-Sequenz eine Sequenz enthält, die mindestens eine 70%-ige Homologie mit der C-terminalen sieben Cystein-Domäne von menschlichem OP-1, Gruppen 38 - 139 der Seq. ID Nr. 5, aufweist; und
(c) Mittel zur Detektion des Komplexes.

11. Verfahren zur Detektion einer reduzierten Leberfunktion oder einer hepatozellulären Verletzung in einem Säuger, oder zur Bestimmung der Wirksamkeit einer Therapie zur Behandlung einer Krankheit, die mit einer reduzierten Leberfunktion oder hepatozellulären Verletzung im Säuger verbunden ist, wobei das Verfahren den Schritt der Detektion in einer vom Säuger erhaltenen Serum- oder peritonealen Fluidprobe aufweist, einem Wechsel in der Konzentration eines Morphogens oder des Titers eines Antikörpers, der dort spezitisch bindet, wobei der Wechsel kennzeichnend für eine Zunahme hepatischen Zelltodes ist, wobei das Morphogen ein dimeres Protein enthält, das hepatische Zellmorphogenese induziert, wobei das dimere Protein ein Paar gefalteter Polypeptide enthält, deren jeweilige Aminosäure-Sequenz eine Sequenz enthält, die mindestens eine 70%-ige Homologie mit der C-terminalen sieben Cystein-Domäne von menschlichem OP-1, Gruppen 38 - 139 der Seq. ID Nr. 5, aufweist.

12. Verwendung eines Morphogens zur Herstellung einer implantierbaren, proliferierenden zellulären Vorrichtung zur Korrektur eines Leberfunktionsdefektes oder Proteinmangels in einem Säuger, wobei die Vorrichtung proliferierende hepatozylische oder hepatische Vorstufen-Zellen und eine biokompatible, azelluläre Matrix mit einer dreidimensionalen Struktur enthält, die zur Anheftung, Infiltration und Differenzierung in vivo der proliferierenden Zellen geeignet ist, wobei das Morphogen ein dimeres Protein enthält, das hepatische Gewebemorphogenese induziert, wobei das dimere Protein ein Paar gefalteter Polypeptide enthält, deren jeweilige Aminosäure Sequenz eine Sequenz enthält, die mindestens eine 70%-ige Homologie mit der C-terminalen sieben Cystein-Domäne von menschlichem OP-1, Gruppen 38 - 139 der Seq. ID Nr. 5, aufweist.

13. Verwendung nach Anspruch 12, wobei die proliferierenden Zellen allogen zum Säuger sind.

14. Verwendung nach Anspruch 12 oder 13, wobei die proliferierenden Zellen fremdes genetisches Material enthalten.

15. Verwendung nach einem der Ansprüche 12 - 14, wobei
(a) die Matrix eine solche wie die in den Ansprüchen 5, 6, 7 oder 8 definierte ist, oder
(b) die Aminosäure-Sequenzen der Morphogen-Polypeptide solche wie die in Anspruch 9 definierten sind.

## Revendications

1. Composition destinée à corriger une insuffisance de la fonction hépatique ou une carence protéinique chez un mammifère, comprenant :
(a) des hépatocytes ou cellules hépatiques souches capables d'exprimer une ou plusieurs protéines in vivo pour corriger ladite insuffisance ou carence ;
(b) une matrice acellulaire, biocompatible, ayant une structure tridimensionnelle pouvant être utilisée pour la fixation, l'infiltration, la différenciation et la prolifération desdites cellules ; et
(c) un morphogène, soit adsorbé sur ladite matrice, soit fourni auxdites cellules à une concentration efficace pour induire lesdites cellules à corriger ladite insuffisance ou carence quand lesdites cellules sont fixées à ladite matrice et implantées chez ledit mammifère, ledit morphogène comprenant une protéine dimère qui induit la morphogenèse du tissu hépatique, ladite protéine dimère comprenant une paire de polypeptides repliés, la séquence d'acides aminés de chacun d'eux comprenant une séquence partageant une homologie d'au moins 70 % avec le domaine de sept cystéines C-terminal de hOP-1, résidus 38 à 139 de la Séq. ID N° 5.

2. Composition selon la revendication 1, dans laquelle les cellules souches sont des cellules allogéniques.

3. Composition selon la revendication 1 ou la revendication 2, dans laquelle lesdites cellules comprennent un matériau génétique étranger.

4. Composition selon la revendication 3, dans laquelle le matériau génétique étranger code lesdites une ou plusieurs protéines.

5. Composition selon l'une quelconque des revendications 1 à 4, dans laquelle ladite matrice est biodégradable in vivo ou comprend un matériau polymère synthétique.

6. Composition selon la revendication 5, dans laquelle ledit matériau polymère comprend l'acide polylactique, l'acide polybutyrique, l'acide polyglycolique, un polyanhydride, ou des copolymères de ceux-ci.

7. Composition selon l'une quelconque des revendications 5 ou 6, dans laquelle ledit matériau polymère comprend de plus une molécule structurale choisie parmi le collagène, la laminine ou l'acide hyaluronique, ou un facteur de fixation cellulaire choisi parmi les glycosaminoglycanes ou les protéoglycanes.

8. Composition selon l'une quelconque des revendications 5 à 7, dans laquelle la matrice est dérivée du tissu hépatique.

9. Composition selon l'une quelconque des revendications précédentes, dans laquelle :
(i) ledit morphogène comprend un polypeptide comprenant une séquence d'acides aminés partageant une homologie d'au moins 80 % avec le domaine de sept cystéines C-terminal de hOP-1, résidus 38 à 139 de la Séq. ID N° 5, ou
(ii) ledit morphogène comprend un polypeptide comprenant une séquence d'acides aminés présentant une identité supérieure à 60 %, de préférence, supérieure à 65 %, avec la séquence d'acides aminés définissant le squelette de six cystéines conservé des résidus hOP1 43 à 139 de la Séq. ID N° 5, ou
(iii) ledit morphogène comprend un polypeptide comprenant une séquence d'acides aminés définie par OPX Séq. ID N° 29, ou
(iv) ledit morphogène comprend un polypeptide comprenant la séquence du domaine de sept cystéines C-terminal de hOP-1, résidus 38 à 139 de la Séq. ID N° 5, ou une variante allélique ou d'espèce de celle-ci, ou
(v) ledit morphogène est homodimère ou hétérodimère.

10. Trousse pour la détection d'une fonction hépatique réduite ou d'une lésion hépatocellulaire chez un mammifère, ou pour l'évaluation de l'efficacité d'une thérapie visant à traiter une maladie associée à une fonction hépatique réduite ou à une lésion hépatocellulaire chez un mammifère, comprenant :
(a) des moyens pour capturer un échantillon de cellule ou de fluide corporel obtenu à partir dudit mammifère ;
(b) une protéine fixatrice qui a une interaction spécifique avec un morphogène dans ledit échantillon de façon à former un complexe protéine fixatrice-morphogène, ledit morphogène comprenant une protéine dimère qui induit la morphogenèse du tissu hépatique, ladite protéine dimère comprenant une paire de polypeptides repliés, la séquence d'acides aminés de chacun d'eux comprenant une séquence partageant une homologie d'au moins 70 % avec le domaine de sept cystéines C-terminal de hOP-1, résidus 38 à 139 de la Séq. ID N° 5 ; et
(c) des moyens pour détecter ledit complexe.

11. Procédé permettant de détecter une fonction hépatique réduite ou une lésion hépatocellulaire chez un mammifère, ou d'évaluer l'efficacité d'une thérapie visant à traiter une maladie associée à une fonction hépatique réduite ou à une lésion hépatocellulaire chez ledit mammifère, ledit procédé comprenant l'étape de détection, dans un échantillon de sérum ou de fluide péritonéal obtenu à partir dudit mammifère, d'un changement de concentration d'un morphogène, ou du titre d'un anticorps qui se lie spécifiquement à celui-ci, ledit changement étant indicateur d'une augmentation de la mortalité des cellules hépatiques, ledit morphogène comprenant une protéine dimère qui induit la morphogenèse du tissu hépatique, ladite protéine dimère comprenant une paire de polypeptides repliés, la séquence d'acides aminés de chacun d'eux comprenant une séquence partageant une homologie d'au moins 70 % avec le domaine de sept cystéines C-terminal de hOP-1, résidus 38 à 139 de la Séq. ID N° 5.

12. Utilisation d'un morphogène pour la fabrication d'un dispositif à cellules proliférantes implantable pour corriger une insuffisance de la fonction hépatique ou une carence protéinique chez un mammifère, ledit dispositif comprenant des hépatocytes ou des cellules hépatiques souches de type proliférant et une matrice acellulaire, biocompatible, ayant une structure tridimensionnelle utilisable pour la fixation, l'infiltration et la différenciation in vivo desdites cellules proliférantes, ledit morphogène comprenant une protéine dimère qui induit la morphogenèse du tissu hépatique, ladite protéine dimère comprenant une paire de polypeptides repliés, la séquence d'acides aminés de chacun d'eux comprenant une séquence partageant une homologie d'au moins 70 % avec le domaine de sept cystéines C-terminal de hOP-1, résidus 38 à 139 de la Séq. ID N° 5.

13. Utilisation selon la revendication 12, dans laquelle les cellules proliférantes sont allogéniques vis-à-vis dudit mammifère.

14. Utilisation selon la revendication 12 ou la revendication 13, dans laquelle les cellules proliférantes comprennent un matériau génétique étranger.

15. Utilisation selon l'une quelconque des revendications 12 à 14, dans laquelle :
(a) ladite matrice est telle que définie dans les revendications 5, 6, 7 ou 8, ou
(b) dans laquelle les séquences d'acides aminés desdits polypeptides du morphogène sont telles que définies dans la revendication 9.
